# EUROPEAN PATENT APPLICATION

(11) **EP 3 327 139 A1**
(43) Date of publication of application: **30.05.2018**
(21) Application number: 17191835.2
(22) Date of filing: 06.04.2010
(51) Int. Cl.: C12Q 1/68, C12N 15/11, G01N 33/52

(54) **DIGITAL QUANTIFICATION OF DNA METHYLATION**

(30) Priority: 06.04.2009 US 166970 P
(62) Divisional of application: 10762298.7
(71) Applicant: The Johns Hopkins University, Baltimore, MD 21218 (US); Case Western Reserve University, Cleveland, OH 44106-7285 (US)
(72) Inventor: VOGELSTEIN, Bert, Baltimore, MD 21208 (US); KINZLER, Kenneth, Baltimore, MD 21015 (US); LI, Ming, Baltimore, MD 21234 (US); DIAZ, Luis, Ellicott City, MD 21075 (US); PAPADOPOULOS, Nickolas, Towson, MD 21204 (US); MARKOWITZ, Sanford, Pepper Pike, OH 44124 (US)
(74) Representative: Tombling, Adrian George

(57) **Abstract**

Abnormal DNA methylation can be used as a biomarker in cancer patients. For such purposes, it is important to determine precisely the fraction of methylated molecules in an analyzed sample. A technology we term Methyl-BEAMing achieves this goal. Individual bisulfite-treated DNA molecules can be PCR-amplified within aqueous nanocompartments containing beads, resulting in a population of beads each containing thousands of copies of the template molecule. After hybridization with probes specific for methylated sequences, the beads can be analyzed by flow cytometry. This approach enables detection and enumeration of one methylated molecule in a population of ∼5000 unmethylated molecules. Methyl-BEAMing provides digital quantification of rare methylation events and is generally applicable to the assessment of methylated genes in clinical samples.

## Description

This invention was made using funding from the National Institutes of Health. The U.S. government retains certain rights in the invention according to the terms of CA 43460, CA 57345, CA 62924, and CA 121113.

### TECHNICAL FIELD OF THE INVENTION

This invention is related to the area of nucleic acid analysis. In particular, it relates to analysis of methylated nucleic acids.

### BACKGROUND OF THE INVENTION

In humans, DNA methylation is largely restricted to cytosines within 5'-CpG dinucleotides. This covalent modification of DNA functions as an important mediator of gene regulation and, together with covalent modifications of histone proteins, forms the cornerstone for the burgeoning field of epigenetics. In addition to its role in development, imprinting, and cellular differentiation, DNA methylation is altered in cancer cells. Though cancers are globally hypomethylated^{1, 2}, specific regions of genes have been shown to be hypermethylated in associated with transcription silencing^{3,4}, . Reversal of the hypermethylation with pharmacological agents such as 5-azacytidine can reactivate such genes⁴. Though many regions of the cancer genome are abnormally methylated, the methylation and silencing of tumor suppressor genes are particularly important. In such cases, methylation serves as a heritable mechanism to inactivate a tumor suppressor gene, with the end result similar to that resulting from mutational inactivation of the gene.

In addition to its implications for gene regulation, DNA methylation is providing a new generation of cancer biomarkers^{5, 6}. Though mutant sequences provide exquisitely specific biomarkers of this class, their utility is compromised by their heterogeneity: the same gene can be mutationally inactivated through many different mechanisms or mutated at many different positions. In contrast, DNA hypermethylation in cancers often affects identical residues in the regulatory regions of particular genes, providing significant advantages in biomarker test design. Accordingly, many studies have employed DNA methylation of specific genes for diagnostics development⁷⁻¹³. Such diagnostic tests can in principle be used for early detection of cancers, prognosis, assessing the effects of therapy or detecting residual diseases.

Many diagnostic tests based on DNA methylation have employed bisulfite to convert cytosine residues to uracils. This conversion alters the sequence of DNA, providing an opportunity to assess DNA methylation with allele-specific PCR, restriction digestion or specific hybridization probes¹⁴⁻¹⁸. Such techniques have been enormously useful for establishing the position of hypermethylated sequences in the cancer genome as well as for documenting the potential utility of such methylation for diagnostic purposes. However, in many important diagnostic scenarios, DNA from the cancer represents only a small fraction of the total DNA in the clinical sample. Such scenarios include the use of DNA from plasma/serum, urine, feces, or sputum for early diagnosis or therapeutic monitoring and the use of DNA from surgical margins or lymph nodes to monitor the extent of disease¹⁹. Currently used techniques for assessing methylation generally do not provide direct quantitative information about the fraction of DNA fragments that are methylated or are incapable of detecting small fractions of methylated fragments. The actual fraction of methylated fragments can be determined through cloning and sequencing of PCR products or next-generation sequencing technologies^{20, 21}, but these approaches cannot easily be used in a clinical diagnostics setting.

There is a continuing need in the art for sensitive and accurate analytic methods.

### SUMMARY OF THE INVENTION

According to one embodiment of the invention a method is provided for determining fraction of molecules comprising a methylated sequence in a sample of analyte DNA molecules comprising the sequence. A sample of analyte DNA molecules is treated with a reagent which selectively modifies methylated cytosine residues or which selectively modifies unmethylated cytosine residues. Microemulsions are formed comprising the treated analyte DNA molecules. A portion of a treated analyte DNA molecule is amplified in the microemulsions in the presence of beads. The portion comprises one or more 5'-CpG methylation sites. The beads are bound to a plurality of molecules of a primer for amplifying the analyte DNA molecules. A plurality of copies of analyte DNA molecule are formed covalently attached to the plurality of molecules of the primer which are bound to beads. Nucleotide sequences at the one or more 5'-CpG methylation sites of analyte DNA molecules which are bound to beads are determined and beads that have modified 5'-CpG methylation sites and beads that have unmodified 5'-CpG methylation sites are quantified.

According to another aspect of the invention a bead is provided which is bound to a plurality of molecules of a primer for amplifying DNA molecules. The primer comprises at least 15 contiguous nucleotides selected from the group consisting of 5' - GTTGTTTAGG TTGTAGGTGN GGG-3 (SEQ ID NO: 2), 5'-CTCNTCCTCC TACCNCAAAA TATTC-3' (SEQ ID NO: 3), and the complements thereof,

These and other embodiments which will be apparent to those of skill in the art upon reading the specification provide the art with

### BRIEF DESCRIPTION OF THE DRAWINGS

Fig. 1 Figure 1 Schematic of Methyl-BEAMing. "Mixtures" represent beads from aqueous nanocompartments that contained both methylated and unmethylated vimentin fragments. "Virgin" beads represent those from aqueous nanocompartments that did not contain any vimentin fragments.
Fig. 2 Representative results of Methyl-BEAMing obtained with flow cytometry. The number of beads representing methylated, unmethylated, mixture, and virgin beads are indicated in the top right corner of each box.
Fig. 3 Vimentin Methyl-BEAMing of plasma from colorectal cancer patients. (a) Receiver operating characteristic (ROC) curves based on the number of methylated vimentin fragments for the indicated classes of samples. (b) The number of methylated vimentin fragments in 2 ml plasma from normal, age and sex-matched control patients. (c) The number of methylated vimentin fragments in 2 ml plasma from colorectal cancer patients of various stages. The doted line represents 1 methylated fragment per 2 ml plasma DNA.

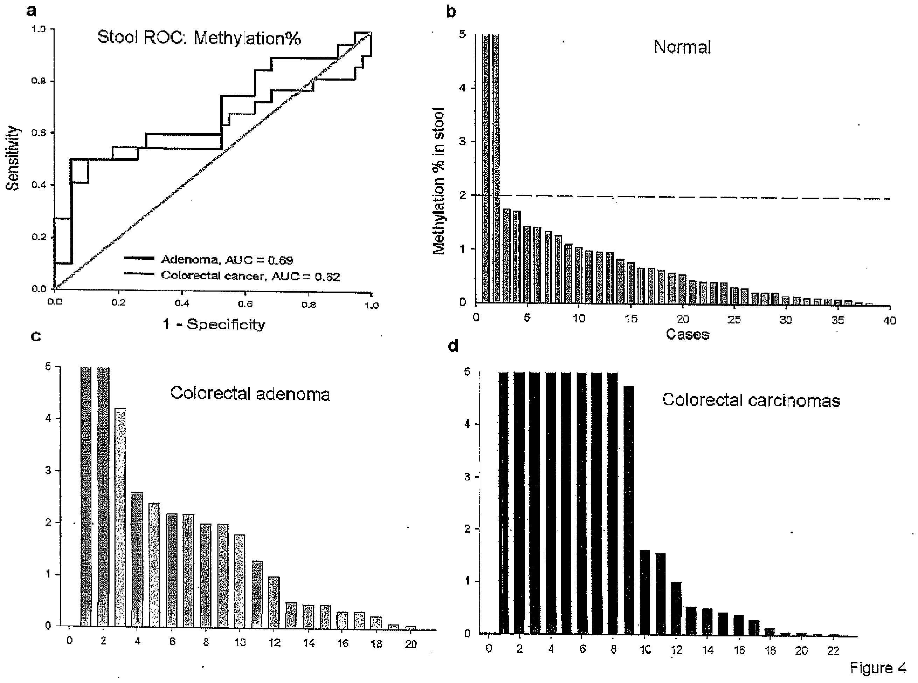

Fig. 4 Vimentin Methyl-BEAMing of fecal DNA from colorectal tumor patients. (a) Receiver operating characteristic (ROC) curves based on the fraction of methylated vimentin fragments for the indicated classes of samples. (b) The fraction of methylated vimentin fragments in 4 g feces from normal, age and sex-matched control patients. (c) The fraction of methylated vimentin fragments in 4 g feces from patients with colorectal adenomas (d) The fraction of methylated vimentin fragments in 4 g feces from patients with colorectal carcinomas (Duke's A or B red; Duke's C or D, blue). The dotted line represents 2% methylated fragments.
Fig. 5 (supp. Fig. 1) Correlation between the results of Methyl-BEAMing and the fraction of methylated fragments used as templates.
Fig. 6. (supp. Fig. 2) Genome equivalents of total DNA in 2 ml plasma from the indicated subgroups of patients.
Fig. 7 (supp. Fig. 3): Genome equivalents of total DNA in 4 g stool from the indicated subgroups of patients.

### DETAILED DESCRIPTION OF THE INVENTION

The inventors have developed a technology for direct quantification of DNA methylation at specific sites that is readily applicable to clinical samples, even when the fraction of methylated fragments in such samples is minute. This approach can be applied to determine the extent of methylation of marker genes in plasma and fecal DNA from cancer patients and healthy controls.

Beads are also known as microspheres or microparticles. Particle sizes can vary between about 0.1 and 10 microns in diameter. Typically beads are made of a polymeric material, such as polystyrene, although nonpolymeric materials such as silica can also be used. Other materials which can be used include styrene copolymers, methyl methacrylate, functionalized polystyrene, glass, silicon, and carboxylate. Optionally the particles are superparamagnetic, which facilitates their purification after being used in reactions.

Beads can be modified by covalent or non-covalent interactions with other materials, either to alter gross surface properties, such as hydrophobicity or hydrophilicity, or to attach molecules that impart binding specificity. Such molecules include without limitation, antibodies, ligands, members of a specific-binding protein pair, receptors, nucleic acids. Specific-binding protein pairs include avidin-biotin, streptavidin-biotin, and Factor VII-Tissue Factor. Primers can be attached via spacer moieties. The spacer moieties may be oligonucleotides.

Beads, after being prepared according to the present invention as product beads, have more than one copy of the same nucleic acid molecule bound to them. Preferably each bead is bound to at least 10, 50, 100, 500, 1000, or 10,000 molecules of the same nucleic acid sequence. In some circumstances some of the product beads are bound to more than one type of nucleic acid molecule. These product beads are generally less useful in the analysis of ratios of methylated sequences in a population of sequences. Such product beads can be readily discriminated and so do not distort the analysis.

A population of product beads will often comprise two or more types of nucleic acids. Such a population is heterogeneous with respect to the nucleic acids. Desirably, a substantial proportion of the product beads comprise only one type of nucleic acid per bead. A substantial proportion can be for example, at least I %, at least 5 %, at least 10 %, or at least 50 %. A product bead with only one type of nucleic acid per bead is termed homogeneous. A product bead with two types of nucleic acid per bead is termed heterogeneous. Heterogeneous product beads may result from aqueous compartments which have more than two molecules of template of non-identical sequence. This could result, for example from incomplete modification of methylated cytosine residues. A population of product beads can be heterogeneous as a population but contain individual product beads that are homogeneous.

Individual product beads preferably comprise more than one copy of template analyte molecule. Each bead may comprise at least 10, at least 50, at least 100, at least 500, at least 1000, or at least 10,000 copies of template analyte. If the bead is homogeneous, each of those copies will be identical.

Populations of product beads can be maintained in a liquid suspension. Alternatively they can be sedimented and dried or frozen. The latter alternatives may be beneficial for storage stability.

Analysis of populations of product beads can be used for distinguishing between methylated and non-methylated or hypomethylated templates. Polynucleotides can be distinguished which differ by as little as a single methylated residue, although differences in multiple residues may by better for detection sensitivity. Increased sensitivity obtained using multiple residues may decrease specificity, however.

One very convenient way for distinguishing between the products of methylated and non-methylated CpG islands is by differentially labeling probes to the expected products with fluorescent dyes. Such labeling can be accomplished by hybridization of a fluorescently labeled oligonucleotide probe to one species of polynucleotide. Alternatively, a fluorescently labeled antibody can be used to specifically attach to one oligonucleotide probe that hybridizes to a particular product. Such antibody binding can be, for example, mediated by a protein or polypeptide which is attached to an oligonucleotide hybridization probe. Of course, other means of labeling polynucleotides as are known in the art can be used without limitation. Another means of labeling different polynucleotide species is by primer extension. Primers can be extended using labeled deoxyribonucleotides, such as fluorescently labeled deoxyribonucleotides.

Populations of product beads can be used as templates. Template analyte molecules on the product beads can be analyzed to assess DNA sequence variations by hybridization, primer-extension methods, mass spectroscopy, and other methods commonly used in the art. Template analyte molecules on product beads can be employed for solid phase sequencing. In one solid phase sequencing technique, product beads are arrayed by placing them on slides spotted with complementary oligonucleotides. In another solid phase sequencing technique, product beads are placed into individual wells. In still another solid phase sequencing technique product beads are incorporated into acrylamide matrices (with or without subsequent polony formation). Sequencing reactions can be performed with any solid phase sequencing method, such as those using unlabeled nucleotide precursors (e.g., pyrosequencing, as described in Ronaghi et al., Anal. Biochem. 267: 65-71, 1999) or labeled nucleotides (*e.g.,* photocleavable reagents described by Mitra et al., Anal. Biochem. 320:55-65, 2003). Product beads can thus be used for and facilitate massively parallel sequencing. Product beads can also be used in sequencing employing Type IIS restriction endonucleases. Product beads can also be used to provide templates for conventional dideoxynucleotide sequencing. To obtain useful data upon sequence analysis, a homogeneous template population is desirable. To provide a homogenous template population, product beads can be diluted, separated, or otherwise isolated so that each sequencing reaction contains a single product bead. Alternatively, product beads can be sorted to provide populations of beads with a single species of template.

Oligonucleotide primers can be bound to beads by any means known in the art. They can be bound covalently or non-covalently. They can be bound via an intermediary, such as via a protein-protein interaction, such as an antibody-antigen interaction or a biotin-avidin interaction. Other specific binding pairs as are known in the art can be used as well. To achieve optimum amplification, primers bound to the bead may be longer than necessary in a homogeneous, liquid phase reaction. Oligonucleotide primers may be at least 12, at least 15, at least 18, at least 25, at least 35, or at least 45 nucleotides in length. The length of the oligonucleotide primers which are bound to the beads need not be identical to that of the primers that are in the liquid phase. Primers can be used in any type of amplification reaction known in the art, including without limitation, polymerase chain reaction, isothermal amplification, rolling circle amplification, self-sustaining sequence replication (3SR), nucleic acid sequence-based amplification (NASBA), transcription-mediated amplification (TMA), strand-displacement amplification (SDA), and ligase chain reaction (LCR). Primers can be designed to hybridize only to modified, only to non-modified, or to both modified and non-modified sequences. Mixtures of primer sequences can be used to capture variants.

Microemulsions are made by stirring or agitation of oil, aqueous phase, and detergent. The microemulsions form small aqueous compartments (nanocompartments) which have an average diameter of 0.5 to 50 microns. The compartments may be from 1 to 10 microns, inclusive, from 11 to 100 microns, inclusive, or about 5 microns, on average. All such compartments need not comprise a bead. Desirably, at least one in 10,000 of said aqueous compartments comprise a bead. Typically from 1/100 to 1/1 or from 1/50 to 1/1 of said aqueous compartments comprise a bead. In order to maximize the proportion of beads which are homogeneous with respect to oligonucleotide, it is desirable that on average, each aqueous compartment contains less than 1 template molecule. Aqueous compartments will also desirably contain whatever reagents and enzymes are necessary to carry out amplification, For example, for polymerase chain reaction (PCR) the compartments will desirably contain a DNA polymerase and deoxyribonucleotides. For rolling circle amplification a DNA polymerase and a generic DNA circle may be present.

Emulsions can be "broken" or disrupted by any means known in the art. One particularly simple way to break the emulsions is to add more detergent. Detergents which can be used include, but are not limited to Triton X100, Laureth 4, Nonidet.

Sample DNA for amplification and analysis according to the present invention can be genomic DNA. Samples can be derived from a single individual, for example, from a body sample such as urine, blood, serum, plasma, sputum, stool, tissue or saliva. Samples can also be derived from a population of individuals. The individuals can be humans, but can be any organism, plant or animal, eukaryotic or prokaryotic, viral or non-viral.

Any type of probe can be used for specific hybridization to the amplified polynucleotides which are bound to the beads. Fluorescently labeled probes are useful because their analysis can be automated and can achieve high throughput. Fluorescence activated cell sorting (FACS) permits both the analysis and the isolation of different populations of beads. One type of fluorescently labeled probe that can be used is a modified molecular beacon probe. These probes have stem-loop structures and an attached fluorescent moiety on the probe, typically on one end of the probe, sometimes attached through a linker. Unlike standard molecular beacon probes, modified molecular beacon probes do not have a quenching moiety. The modified molecular beacon probe can have the fluorescent moiety attached on either end of the probe, 5' or 3'. One such probe will hybridize better to a sequence from a methylated gene than to a non-methylated gene. Another such probe will hybridize better to a sequence from a non-methylated gene than to a sequence from the methylated gene.

Microemulsions or nanocompartments are formed with beads and primers. Because BEAMing requires thermal cycling, an emulsifier which is thermostable can optionally be used. One such emulsifier is Abil® EM90 (Deguss -Goldschmidt Chemical, Hopewell, VA). Other such emulsifiers can be used as are known in the art.

Chemical reagents can be used which selectively modify either the methylated or non-methylated form of CpG dinucleotide motifs. Alternatively, methylation-sensitive restriction endonucleases can be used to detect methylated CpG dinucleotide motifs. Such endonucleases may either impartially cleave within methylated CpG recognition sites relative to non-methylated CpG recognition sites or preferentially cleave within non-methylated relative to methylated CpG recognition sites. Examples of the former are Acc III, BstN I, and Msp I. Examples of the latter are Acc II, Ava I, BssH II, BstU I, Hpa II, and Not I.

Modified products can be detected directly, or after a further reaction which creates products which are easily distinguishable. Means which detect altered size and/or charge can be used to detect modified products, including but not limited to electrophoresis, chromatography, and mass spectrometry. Examples of such chemical reagents for selective modification include hydrazine and bisulfite ions. Hydrazine-modified DNA can be treated with piperidine to cleave it. Bisulfite ion-treated DNA can be treated with alkali.

One way to distinguish between modified and nonmodified DNA is to use oligonucleotide probes which are specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

Test cells can be obtained from surgical samples, such as biopsies or fine needle aspirates, from paraffin embedded tissues, from a body fluid such as bone marrow, blood, serum, plasma, lymph, cerebrospinal fluid, saliva, sputum, stool, urine, or semen. Such sources are not meant to be exhaustive, but rather exemplary.

In one aspect of this embodiment, a gene can be contacted with hydrazine, which modifies cytosine residues, but not methylated cytosine residues, then the hydrazine treated gene sequence is contacted with a reagent such as piperidine, which cleaves the nucleic acid molecule at hydrazine modified cytosine residues, thereby generating a product comprising fragments. The fragments can then be used in amplification reactions and the products analyzed.

Bisulfite ions, for example, sodium bisulfite, convert non-methylated cytosine residues to bisulfite modified cytosine residues. The bisulfite ion treated gene sequence can be exposed to alkaline conditions, which convert bisulfite modified cytosine residues to uracil residues. Sodium bisulfite reacts readily with the 5,6-double bond of cytosine (but poorly with methylated cytosine) to form a sulfonated cytosine reaction intermediate that is susceptible to deamination, giving rise to a sulfonated uracil. The sulfonate group can be removed by exposure to alkaline conditions, resulting in the formation of uracil. The DNA can be amplified, for example, by PCR, and sequenced to determine whether CpG sites are methylated in the DNA of the sample . Uracil is recognized as a thymine by Taq polymerase and, upon PCR, the resultant product contains cytosine only at the position where 5-methylcytosine was present in the starting template DNA. One can compare the amount or distribution of uracil residues in the bisulfite ion treated gene sequence of the test cell with a similarly treated corresponding non-methylated gene sequence. A decrease in the amount or distribution of uracil residues in the gene from the test cell indicates methylation of cytosine residues in CpG dinucleotides in the gene of the test cell. The amount or distribution of uracil residues also can be detected by contacting the bisulfite ion treated target gene sequence, following exposure to alkaline conditions, with an oligonucleotide that selectively hybridizes to a nucleotide sequence of the target gene that either contains uracil residues or that lacks uracil residues, but not both, and detecting selective hybridization (or the absence thereof) of the oligonucleotide.

Methyl-BEAMing can quantitatively assess DNA methylation in clinical samples. In addition to its potential value as a platform for clinical diagnosis, these studies have illuminated several features with important implications for the use of DNA methylation in cancer diagnostics, regardless of the platform used. First, the correspondence between the number of methylated vimentin DNA fragments and mutant APC and PIK3CA DNA fragments in the plasma samples from cancer patients provides strong evidence that circulating methylated DNA fragments are derived from the cancer cells themselves. As DNA methylation is plastic within mammalian cells, it was possible that such circulating DNA was derived from non-neoplastic stromal or inflammatory cells adjacent to the tumor or from elsewhere in the host. As far as we know, this is the first study to provide evidence of this kind, as it requires quantitative measures of both methylation and mutation to make the argument.

Second, our results demonstrate that several methylation sites within a region of interest should be concurrently assessed to achieve the required sensitivity and specificity in a diagnostic test based on methylation. This conclusion is based on the fact that even with carefully optimized bisulfite treatments, ∼0.5% of individual cytosines are unconverted. PCR errors can further contribute to this background level³². As less than 1 % of the DNA in some clinical samples, such as those from plasma, are derived from tumors, it is critical to be able to differentiate biologically methylated molecules from such technical artifacts.

Third, our results demonstrate that the fraction of methylated fragments derived from cancers is higher in fecal DNA than in plasma DNA. Moreover, the fraction of methylated DNA fragments derived from adenomas is sufficiently high in fecal DNA to be detected (Table 2). We did not attempt to detect circulating DNA in adenoma patients, as previous studies have shown that there is very little, if any, circulating DNA derived from pre-malignant lesions of the colon²². This result suggests that fecal DNA is the preferred analyte for colorectal cancer screening in methylation assays. However, the fraction of methylated vimentin molecules from normal individuals is substantially higher in feces than in plasma (mean fraction of 0.96% and 0.025% in feces and plasma, respectively). It is known that DNA methylation is tissue specific, and normal cells within the gastrointestinal tract presumably contribute more methylated DNA to the feces than to the circulation. This background limits the sensitivity and specificity of Methyl-BEAMing of fecal DNA by raising the threshold for positivity.

The ability to quantitatively measure the fraction of methylated molecules via Methyl-BEAMing provides several diagnostic opportunities. There is nearly a 2000-fold difference between the concentrations of methylated vimentin molecules in the plasma of advanced cancer patients compared to normal individuals (Table 1). This difference could easily be translated into simple tests for assessing the extent of disease following therapeutic procedures ³⁰. One could also imagine a prenatal test for Down's Syndrome based on the assessment of methylation of DNA fragments of chromosome 21³³. The development of such a test would require discovery of sequences that are methylated in the fetus but not in adults. Both these applications require a quantitative, rather than qualitative, assessment of DNA methylation such as afforded by Methyl-BEAMing. Though sequencing-by-synthesis instruments, such as the one employed in this study, also provide such quantification, they are not well-suited for high-throughput clinical analyses because of relatively long process times and high cost.

One of the most important applications of Methyl-BEAMing is in early diagnosis. In this study, 59 % of patients with colorectal cancer could be detected by a plasma-based Methyl-BEAMing assay. Note that this sensitivity is close to the maximal detectable with the vimentin biomarker, which has been shown to be methylated in 53-83 % of colorectal cancers²⁵. The fact that 50 % of presumably curable Duke's A and B stage cancers could be detected by this method was particularly encouraging.

Equally encouraging was the observation that 45% of patients with clinically significant pre-malignancies could be detected by Methyl-BEAMing of fecal DNA. Patients with these lesions would generally be treated by endoscopic excision without abdominal surgery. In screening an asymptomatic cohort of adults over age 50, the prevalence of colon cancers and of adenomas ≥ 1 centimeter is expected to be 0.5-1.0 % and 6.0 %, respectively^{34, 35}. Detection of 41% of cancers and 45 % of adenomas by Methyl-BEAMing of stool DNA, at 95 % specificity, would translate into a positive predictive value of 40%. This value exceeds that achieved in many studies of mammography, a cancer screening test of established clinical value in reducing mortality³⁶.

How could the Methyl-BEAMing approach be improved? As it could in principle be applied to any gene that is hypermethylated in cancers, the sensitivity for early-stage tumors could easily be raised by including one or a few additional genes in Methyl-BEAMing assays. The specificities of plasma-based and fecal-based Methyl-BEAMing assays were 95 % and 92 %, respectively. Though specificities in this range are typical of diagnostic screening tests that are widely used³⁶, the inclusion of additional genes in a methylation panel would undoubtedly lower the aggregate specificity. One challenge is therefore the identification of sequences in addition to vimentin that are methylated in tumor DNA but very rarely methylated in any normal adult cells. We have tested several commonly used methylated biomarkers in the context of selecting a biomarker that would be useful for testing in a body fluid and found most of them to be unsuitable. Hopefully, currently ongoing genome-wide epigenetic projects will identify new, more specific methylated sequences in the future^{37, 38}. Another potential enhancement would involve the combined analysis of vimentin methylation with mutations of commonly mutated genes, such as KRAS, PIK3CA and TP53, which could in theory increase sensitivity without compromising specificity.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLE 1

### Methods

Sample collection and DNA purification. Plasma samples were collected by study nurses of Indivumed GmbH, Hamburg, Germany, from surgery patients treated in hospitals of Indivumeds' collaborative network, in particular from patients at the Israelitic Hospital and Clinic Alten Eichen (both in Hamburg, Germany) following strictly controlled SOP criteria. IRB approval was provided by the Ethical Board of the Physicians Association of Hamburg and patient samples and data were collected only after informed, written consent was obtained. The samples used in the current study were randomly chosen from those contributing through this protocol. Shortly before surgery, 18 ml of blood was taken from a central catheter and loaded into a standard blood collection tube containing EDTA. The tube was immediately chilled to 8 °C and transported to the laboratories within 30 min for plasma preparation. The blood cells were pelleted for 15 min at 200 g in a Leucosep tube (Greiner, Frickenhausen, Germany) filled with 15 ml of Ficoll-Paque solution. After centrifugation, the supernatant (i.e., plasma) was transferred into 1.5 ml tubes, immediately frozen, and stored at -80 °C. The plasma samples were thawed at room temperature for 5 min, and any remaining debris was pelleted at 16,000 g for 5 min. The supernatant was transferred to a new tube. DNA was purified from the plasma supernatant using the QIAamp MinElute Virus Vacuum Kit (Qiagen) as recommended by the manufacturer. The DNA was eluted in EB buffer (Qiagen), and stored at -20 °C.

Stool samples and clinical data were collected from patients undergoing colonoscopy at Aarhus University Hospital. The study was approved by the Regional Scientific Ethics Committee, Aarhus, Denmark and by the Danish Data Protection Agency and informed, written consent was obtained in every case. Stool samples were collected on a weekday before bowel preparation for colonoscopy or operation for colorectal cancer. The patients placed the container in an insulated box containing frozen bags which had been stored in a freezer at least 24 hours. The box was collected at the patient's home within eight hours. Immediately after the stool sample arrived at Aarhus University Hospital, the container was frozen at -80 °C until it was thawed for DNA purification. Stool samples were homogenized with a stool shaker (Exact Sciences Corp, Marlborough, MA). After homogenization, a 4 g stool equivalent of each sample was subjected to 2 centrifugations (5 min at 2536 g and 10 min at 16,500 g). The supernatant was incubated with 20 µL RNase (0.5 mg/mL) for one hour at 37 °C, followed by precipitation with 1/10 volume of sodium acetate (3 M) and an equal volume of isopropanol. Stool DNA was dissolved in 4 mL of TE buffer and prepared for electrophoretic capture by mixing with 4 mL of water, 1 mL of 10× TBE (890 mM Tris base, 890 mM boric acid, 20 mM EDTA), and 1 mL of 10% SDS. DNA samples were denatured at 95 °C for 15 min and then cooled on ice for 15 min. Vimentin fragments were then purified using a Reversible Electrophoretic Capture Affinity Protocol (RFCAP)³¹ . This was accomplished by repeatedly driving the DNA back and forth through the capture device loaded with oligonucleotide probes for the Vimentin gene target (capture probe sequences were 5'-Biotin-CGCCACCCTCCGCAGCCATGTCCACCAGGTCCG-3' (SEQ ID NO: 4) and 5'-Biotin-GGCCATCGCCACCCTCCGCAGCCATGTCCACCAGG-3'(SEQ ID NO: 5)). After completion of electrophoretic cycling, the capture device was moved to a wash plate and washed four times with 500 µL of ST buffer (150 mM NaCl, 15 mM Tris-HCl, pH 7.4). The captured human DNA was eluted by adding 100 µL of 0.1 M NaOH to the capture device. The eluate was then neutralized with 10 µL of neutralization buffer (60 mM Tris pH 9.0, 6 mM EDTA, 0.5 M HCl).

Real-Time PCR. The amount of DNA per 2 ml plasma samples was quantified using a modified version of a human LINE-1 quantitative real-time PCR assay 39. Two primer sets were designed to amplify differently sized regions within the most abundant consensus region of the human LINE-1 family (for the 91-bp product, the primers were 5'-TGGCACATATACACCATGGAA-3'(SEQ ID NO: 6) and 5'-TGATGGTTTCCAATTTCATCC-3'(SEQ ID NO: 7); for the 69-bp product, the primers were: 5'-TGGCACATATACACCATGGAA-3' (SEQ ID NO: 8) and 5'-TGTCCCTACAAAGGATATGAACTC-3' (SEQ ID NO: 9)). PCR was performed in 25 µL reaction volumes consisting of template DNA equivalent to 25 µL of plasma, 0.5 U of Platinum Taq DNA Polymerase, 1× PCR buffer (see above), 6% (v/v) DMSO, 1 mM of each dNTP, 1:100,000 dilution of SYBR Green I (Invitrogen), and 0.2 µM of each primer. Amplification was carried out in an iCycler (Bio-Rad) using the following cycling conditions: 94 °C for 2 min; 3 cycles of 94 °C for 10 s, 67 °C for 15 s, 70 °C for 15 s; 3 cycles of 94 °C for 10 s, 64 °C for 15 s, 70 °C for 15 s; 3 cycles of 94 °C for 10 s, 61 °C for 15 s, 70 °C for 15 s; 35 cycles of 94 °C for 10 s, 58 °C for 15 s, 70 °C for 15 s. Various dilutions of normal human lymphocyte DNA were incorporated into each plate to serve as standards (0.006 to 1.56 ng). The threshold cycle number was determined using Bio-Rad analysis software. Each quantification for each LINE-1 product was performed in duplicate and the average value was used to determine the number of genome equivalents in the sample by comparison to the standards.

The amount of DNA per 4 g stool samples was quantified by real-time PCR assay using three sets of Vimentin specific primers (for the 81-bp product, the primers were 5'-GGGTGGACGTAGTCACGTAG-3 (SEQ ID NO: 10)and 5'-CCTCCTACCGCAGGATGTT-3' (SEQ ID NO: 11); for the 91-bp product, the primers were 5'-CTGTAGGTGCGGGTGGAC-3' (SEQ ID NO: 12) and 5'-CCTCCTACCGCAGGATGTT-3' (SEQ ID NO: 13); for the 100-bp product, the primers were 5'-CCTCCTACCGCAGGATGTT-3' (SEQ ID NO: 14) and 5'-CTGCCCAGGCTGTAGGTG-3' (SEQ ID NO: 15)). PCR was performed in 25 µL reaction volumes consisting of template DNA equivalent to 0.016g stool, 0.5 U of Platinum Taq DNA Polymerase, 1× PCR buffer , 6 % (v/v) DMSO, 1 mM of each dNTP, 1:100,000 dilution of SYBR Green I (Invitrogen), and 0.2 µM of each primer. Amplification was carried out in an iCycler (Bio-Rad) using the following cycling conditions: 94 °C for 2 min; 3 cycles of 94 °C for 10 s, 67 °C for 15 s, 70 °C for 15 s; 3 cycles of 94 °C for 10 s, 64 °C for 15 s, 70 °C for 15 s; 3 cycles of 94 °C for 10 s, 61 °C for 15 s, 70 °C for 15 s; 35 cycles of 94 °C for 10 s, 59 °C for 15 s, 70 °C for 15 s. Various dilutions of normal human lymphocyte DNA were incorporated in each plate to serve as standards (0.39 to 25 ng). The threshold cycle number was determined using Bio-Rad analysis software. Each quantification for each Vimentin fragment was performed in duplicate and the average value was used to determine the number of genome equivalents in the sample by comparison to the standards.

Bisulfite conversion. DNA isolated from plasma and stool was treated with bisulfite using an Epitect Bisulfite Conversion kit (Qiagen), modified for isolating degraded DNA such as that found in plasma or stool. In brief, each bisulfite conversion reaction contained 18 µl purified DNA solution, 2 µl 0.5 g/ml single-stranded sonicated salmon sperm DNA (5S DNA), 85 µl bisulfite mix and 35 µl DNA protect buffer. Bisulfite conversion was carried out in a thermocycler at 99 °C for 5 min, 60 °C for 25 min, 99 °C for 5 min, 60 °C for 85 min, 99 °C for 5 min, and 60 °C for 175 min. The cleanup of bisulfited converted DNA followed the manufacturer's recommendations for formalin-fixed, paraffin-embedded (FFPE) DNA samples with the modification that MinElute Spin Columns (Qiagen) instead of Epitect spin columns were used for DNA disulfonation and purification. Bisulfite treated DNA was eluted into EB buffer (Qiagen) and stored at -20 °C.

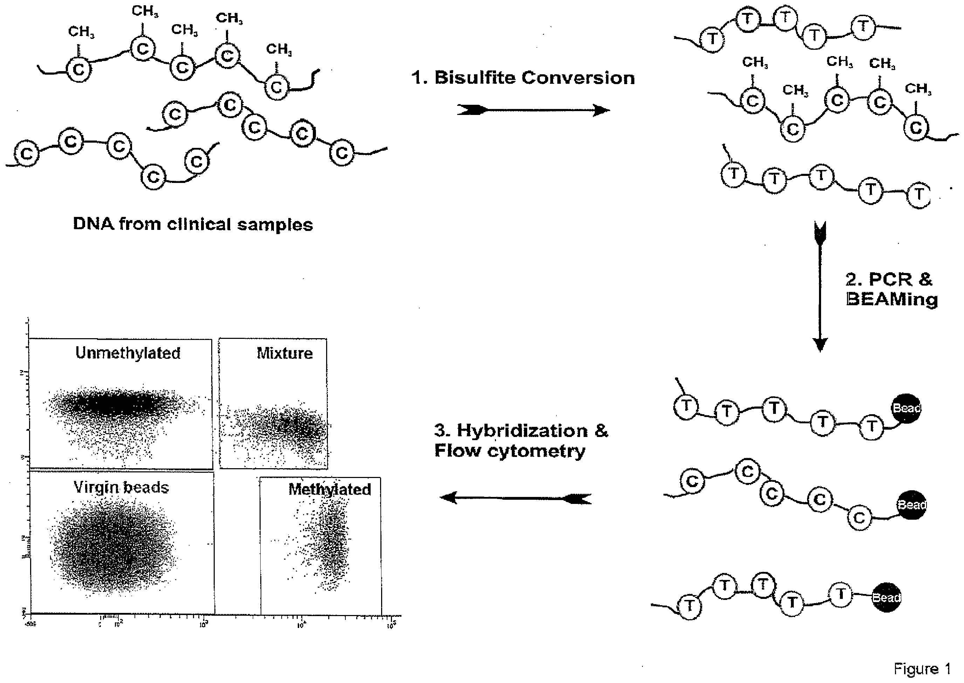

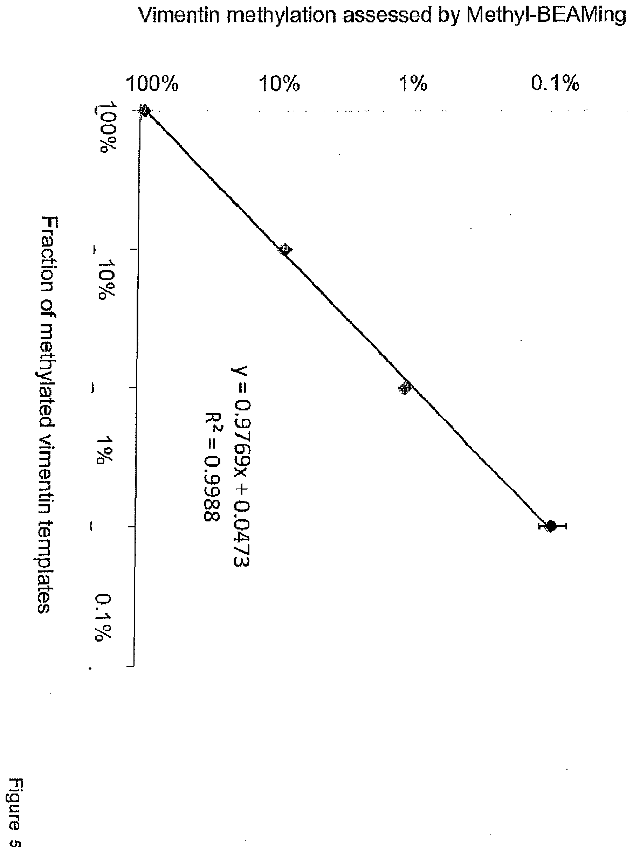

PCR amplification of bisulfite converted DNA. PCR was carried out by using a mixture of four primers that amplified both the methylated and the unmethylated Vimentin sequences (primers for methylated sequences were 5'-tcccgcgaaattaatacgacCTCGTCCTCCTACCGCAAAATATTC-3' (SEQ ID NO: 16) and 5'-GTTGTTTAGGTTGTAGGTGCGGG-3' (SEQ ID NO: 17); primers for unmethylated sequences were 5'-tcccgcgaaattaatacgacCTCATCCTCCTACCACAAAATATTC-3' (SEQ ID NO: 18) and 5'-GTTGTTTAGGTTGTAGGTOTGGG-3' (SEQ ID NO: 19)). PCR was performed in 50 µL reaction volumes consisting of 8 µl bisulfite-converted DNA, .2.5 U of AmpliTaq Gold DNA Polymerase (Applied Biosystems), 1× PCR Gold Buffer, ImM MgCl₂, 0.2 mM dNTP mix, 0.1 uM of each primer. Amplification was carried out in a Thermo Hybrid thermocycler using the following conditions: 95 °C for 5 min; 3 cycles of 95 °C for 45 s, 67 °C for 45 s, 72 °C for 45 s; 3 cycles of 95 °C for 45 s, 64 °C for 45 s, 72 °C for 45 s; 3 cycles of 95 °C for 45 s, 61 °C for 45 s, 72 °C for 45 s; 45 cycles of 95 °C for 45 s, 58 °C for 45 s, 72 °C for 45 s; 1 cycle of 72 °C for 10 min. Note that these primers and PCR conditions result in a preferential amplification of methylated sequences ⁴⁰, This preferential amplification was confirmed using mixtures of fully methylated and fully unmethylated sequences (Fig. 5; supp, fig. 1). Values for the fraction of methylated fragments found in the plasma and stool were accordingly adjusted (*i.e*., the actual fractions of methylated Vimentin fragments determined experimentally were divided by 7). This normalization had no effect on the calculated sensitivity or specificity because all samples were adjusted equivalently.

BEAMing. Emulsion PCR was performed using modifications of the procedures described by Diehl et al. ²⁹. We first prepared a 150 µL PCR mixture containing 15 pg template DNA, 45 U of Platinum Taq DNA polymerase (Invitrogen), 1× PCR buffer (67 mM Tris-HCl (pH 8.8), 16.6 mM (NH4)2SO4, 10 mM β-mercaptoethanol, 1.17 mM MgC12), 0.2 mM dNTPs, 5 mM MgCl2, 0.05 µM universal forward primer (5'-tcccgcgaaattaatacgac-3' (SEQ ID NO: 20)), 8 µM Vimentin specific reverse primers (5'-GTTGTTTAGGTTGTAGGTGCGGG-3' (SEQ ID NO: 21) and 5'-GTTGTTTAGGTTGTAGGTGTGGG-3' (SEQ ID NO: 22)) and 6×107 magnetic streptavidin beads (MyOne, Invitrogen) coated with universal forward primer (5'-dual biotin-T-Spacer18-tcccgcgaaattaatacgac-3' (SEQ ID NO: 23)). This PCR reaction mixture was combined with 600 µL of an oil/emulsifier mix (7% ABIL WE09, 20% mineral oil, 73% Tegosoft DEC; Degussa Goldschmidt Chemical, Hopewell, VA) and one 5 mm steel bead (Qiagen) in one well of a 96 deep-well plate (Abgene). Emulsions were prepared by shaking the plate in a TissueLyser (Qiagen) for 10 s at 15 Hz and 7 s at 17 Hz and dispensed into eight wells of a 96 well PCR plate. Temperature cycling was performed at 94 °C for 2 min; 3 cycles of 94 °C for 10 s, 68 °C for 45 s, 70 °C for 75 s; 3 cycles of 94 °C for 10 s, 65 °C for 45 s, 70 °C for 75 s; 3 cycles of 94 °C for 10 s, 62 °C for 45 s, 70 °C for 75 s; 50 cycles of 94 °C for 10 s, 59 °C for 45 s, 70 °C for 75 s.

To break the emulsions, 150 µL breaking buffer (10 mM Tris-HCl, pH 7.5, 1% Triton-X 100, 1% SDS, 100 mM NaCl, 1 mM EDTA) was added to each well and mixed in a TissueLyser at 20 Hz for 20 s. Beads were recovered by centrifuging the suspension at 3200 g for 2 min and removing the oil phase. The breaking step was repeated twice. All beads from eight PCR wells were combined and resuspended in 100 µL wash buffer (20 mM Tris-HCl, pH 8.4, 50 mM KCl). The DNA on the beads was denatured for 5 min with 0.1 M NaOH. Finally, beads were washed with 100 µL wash buffer and resuspended in 100 µL of wash buffer.

Probe hybridization. To determine the fraction of beads containing PCR products from methylated DNA templates, 0.5 uM Cy5-labeled oligonucleotide (5'-Cy5-TCGGTCGGTTCGCGGTGTTCGA-3' (SEQ ID NO: 24), complimentary to the bisulfite converted methylated sequence), and 0.5 uM FITC labeled oligonucleotide (5'-FAM-TTGGTTGGTTTGTGGTGTTTGA-3' (SEQ ID NO: 25), complimentary to the bisulfite converted unmethylated sequence) were hybridized to the DNA on ∼107 beads in 100 µl hybridization buffer (30 mM Tris-HCl, pH 9.5, 13.4 mM MgCl2, 5% formamide) at 50 °C for 15 minutes. Beads were then collected with a magnet and resuspended in 200 µl 10 mM Tris, pH 7.5, 1 mM EDTA, pH 7.5.

Flow Cytometry. Beads were analyzed with a LSR II flow cytometer and data were analyzed with FACSDiva software (BD Biosciences). The flow rate was typically set at 5,000-10,000 events per second. Events were gated to exclude doublets and aggregates.

Massively parallel sequencing-by-synthesis. DNA isolated from normal lymphocytes (control) and stool was treated with bisulfite as described above. The converted DNA was PCR amplified using the same protocol as used for Methyl-BEAMing. The PCR product was purified with a QIAquick PCR purification column (Qiagen) and used to construct libraries for Solexa sequencing following Illumina's standard DNA sample preparation instructions. Briefly, (1) the PCR products were blunt-ended with T4 DNA polymerase, Klenow polymerase, and T4 polynucleotide kinase; (2) a dA was added to the 3' end of each strand with Klenow (exo-) polymerase; (3) adapters designed for library construction were ligated to the PCR fragments; (4) ligation products were gel-purified to select for ∼180bp fragments; and (5) PCR amplification was performed to enrich ligated fragments. The amplified library was quantified by real-time PCR and was denatured with 0.1 M NaOH to generate single-stranded DNA molecules, captured on Illumina flow cells, and amplified in situ. A Vimentin-specific oligonucleotide (5-GTGC/TGGGTGGAC/TGTAGTTAC/TGTAGT TTC/TGGTTGGA-3 (SEQ ID NO: 26), C/T indicates that the position was an equimolar mix of cytosine and thymidine) was used to prime sequencing for 36 cycles on an Illumina Genome Analyzer. Data analysis: The sequences generated were aligned to the bisulfite converted unmethylated Vimentin sequence. The following criterions were used to filter the tags for further analysis. Each tag was required (i) to pass the Illumina chastity filter; (ii) to have an average Phred sequence quality score above 20; and (iii) to have perfect match to the reference sequence at A,G, and T positions (allowing mismatched at C positions). At least 80,000 tags that met these criteria were evaluated in each case,

### EXAMPLE 2

### Technical overview of Methyl-BEAMing

The first hurdle to overcome in developing Methyl-BEAMing was bisulfite conversion. DNA in clinical samples such as plasma is already degraded to small size by circulating nucleases and is present at only a few nanograms per mililiter²². Conventional methods for bisulfite conversion further degrade DNA and are difficult to implement with samples containing only small amounts of DNA because of cumulative losses during the procedure^{23, 24}. After much optimization, we identified conditions that resulted in nearly complete (Mean + SEM: 99.4% + 0.4%) conversion of dC to dU residues while preserving the majority of the initial DNA in a fashion permitting subsequent PCR amplification (see Experimental Methods for details).

The exon 1 region of the vimentin gene has been shown to be hypermethylated in colorectal cancers when compared to normal colorectal mucosae and other normal tissues^{25, 26}. Moreover, this difference is the basis for the only commercially available diagnostic test based on DNA methylation (ColoSure, LabCorp). We therefore chose this region of vimentin to assess the dynamic range and accuracy of Methyl-BEAMing. The region of vimentin queried contains 5'- CpG sites that have been shown to be methylated in cancer cells, Primers surrounding this region were designed to amplify it, whether it was methylated or unmethylated, following bisulfite conversion. The PCR products were only ∼100 bp in length so as to accommodate the small size of circulating DNA molecules^{22, 27}. These amplicons were then used for BEAMing (Beads, Emulsion, Amplification and Magnetics)^{28, 29}.

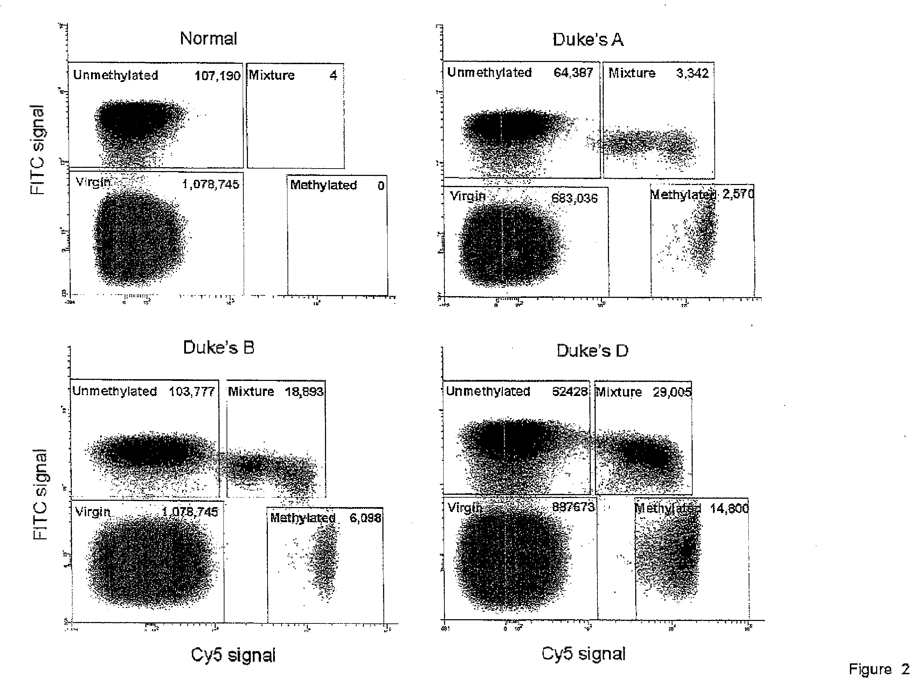

BEAMing employs aqueous nanocompartments suspended in a continuous oil phase (Fig. 1). Each aqueous nanocompartment contains DNA polymerase, cofactors, and dNTP's required for PCR. When a compartment contains a single DNA template molecule as well as a bead, the PCR product within the compartment becomes bound to the bead. Each bead thereby ends up with thousands of identical copies of the template within its nanocompartment - a process similar to that resulting from cloning an individual DNA fragment into a plasmid vector to form a bacterial colony. After PCR, the beads are collected by breaking the emulsion and incubated with fluorescent probes that specifically hybridize to either methylated or unmethylated sequences. Flow cytometry then provides an accurate read-out of the fraction of original template molecules that were methylated or unmethylated within the queried sequence (examples in Fig. 2).

As a prelude to the experiments reported below, we tested Methyl-BEAMing on mixtures of templates representing DNA from peripheral blood lymphocytes (unmethylated vimentin) and a colorectal cancer cell line (fully methylated vimentin). We found that the fraction of beads containing methylated vimentin sequences was directly proportional to the fraction of methylated input DNA when the latter represented 0.1% to 100% of the input DNA (R2-1.00, Fig. 5; supp. fig. 1).

### EXAMPLE 3

### Methylated vimentin gene fragments in the circulation come from cancer cells

Some of the most important uses of cancer biomarkers involve the assessment of circulating molecules, either for early detection or disease-monitoring following therapy. To use methyl-BEAMing for such purposes, or indeed to use any test based on DNA methylation, it is assumed that the methylated molecules in the circulation emanate from cancer cells. To explicitly test this assumption, we compared the fraction of methylated vimentin molecules with the fraction of mutant molecules (APC G4189T and PIK3CA G 1624A) in the plasma of two colorectal cancer patients. In these patients, the mutations were somatic and therefore exclusively present in the patient's neoplastic cells. Methyl-BEAMing was used to assess methylation and standard BEAMing was used to assess mutations³⁰. The fraction of methylated vimentin templates in the first sample was 13.6%, quite similar to the fraction of mutated APC (17.5%). In the second patient's plasma, methylated vimentin represented 3.5% of the total vimentin templates while mutated PIK3CA represented 3.0% of the PIK3CA templates.

### EXAMPLE 4

### Methyl-BEAMing of circulating DNA

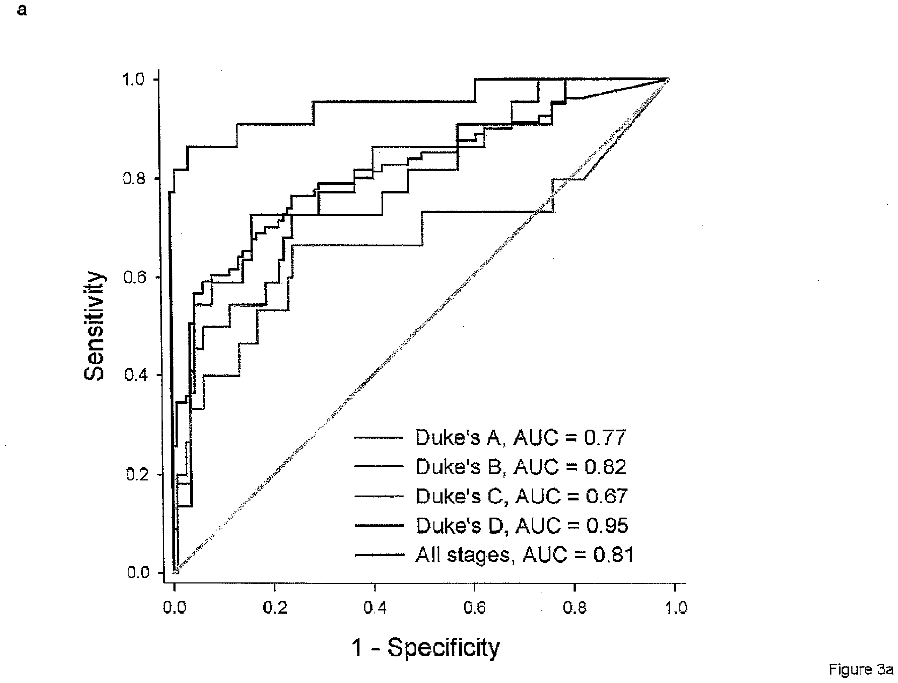

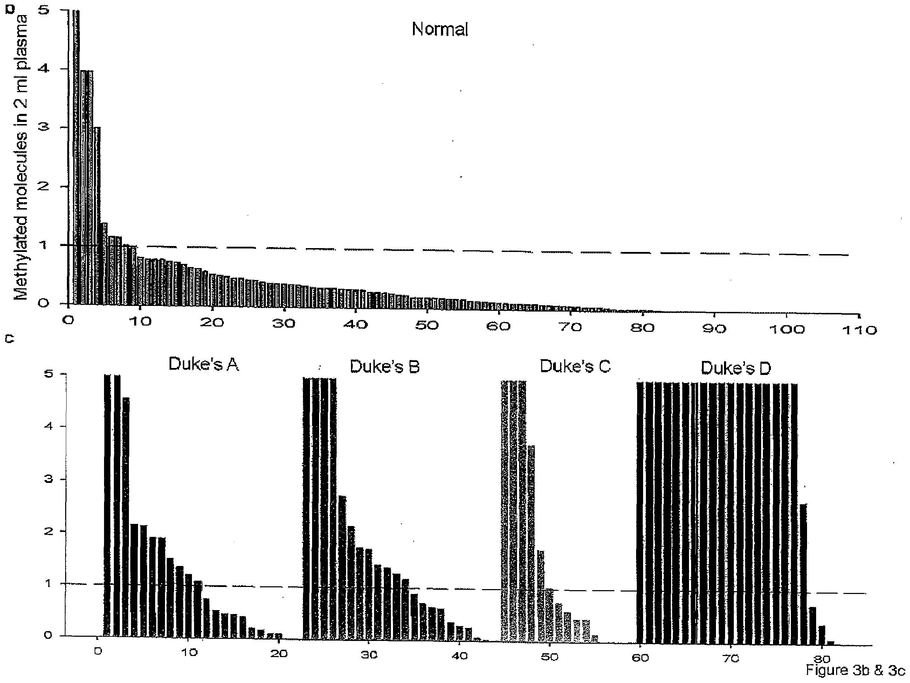

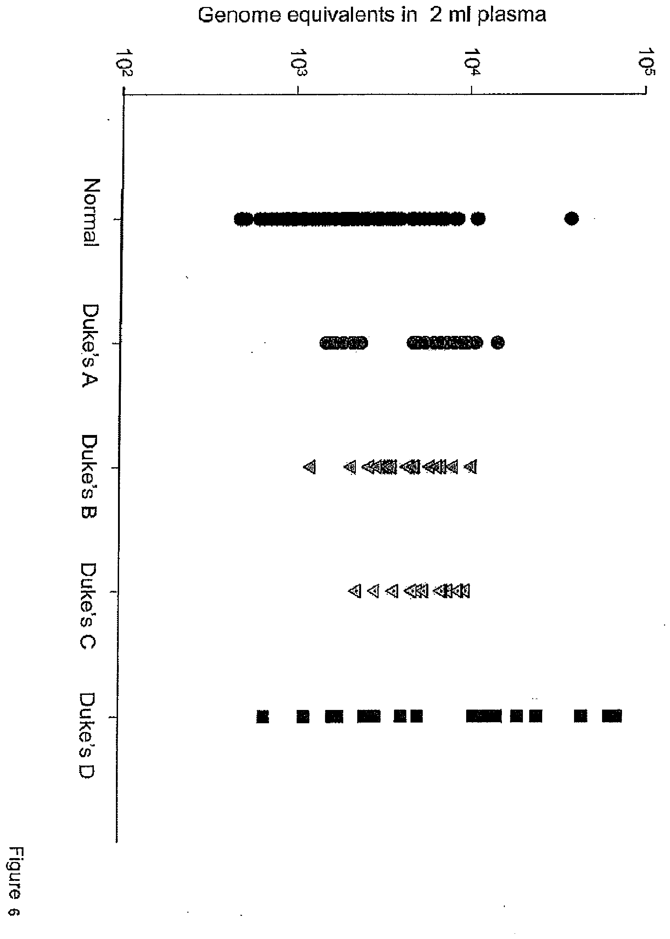

To determine the sensitivity and specificity of Methyl-BEAMing in plasma samples, we evaluated 191 samples, 81 from patients with colorectal cancer and 110 from age- and sex-matched controls. The total amount of DNA in the plasma was somewhat higher in patients with cancer than in the cancer-free controls, as noted previouslyl2 (Fig. 6; supp. fig. 2). The average fraction of methylated vimentin fragments in these patients varied greatly but in a specific way. It was very low in the normal controls with a mean of 0.026% and gradually increased with cancer stage up to a mean of 4.4% in the most advanced disease (Duke's D; Table 1). The theoretical limit of detection in any digital assay is one event, *i.e*., in the current study, the limit of detection was one methylated vimentin fragment in the 2 ml plasma that was assayed for each patient. Using this cutoff, the sensitivity of Methyl-BEAMing was 59% in cancer patients, and the specificity was 93% (eight of the 110 normal samples contained ≥ 1 methylated vimentin fragment per 2 ml plasma, Table 1, Fig. 3), The Area under the Receiver Operating Characteristic Curve (AUC) of this assay in cancers was 0.81, and varied from 0.67 to 0.95 among the four cancer stages (Fig. 3a). As expected, the absolute number of methylated vimentin fragments in plasma increased with tumor stage, ranging from a mean of 1.8 in Duke's A patients with detectable circulating methylated vimentin to 1195 in Duke's D patients (Table 1 and Fig. 3c). Importantly, the sensitivity for detecting colorectal cancer was ∼50% in the patients with Duke's A and B cancers, nearly all of which were likely to be curable by conventional surgery (Table 1 and Fig. 3c).

**Table 1 Methylated vimentin DNA fragments in plasma**

| **Sample Type** | **Number of Patients** | **Total vimentin fragments (methylated plus unmethylated) (mean ± SEM)** | **Fraction of methylated fragments (mean** ± **SEM)** | **# of Vimentin methylated fragments in 2 ml plasma (mean** ± **SEM)** | **# of samples with ≥ 1.00 methylated fragment in 2 ml plasma (fraction in parentheses)** |
|---|---|---|---|---|---|
| Normal | 110 | 3170 + 380 | 0.026% ± 0.013% | 0.60 ± 0.27 | 8 (7%) |
| Colorectal cancers (all stages) | 81 | 8240 ±1180 | 1.4% ± 0.40% | 334.6 ± 177.2 | 48 (59%) |
| Duke's A | 22 | 5830 ± 781 | 0.052% ± 0.025% | 1.8 ±0.54 | 11 (50%) |
| Duke's B | 22 | 4560 ± 458 | 0.31 % ± 0.23% | 8.9 ± 5.3 | 12 (55%) |
| Duke's C | 15 | 6530 ± 632 | 0.76% ± 0.65% | 38.4 ± 31.6 | 6 (40%) |
| Duke's D | 22 | 15,500 ± 4270 | 4.4% ± 1.2% | 1195 ± 626 | 19 (86%) |

### EXAMPLE 5

### Methyl-BEAMing of fecal DNA

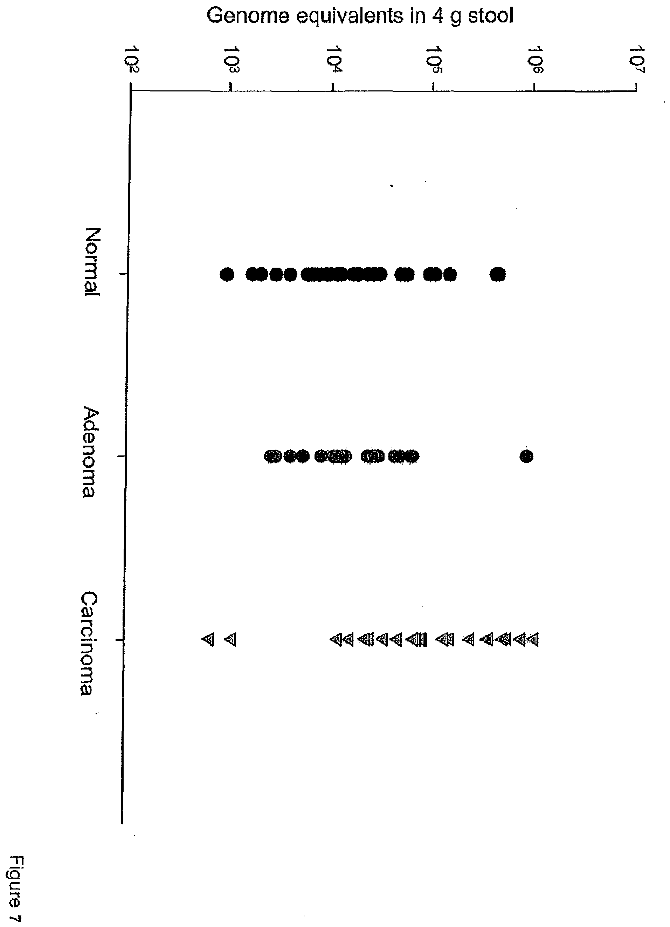

Next, we used Vimentin Methyl-BEAMing to screen 80 stool samples, including 38 from normal individuals, 20 from patients with > 1 centimeter adenomas, and 22 from colorectal cancer patients of various stages. In each case, we homogenized 4 g of stool and captured vimentin fragments through electrophoresis-driven hybridization to oligonucleotides on a solid phase matrix 31. The concentration of vimentin DNA fragments in stool varied widely, as expected for samples from individuals with various diets and bowel habits (Fig. 7; supp. fig. 3). Though there were no reproducible differences between the concentrations of total vimentin DNA fragments in the three patient groups, there was a significant difference in the fraction of methylated fragments. This fraction increased from a mean of 0,96% in normal individuals to 3.8% and 7.3% in patients with adenomas or carcinomas, respectively (Table 2). Using a threshold of 2% methylation, 45% and 41% of the patients with adenomas and carcinomas, respectively, scored positive in the Methyl-BEAMing assay, while only 5% of the samples from normal individuals did so (Table 2, Fig. 4). The corresponding areas under the ROC curves were 0.69 and 0.62 for adenomas and carcinomas (Fig. 4).

**Table 2 Methylated vimentin DNA fragments in fecal DNA**

| **Sample type** | **Number of Patients** | **Total vimentin fragments (methylated plus unmethylated) (mean ± SEM)** | **Fraction of methylated fragments (mean ± SEM)** | **# of samples with ≥ 2.0% methylated fragment in 4 g stool (fraction in parentheses)** |
|---|---|---|---|---|
| Normal | 38 | 47342.3 ±16731.6 | 0.96% ±0.25% | 2 (5.3%) |
| Adenoma | 20 | 69588.8 ±45185.7 | 3.83% + 0.86% | 9 (45%) |
| Colorectal cancer (mixed stages) | 22 | 236182.6 ± 66576.8 | 7.30% + 3.28% | 9 (41%) |

### EXAMPLE 6

### Validation of Methyl-BEAMing through massively parallel sequencing

To further validate the accuracy of Methyl-BEAMing for the quantification of methylated vimetin fragments in clinical samples, we evaluated samples with low and high fractions of methylated fragments on the Illumina Genome Analyzer sequencing instrument. Following bisulfite conversion and PCR amplification (Fig. 1), the PCR products were ligated to adapters and sequenced using a standard Illumina protocol. The 36-base region sequenced encompassed the five core CpG's that were assessed with the hybridization probes employed in Methyl-BEAMing. Through the analysis of C residues not present within CpG motifs from normal lymphocyte DNA, we found that the bisulfite conversion efficiency used for Methyl-BEAMing was ∼99.4% . In this lymphocyte sample, the fraction of sequenced fragments in which four or five of the five core CpG sites were methylated was 0.015%. This fraction was determined to be 0.018% in the Methyl-BEAMing assay. Likewise, the fraction of methylated fragments in a fecal DNA sample with a high degree of methylation was similar when assessed by either sequencing or Methyl-BEAMing (11.3% vs. 10.8%, respectively).

### References

The disclosure of each reference cited is expressly incorporated herein.
1. Feinberg, A.P. & Vogelstein, B. Hypomethylation distinguishes genes of some human cancers from their normal counterparts. Nature 301, 89-92 (1983).
2. Feinberg, A.P. & Tycko, B. The history of cancer epigenetics. Nat Rev Cancer 4, 143-153 (2004).
3. Herman, J.G. & Baylin, S.B. Gene silencing in cancer in association with promoter hypermethylation. N Engl J Med 349, 2042-2054 (2003).
4. Jones, P.A. & Baylin, S.B. The epigenomics of cancer. Cell 128, 683-692 (2007).
5. Esteller, M., Corn, P.G., Baylin, S.B. & Herman, J.G. A gene hypermethylation profile of human cancer. Cancer Res 61, 3225-3229 (2001).
6. Laird, P.W. The power and the promise of DNA methylation markers. Nat Rev Cancer 3, 253-266 (2003).
7. Grady, W.M., Rajput, A., Lutterbaugh, J.D. & Markowitz, S.D. Detection of aberrantly methylated hMLH1 promoter DNA in the serum of patients with micro satellite unstable colon cancer. Cancer Res 61, 900-902 (2001).
8. Kawakami, K. et al. Hypermethylated APC DNA in plasma and prognosis of patients with esophageal adenocarcinoma. J Natl Cancer Inst 92, 1805-1811 (2000).
9. Wong, I.H., Zhang, J., Lai, P.B., Lau, W.Y. & Lo, Y.M. Quantitative analysis of tumor-derived methylated pl6INK4a sequences in plasma, serum, and blood cells of hepatocellular carcinoma patients. Clin Cancer Res 9, 1047-1052 (2003).
10. Hoque, M.O. et al. Quantitation of promoter methylation of multiple genes in urine DNA and bladder cancer detection. J Natl Cancer Inst 98, 996-1004 (2006).
11. Machida, E.O. et al. Hypermethylation of ASC/TMS1 is a sputum marker for late-stage lung cancer. Cancer Res 66, 6210-6218 (2006),
12. Fleischhacker, M. & Schmidt, B. Circulating nucleic acids (CNAs) and cancer--a survey. Biochim Biophys Acta 1775, 181-232 (2007).
13. Lofton-Day, C. et al. DNA methylation biomarkers for blood-based colorectal cancer screening. Clin Chem 54, 414-423 (2008).
14. Herman, J.G., Graff, J.R., Myohanen, S., Nelkin, B.D. & Baylin, S.B. Methylation-specific PCR: a novel PCR assay for methylation status of CpG islands. Proc Natl Acad Sci U S A 93, 9821-9826 (1996).
15. Xiong, Z. & Laird, P.W. COBRA: a sensitive and quantitative DNA methylation assay. Nucleic Acids Res 25, 2532-2534 (1997).
16. Gonzalgo, M.L. & Jones, P.A. Rapid quantitation of methylation differences at specific sites using methylation-sensitive single nucleotide primer extension (Ms-SNuPE). Nucleic Acids Res 25, 2529-2531 (1997).
17. Lo, Y.M. et al. Quantitative analysis of aberrant p16 methylation using real-time quantitative methylation-specific polymerase chain reaction. Cancer Res 59, 3899-3903 (1999).
18. Eads, C.A. et al. MethyLight: a high-throughput assay to measure DNA methylation. Nucleic Acids Res 28, E32 (2000).
19. Sidransky, D. Emerging molecular markers of cancer. Nat Rev Cancer 2, 210-219 (2002).
20. Korshunova, Y. et al. Massively parallel bisulphite pyrosequencing reveals the molecular complexity of breast cancer-associated cytosine-methylation patterns obtained from tissue and serum DNA. Genome Res 18, 19-29 (2008).
21. Weisenberger, D.J. et al. DNA methylation analysis by digital bisulfite genomic sequencing and digital MethyLight. Nucleic Acids Res 36, 4689-4698 (2008).
22. Diehl, F. et al. Detection and quantification of mutations in the plasma of patients with colorectal tumors. Proc Natl Acad Sci U S A 102, 16368-16373 (2005).
23. Grunau, C., Clark, S.J. & Rosenthal, A. Bisulfite genomic sequencing: systematic investigation of critical experimental parameters. Nucleic Acids Res 29, E65-65 (2001).
24. Okamoto, A. Chemical approach toward efficient DNA methylation analysis. Org Biomol Chem 7, 21-26 (2009).
25. Chen, W.D. et al. Detection in fecal DNA of colon cancer-specific methylation of the nonexpressed vimentin gene. J Natl Cancer Inst 97, 1124-1132 (2005).
26. Zou, H. et al. Highly methylated genes in colorectal neoplasia: implications for screening. Cancer Epidemiol Biomarkers Prev 16, 2686-2696 (2007).
27. Jahr, S. et al. DNA fragments in the blood plasma of cancer patients: quantitations and evidence for their origin from apoptotic and necrotic cells. Cancer Res 61, 1659-1665 (2001).
28. Dressman, D., Yan, H., Traverso, G., Kinzler, K.W. & Vogelstein, B. Transforming single DNA molecules into fluorescent magnetic particles for detection and enumeration of genetic variations. Proc Natl Acad Sci U S A 100, 8817-8822 (2003).
29. Diehl, F. et al. BEAMing: single-molecule PCR on microparticles in water-in-oil emulsions. Nat Methods 3, 551-559 (2006).
30. Diehl, F. et al. Circulating mutant DNA to assess tumor dynamics. Nat Med 14, 985-990 (2008).
31. Kent Moore, J., Smith, J.A., Whitney, D.H., Durkee, K.H. & Shuber, A.P. An electrophoretic capture method for efficient recovery of rare sequences from heterogeneous DNA. Biotechniques 44, 363-374 (2008).
32. Li, M., Diehl, F., Dressman, D., Vogelstein, B. & Kinzler, K.W. BEAMing up for detection and quantification of rare sequence variants. Nat Methods 3, 95-97 (2006).
33. Lo, Y.M. Noninvasive prenatal detection of fetal chromosomal aneuploidies by maternal plasma nucleic acid analysis: a review of the current state of the art. Bjog 116, 152-157 (2009).
34. Lieberman, D.A. et al. Use of colonoscopy to screen asymptomatic adults for colorectal cancer. Veterans Affairs Cooperative Study Group 380. N Engl J Med 343, 162-168 (2000).
35. Kahi, C.J., Rex, D.K. & Imperiale, T.F. Screening, surveillance, and primary prevention for colorectal cancer: a review of the recent literature. Gastroenterology 135, 380-399 (2008).
36. Humphrey, L.L., Helfand, M., Chan, B.K. & Woolf, S.H. Breast cancer screening: a summary of the evidence for the U.S. Preventive Services Task Force. Ann Intern Med 137, 347-360 (2002).
37. Esteller, M. Cancer epigenomics: DNA methylomes and histone-modification maps. Nat Rev Genet 8, 286-298 (2007).
38. Moving AHEAD with an international human epigenome project. Nature 454, 711-715 (2008).
39. Rago, C. et al. Serial assessment of human tumor burdens in mice by the analysis of circulating DNA. Cancer Res 67, 9364-9370 (2007).
40. Warnecke, P.M. et al. Detection and measurement of PCR bias in quantitative methylation analysis of bisulphite-treated DNA. Nucleic Acids Res 25, 4422-4426 (1997).

### CLAUSES

1. A method for determining fraction of molecules comprising a methylated sequence in a sample of analyte DNA molecules comprising the sequence, comprising:
   **treating** a sample of analyte DNA molecules with a reagent which selectively modifies methylated cytosine residues or which selectively modifies unmethylated cytosine residues;
   **forming** microemulsions comprising the treated analyte DNA molecules;
   **amplifying** a portion of a treated analyte DNA molecule in the microemulsions in the presence of beads, wherein said portion comprises one or more 5'-CpG methylation sites, wherein the beads are bound to a plurality of molecules of a primer for amplifying the analyte DNA molecules, whereby a plurality of copies of analyte DNA molecule are formed covalently attached to the plurality of molecules of the primer which are bound to beads;
   **determining** nucleotide sequences at the one or more 5'-CpG methylation sites of analyte DNA molecules which are bound to beads and quantitating beads that have modified 5'-CpG methylation sites and beads that have unmodified 5'-CpG methylation sites.
2. The method of clause 1 wherein the amplified portion of analyte DNA molecule comprises at least two 5'-CpG sites.
3. The method of clause 1 wherein the amplified portion of analyte DNA molecule comprises at least three 5'-CpG sites.
4. The method of clause 1 further comprising the step of isolating beads which are bound to modified analyte DNA molecule from beads which are bound to unmodified analyte DNA molecule.
5. The method of clause 4 wherein the step of isolating is performed using fluorescence activated cell sorting.
6. The method of clause 1 wherein prior to the step of determining, the microemulsions are broken by addition of one or more detergents.
7. The method of clause 1 wherein the step of determining is performed by hybridization to oligonucleotide probes which are differentially labeled.
8. The method of clause 1 wherein the step of determining is performed by nucleotide sequencing.
9. The method of clause 1 wherein the step of determining is performed by primer extension.
10. The method of clause 1 wherein the analyte DNA molecules are genomic DNA.
11. The method of clause 1 wherein the reagent is bisulfite ions which selectively modify unmethylated cytosine residues.
12. The method of clause 11 wherein the bisulfite ion-treated sample is exposed to alkaline conditions, whereby modified cytosine residues are converted to uracil residues.
13. The method of clause 1 wherein the sample is from a human being screened for or suspected of having a neoplasm.
14. The method of clause 1 wherein the sample is from a human being screened for or suspected of having a colon adenoma or colon cancer.
15. The method of clause 1 wherein the sample is from a patient undergoing anti-cancer therapy.
16. The method of clause 1 wherein the sample is from a patient who previously was treated to remove or ablate a cancer.
17. The method of clause 1 wherein the sample is from a tumor margin.
18. The method of clause 1 wherein the sample is from blood, serum, or plasma.
19. The method of clause 1 wherein the sample is from stool.
20. The method of clause 1 wherein the sample is from lymph nodes.
21. The method of clause 1 wherein the sample is from urine.
22. The method of clause 1 wherein the sample is from sputum.
23. The method of clause 1 wherein the sample is from a tissue.
24. The method of clause 1 wherein the amplified DNA comprises a portion of a vimentin gene.
25. The method of clause 1 wherein the amplified DNA comprises a converted sequence derived from all or a portion of 
5′-CTCGTCCTCCT ACCGCAGGAT GTTCGGCGGC CCGGGCACCG CGAGCCGGCC
GAGCTCCAGC CGGAGCTACG TGACTACGTC CACCCGCACC TACAGCCTGG GCAGC-3′
(SEQ ID NO: 1).
26. The method of clause 1 wherein the amplified DNA comprises a converted sequence derived from all or a portion of 5'-CTCGTCCTCCT ACCGCAGGAT GTTCGGCGGC CCGGGCACCG CGAGCCGGCC GAGCTCCAGC CGGAGCTACG TGACTACGTC CACCCGCACC TACAGCCTGG GCAGC-3' (SEQ ID NO: 1), in which the converted sequence is derived by replacing each of one or more unmethylated C bases with a T base.
27. The method of clause 1 wherein the amplified DNA comprises a converted sequence derived from all or a portion of the reverse complement of SEQ ID NO: 1, 5'-GAGCAGGAGGATGGCGTCCTACAAGCCGCCGGGCCCGTGGCGCTCGGCCGGCTC GAGGTCGGCCTCGATGCACTGATGCAGGTGGGCGTGGATGTCGGACCCGTCG -3' (SEQ ID NO: 28)
28. The method of clause 1 wherein the amplified DNA comprises a converted sequence derived from all or a portion of the reverse complement of SEQ ID NO:1 in which the converted sequence is derived by replacing each of one or more unmethylated C bases with a T base.
29. The method of clause 1 wherein the amplified DNA comprises all or a portion of 
5’-
GTTGTTTAGGTTGTAGGTGCGGGTGGACGTAGTTACGTAGTTTCGGTTGGAGTTCGG
TCGGTTCGCGGTGTTCGGGTCGTCGAATATTTTGCGGTAGGAGGACGAG-3’ (SEQ ID
NO: 29) .
30. The method of clause 1 wherein the primer for amplifying the analyte DNA molecules is selected from the group consisting of 5'-GTTGTTTAGG TTGTAGGTGN GGG-3 (SEQ ID NO: 2) and 5'-CTCNTCCTCC TACCNCAAAA TATTC-3'(SEQ ID NO: 3).
31. The method of clause 1 wherein the primer for amplifying the analyte DNA molecules comprises at least 15 contiguous nucleotides selected from the group consisting of 5'-GTTGTTTAGG TTGTAGGTGN GGG-3 (SEQ ID NO: 2) and 5'-CTCNTCCTCC TACCNCAAAA TATTC-3'(SEQ ID NO: 3).
32. The method of clause 1 wherein nucleotide sequences are determined by hybridization to fluorescent probes.
33. The method of clause 1 wherein the amplified portion of analyte DNA comprises between 50 and 200 nucleotides.
34. The method of clause 1 wherein the amplified portion of analyte DNA comprises between 75 and 125 nucleotides.
35. The method of clause 1 further comprising testing for the presence of one or more mutations in a tumor suppressor or oncogene in the sample.
36. A bead which is bound to a plurality of molecules of a primer for amplifying DNA molecules, wherein the primer comprises at least 15 contiguous nucleotides selected from the group consisting of 5' -GTTGTTTAGG TTGTAGGTGN GGG-3 (SEQ ID NO: 2) 5' - CTCNTCCTCC TACCNCAAAA TATTC-3' (SEQ ID NO: 3); and the complements thereof.
37. The bead of clause 36 wherein the primer is covalently bound to the bead.
38. The bead of clause 36 wherein the primer is non covalently bound to the bead.
39. The bead of clause 36 which is superparamagnetic.

## Claims

1. A bead which is bound to a plurality of molecules of a primer for amplifying DNA molecules, wherein the primer comprises at least 15 contiguous nucleotides selected from the group consisting of:
5'-GTTGTTTAGGTTGTAGGTGNGGG-3' (SEQ ID NO: 2);
5'-CTCNTCCTCCTACCNCAAAATATTC-3' (SEQ ID NO: 3); and
the complements thereof.

2. The bead of claim 1, wherein the primer is covalently bound to the bead.

3. The bead of claim 1, wherein the primer is non covalently bound to the bead.

4. The bead of claim 1, wherein the bead is superparamagnetic.

5. The bead of claim 1, wherein at least 10 copies of the primer are bound to the bead.

6. The bead of claim 1, wherein at least 50 copies of the primer are bound to the bead.

7. The bead of claim 1, wherein at least 100 copies of the primer are bound to the bead.

8. The bead of claim 1, wherein at least 500 copies of the primer are bound to the bead.

9. The bead of claim 1, wherein at least 1,000 copies of the primer are bound to the bead.

10. The bead of claim 1, wherein at least 10,000 copies of the primer are bound to the bead.

11. The bead of claim 1, wherein the bead comprises a polymeric material.

12. The bead of claim 1, wherein the bead comprises a nonpolymeric material.

13. The bead of claim 1, wherein the bead comprises a material selected from the group consisting of: polystyrene, a styrene copolymer, methyl methacrylate, functionalized polystyrene, glass, silicon, and carboxylate.

14. The bead of claim 1, wherein the primer is at least 18 nucleotides in length.

15. The bead of claim 1, wherein the primer is at least 25 nucleotides in length.


SEQUENCE LISTING

<110> Vogelstein, Bert
      Kinzler, Kenneth W.
      Li, Meng
      Diaz, Luis
      Papadopoulos, Nickolas
      Markowitz, Sanford

<120> DIGITAL QUANTIFICATION OF DNA
  METHYLATION

<130> 001107.00770

<160> 29

<170> FastSEQ for Windows Version 4.0

<210> 1
<211> 106
<212> DNA
<213> Homo sapiens

<400> 1
ctcgtcctcc taccgcagga tgttcggcgg cccgggcacc gcgagccggc cgagctccag      60
ccggagctac gtgactacgt ccacccgcac ctacagcctg ggcagc                    106

<210> 2
<211> 23
<212> DNA
<213> Homo sapiens

<220>
<221> misc_feature
<222> (1)...(23)
<223> n = A,T,C or G

<400> 2
gttgtttagg ttgtaggtgn ggg                                              23

<210> 3
<211> 25
<212> DNA
<213> Homo sapiens

<220>
<221> misc_feature
<222> (1)...(25)
<223> n = A,T,C or G

<400> 3
ctcntcctcc taccncaaaa tattc                                            25

<210> 4
<211> 33
<212> DNA
<213> Homo sapiens

<400> 4
cgccaccctc cgcagccatg tccaccaggt ccg                                   33

<210> 5

<211> 35
<212> DNA
<213> Homo sapiens

<400> 5
ggccatcgcc accctccgca gccatgtcca ccagg                                35

<210> 6
<211> 21
<212> DNA
<213> Homo sapiens

<400> 6
tggcacatat acaccatgga a                                               21

<210> 7
<211> 21
<212> DNA
<213> Homo sapiens

<400> 7
tgatggtttc caatttcatc c                                               21

<210> 8
<211> 21
<212> DNA
<213> Homo sapiens

<400> 8
tggcacatat acaccatgga a                                               21

<210> 9
<211> 24
<212> DNA
<213> Homo sapiens

<400> 9
tgtccctaca aaggatatga actc                                            24

<210> 10
<211> 20
<212> DNA
<213> Homo sapiens

<400> 10
gggtggacgt agtcacgtag                                                 20

<210> 11
<211> 19
<212> DNA
<213> Homo sapiens

<400> 11
cctcctaccg caggatgtt                                                  19

<210> 12
<211> 18
<212> DNA
<213> Homo sapiens

<400> 12
ctgtaggtgc gggtggac                                                   18

<210> 13
<211> 19
<212> DNA
<213> Homo sapiens

<400> 13
cctcctaccg caggatgtt                                                    19

<210> 14
<211> 19
<212> DNA
<213> Homo sapiens

<400> 14
cctcctaccg caggatgtt                                                    19

<210> 15
<211> 18
<212> DNA
<213> Homo sapiens

<400> 15
ctgcccaggc tgtaggtg                                                     18

<210> 16
<211> 45
<212> DNA
<213> Homo sapiens

<400> 16
tcccgcgaaa ttaatacgac ctcgtcctcc taccgcaaaa tattc                       45

<210> 17
<211> 23
<212> DNA
<213> Homo sapiens

<400> 17
gttgtttagg ttgtaggtgc ggg                                               23

<210> 18
<211> 45
<212> DNA
<213> Homo sapiens

<400> 18
tcccgcgaaa ttaatacgac ctcatcctcc taccacaaaa tattc                       45

<210> 19
<211> 23
<212> DNA
<213> Homo sapiens

<400> 19
gttgtttagg ttgtaggtgt ggg                                               23

<210> 20
<211> 20
<212> DNA
<213> Homo sapiens

<400> 20
tcccgcgaaa ttaatacgac                                                   20

<210> 21
<211> 23
<212> DNA
<213> Homo sapiens

<400> 21
gttgtttagg ttgtaggtgc ggg                                                23

<210> 22
<211> 23
<212> DNA
<213> Homo sapiens

<400> 22
gttgtttagg ttgtaggtgt ggg                                                23

<210> 23
<211> 20
<212> DNA
<213> Homo sapiens

<400> 23
tcccgcgaaa ttaatacgac                                                    20

<210> 24
<211> 22
<212> DNA
<213> Homo sapiens

<400> 24
tcggtcggtt cgcggtgttc ga                                                 22

<210> 25
<211> 22
<212> DNA
<213> Homo sapiens

<400> 25
ttggttggtt tgtggtgttt ga                                                 22

<210> 26
<211> 35
<212> DNA
<213> Homo sapiens

<400> 26
gtgygggtgg aygtagttay gtagtttygg ttgga                                   35

<210> 27
<211> 94168
<212> DNA
<213> Homo sapiens

<400> 27
aacagtgaga gggccctcac gtatacactg atccatgggt aaatggaacc gagagagtgg        60
cattgtctct gcttttagat tcttgccagt aacaccactg taagtgagtg aaggcttaac       120
tcttttgttt ttggcttgtg gccttaatca gctactttaa cgctcttgac agctgagctt       180
tctctttccc agctctgctg agaccctgac aacaacactg aataatgtcc taaaaaattg       240
atagtttcat ttatctgcaa agacatattt tttgtgtctc actgcctctt cccactccga       300
ttacattatt atttctacac tgcaaagctt tacaacattt tcagctattt tctaaccata       360
gttttcctcc taacacggca gcttcattcc tgggttttct actttgattc atctcttgct       420
cagctagatt aagtgatgca gtcatctgtt ccagaagggc tcatggatgt tatattgtaa       480

atttcattct agaaaaattt ttatctattt ttattaagag tttttgttcc actggagtca 540
ttctcctctt cagaggcatt aatgatattt atcacggatc actctgtctt cggtagctat 600
catttactct acagtatgta actggagaaa gtgaagaaag agaacagaga ttatttttga 660
agcaataatg gtcaaacccc ccaaatttga tgaaaattat aaataagaaa agcaccacaa 720
ggtccaagca ctacaaataa agaaaaacat acagagcaca tcaaaataaa acttttcaaa 780
acacgtgata aagataaaat cttaaatgta gacaaagaaa gacacaatat attaagagga 840
atgaaagcag aaattattac agaccttaag tcagaaatta agaaacccag ataacactgg 900
aaagtattaa aagaaaacaa acagattttt acaaaaatat ctttcaaaat gaatgcaatc 960
aaagattcag tgggccaggc acagtggctc atgcctgtaa tcccagcact ttgagaggct 1020
gatatgggag gaccagttga gcccaagagt ttgagaccag cctgggcaac atagtgagac 1080
cctgcctctc caaaaaagaa aaaaaaatga gtaaaaatta tatttcacag tgaagacaaa 1140
ataaaacact tttttactca aaagcaccaa ggtaatttgt ccctttccac atataaccaa 1200
caagagacac taaagaaggt ttttcaggaa gaagaaaaat tatatcacat gcaaactttg 1260
atctaaaaca caaggataaa aagtgctgaa aattgtaggt atatagataa atataaaaga 1320
ctttgtgctt tttaaaagtt ctttaataga tatttgacta tttaatccaa aaaacagtaa 1380
caatgtatgt tggtgcctat aacattcgta gcagtaaagt gcaagacaaa atatcaaaat 1440
agaccagtag gggaaattgg aggaatactg ttaaaattta aacataaagt ggtataatgt 1500
tatttgaagg cgggcttcca caagtaaaag atgcatcttg taaaccctag agaatccaac 1560
aggaaaataa aacaaagata gatatagata tttagatatt taaaaataaa tttcagttta 1620
cattgaataa atattatgac taattgtctt taaaaagaca ttagtgaaga ataatgaatt 1680
ttttaaaaat ccaaataaaa atagtgacaa tgtaaaatgg taaaaacact tcaaaaactt 1740
tgaaaatttg gtattttctc ataaagttta ataaatgtag gtatttatcc acaataaatg 1800
aaaactaaga tccacacaga tttgtacata tatgttcatt gcaactttac ttgtaatggt 1860
aaaaaactag aaactactta aatgtctata ataatgtgaa tacatacaca tattgtagta 1920
tatgatattc tactgtacaa aatatcactc aacaataaaa aagagcaaat tattgatata 1980
caacaaaaca tgatgaattc caaaaccatc aacactaaac aaaagcagtt ccactcaaaa 2040
gaactacact cttatgattt tatttgtgat atactaggaa gggaaaactt aatccataat 2100
gaaaagaaag cagatccatt gtcgcctggg ttggggttgg agactgacag aaatgtggca 2160
ctaagaacaa tttagcatga tggaagtgtt ctaagtcttg attgtggtgg tagtagttac 2220
acagttgatg attgagtcga aactcaaact gcccacttaa aattacgtac aataaactac 2280
atatttattg catgtaaatt atgagaaatt tcatgagaaa tttgtttgta aaatttttca 2340
tgataaatta ggagaaacaa cacaatttct tttttttttt tttttttttg agacaaagtc 2400
gaccaggctg gagtgcagtg gcgcgatctc agctcactgc aacctccgcc tcctgggttc 2460
aagcgattct cctgcctcag cctcccaagt agctgggact acaggcatgt gccaacacac 2520
ctggctaatt tttgtatttt tagtagagac caggttttgc tatgtcagcc aggctggtct 2580
cgaactcata acttcgtgat ccacctgtct cggcctccca aagtgctggc attacaggcg 2640
tcagccaccg cgcccagcca atttcacatt atttaaaaaa taactcaaca taaatcagtg 2700
tttcaatact tgtgctgaag tcaccacagg tgaaaaaggt taaccttgac aggtttgcta 2760
aaataagaag cattcagtaa aagccaatga ttttctccct tgttcttact gcatgctata 2820
tatattgcat aagtatttaa taaaagtaaa tactttttcg aagttcaata tcaccacaga 2880
accaagcatt tagaagtcaa gcatatcctt acatttcaat tatttctctg aaagaccaaa 2940
atattgatct ccctgtctat agctggactt ttttttaaaa gcatgctatc ttctgatagg 3000
atataaaatg ttaatctcct tctcctgaac tcttaaattt atatatgctt tatcttccaa 3060
ttaagacaac ggaaaccagt attaggcagt gtttttcaat cacatttccc ttgctacata 3120
cttattaaaa caatgacaat tactcacttc aatcgtcttg gcaagtaact gtgtgtgagg 3180
aaaattatac ttgtatactt cattagcgac agtgttgaca tcaatggcag ccaccacttg 3240
tgcaggtata cagctttctg taatgataaa tggagtatct tgaaaagaaa agtccttaag 3300
ttcattatag aaagaaatca aaatagcaga atttagtaat ccatttcctt atttcttcat 3360
ttattagcct catctcagac tgtcagtgga aggtcaggcc aggggaagcc aggcatgaag 3420
tgaaatattt gaacatggac ttctggttca tctctctaaa cagcattcca atactaaaga 3480
gaagatttaa atattttagt gcttacctct tatatttgct ttcctccctc attaagagac 3540
acacttataa gtcaaataga aagttttcct aagttaaaac aaactaaaaa ttgaaaagtg 3600
agatatattc taaaagccct atgatttaga actttatata aataaactac catttgttct 3660
gcaccttctc acattaatac aatgttgctt cacagtacca ttgaaaatgc tttaattgtt 3720
tttttagtaa cagctttatt gagatgtaat tcacatttga caaaattcac ccaattagag 3780
cttacataag tttttaaatg agatgaacaa ttaattggta tccaaatgta aatggttcat 3840
ttaccaacat tttaaatgtt gacagaccag aagagaatat aagaagaata agggaaataa 3900
ttataaagga atgccttcac tgcccttctg tcttgctggt gaagaaaacc attttcaaaa 3960
tgttttcaga tccagagaat tctagcatat tctttaatgt tagccgaaat tggaactcca 4020
caattgtagc cgtagctata ttttacaatg accaccctct ttccccaaat atgtataaaa 4080
ccaattatgt gcatgccctg acccaagtca ggagaaagtg tgaagtttta aaaatgaaag 4140
tatttgagac ttcagaaaat catcagagaa agcaatacaa tattacatag tagatccaag 4200
tagccgattc tttaatacca ttagttgatt aatagttagc tgattagctt accccaaata 4260

tcaaaaaagg aaaaaaaaaa aaaaagagtg gtgtgaggaa ctaggactca tgcagcaggc 4320
acccggaaat aaaagcagac cacaggagac agggcaagga gaaaggctca cacacgctca 4380
gaaaacaatc ataaccttcc actgaggcct agaagagatg aggccaggaa gaaaagaaag 4440
gcacaaatgt ctccaaggat tgatactaaa tggctgctag gctcaagaca ctcagcaggt 4500
atcctgctga aaagtaagat cccctgcgtg ctgcaaactt ggtggtctaa tcagtaatgc 4560
catagacagc tgcctcttct gcattgaagt gcaataacta gttactggcc ttagtataaa 4620
ataaatttga ggttataaat tgtatctgtt aaagccatca gatcttgaaa cataaatcaa 4680
gaaggcttct aaacaaaaga aaatattaat atataaggat ccatttgcaa acacaaaact 4740
atcataattc aagtcaagaa ttaagctact actaatcact gtaagagagc tgaaaattaa 4800
ataaacttca ttgtactatt ttactgtttt gttaaatatg ttgtaatcag catgatgtag 4860
cctgcttgta tttccaccat atatgaagca aactgggatt gcctgacatt ggttaatagg 4920
acttgtatct tttttcaggc agagtgaaaa tgtacagtga tgtgctgaat ctgcactgta 4980
atcaataatt aattttatgt tcttgtgtta tatgcctgga ggtcataaaa actgacaaaa 5040
atcaactgcc aaatgagtat ttcatatggt gatccaaaaa ctgatgaact gaaatttctt 5100
tctcaggaaa gaacagattt tcaaagccaa taagcaaatg cactgtttgc atggattgat 5160
gcacatattt tagactgtta taaaattagg acctactgca taacttaaaa agttatttct 5220
attaagagaa atggatgtac taagatatta ctgacactaa ttcaacttgc cttctcaaaa 5280
tagaattggg gggtggttat tgtttctaag gaaaaggtta aacttacaga taagttttaa 5340
aaagcaaacc tgtcaattct gtcaaaatct taacttgaat tccaaagact agttgccttg 5400
atatttctta gtatacagtt taaatctaca atctccaaaa ttctttactt ttaagttaca 5460
tatatagatt ttgaaaatgt ataattaaaa atgtcaaagt gaatataatt ctatagaatt 5520
ttgcctatgt gagctccatc acttgagtga tccaagagta gtaattctca aaaaggaggc 5580
agaatttggg aaagggattc gtgagtatct tttcattgaa acagaggaag aggatgtgtt 5640
ttcagtactt tgatgtatta ctttgcctag actttattca tgatatttca gttattattc 5700
aggatttcat atttattaac aggaagcctg gtttaaaaaa aaaaggtgag atatcctact 5760
tagagaaaca catttatttt atttatttat ttatttattt atttattttt tgagatagtg 5820
tctcactctg ttgcccaggc tggagttcag tggcatgatc tcggctcact gcaacctcca 5880
cctctcaggt tcaagcaatt tgattctcct gcctcagtct ccagagtaac agaaactaca 5940
gacacttgcc accacaccca gctaatttct atatttttag tagggacagg gtttcaccat 6000
attggccagg ctggtctcaa actcctaacc tcaagtgatc tgcctgcctc agccttccaa 6060
agtgctggga ttacaggtgt gggccaccac gcttggctga aaaacacagt acaaattatt 6120
ttaaaaggtt gtcagattgg ctccgaaagg aatacataag gacatcacgc ttccatttat 6180
atgaggacta aatttaaaag gtagatttag ttatttcaag agagacttct aggtaagatg 6240
ttagaaatgt cactgaggat acatacataa ttcttgcttt caatatgctt cttacaaaaa 6300
tagacaagaa acacacacac acacacacac acacacacac acagacattt gaatagcaat 6360
actttccttt ctttttctga tgattcttct ttctggaaac gctgtaggga agaacttttt 6420
gttggtagta gtgaaaagct ttacaagttc acactcttct tgactttcct aaccaaataa 6480
ataaaaaaat ttaactgttc ttgtgtagtt attaatgtgg gaaaaaaatc aatatttaca 6540
agatgtaaat gcaaatgtaa aaaatgtaaa tgtaaagacg ttatgttaga taatataatg 6600
cagtttgaag ttttgaggag gaccttgaga aagaaaatta aggcataacc agtccacttt 6660
gaaataagga aagaaatttt taaaattcta ttccttcttt attatttatt ttctaatcta 6720
tatctcatat aggtgagtaa ctactgaatt actcattata ctcacacctg agacatttaa 6780
tcaagtatgt taacactgac atctagttgt aagatgaaag atcagttgaa tacatccaat 6840
gtcctttcat tcaaagtgct ggcttagtta ggaataaaag tactaaagaa cgtacagacc 6900
cataaggatc tgaacttaaa attgctgcca aatgttttat cactattagt aaagtatatt 6960
gtttggttct ttttaaaagt tcttgtattt tgctgggtga ggtggctcac acctgtaatt 7020
ccagcacttt gggaagccaa ggagggcaca ccatttgagg ttcggagttt gagaccagcc 7080
tggccaaaat agtgaaacct catctctact aaaaatacaa aaattagcca ggcatggtgg 7140
cacacgcctg taatcccagc tactcaggag gctgaagcag gagaatcgct tcaaccctgg 7200
aggcagcggg tacagtgagc tgagattgca ccattgcact ccagcctaga tgacagaatg 7260
agactctgtc tcggaaaaaa aaaaaaaaaa aaagttcttg cattttaaaa attgctgata 7320
aattatttcc aagaataaaa taaaaaatac attaaaaata tttttcaaag atccagtttc 7380
cttagaaagt ttccatagtc agctaactgt ccaaacaaaa tttactttgt gtcaagacat 7440
accttagatt attattgtta ctaacaccag actaaatata agagaccacc agccaaaata 7500
cggataggaa ctgctggaga tctatgaaat tatcacgtcc ttgacactat atcttaggag 7560
aatttatttt tcaacaaaat accaaaagac ataccagagc cagagaagtg gtggaagaga 7620
gaaatatggc aaattaatgt aaaaaaaaag gagcacagtg aaggaatcta caaggaatgg 7680
aaaataaaga tgttatgtca ggtagtataa tgcaatttgg agttttgagg aggaccttga 7740
gaaagaaaat taaggcatga gtaatctttc cggaacgtct ctagaaagtc tatttaaagt 7800
catgttgatg tcatgaggta gctaatacag cttctccagc ccctcaaccc acaacctcgc 7860
accagccctg aagctttcct tggcagaaga gaagagaaaa agcaatttag ctattcactt 7920
tcagcagggg ctacagaaag gccacatagc ccagcattac ccttagtcct gaggatgcag 7980
ggacctcaat ttccaagggc ataaactggt ctctaaaaag aatataggta acgaacgcct 8040

aggatacagt aaatgctcaa taattattaa aattcatatt cactgcctgg catttatctg 8100
gaatattata gattaaatag ataaaataat aatgataatg attattttaa ctaaaattta 8160
aaaatcaaaa ttaaaacaat cattatcatt ataattaagg ttagaaaatt agaccagtga 8220
caaaagctta agactcatac tgttcatctg ggagatgaat tgcaggagaa atatttttga 8280
aagagtttca agtaatgtaa gaagagatct tcacaaatga caaaaactag ctgattccaa 8340
tctctactaa taaaaaaaaa aaaaaaaagg aaaaacagaa acgggctaaa atttaatccc 8400
aagagactag cattgatggg aaaagctttc ctgaccttgc tatcaaaccc tagaatgaaa 8460
gagagaaatc ggaggtattg gggaaaggat ttttttaaca cagggcagaa aaattttctt 8520
cccaggatat ttattacttg ttttctccaa tttggaggag gatagtccca ctaagcaatc 8580
cctaaagaca ctttctagcc ctgatactct atgagcattt aagtaagaag tataggtctt 8640
cagatcatct tttactacct gccattattg acaaagatct tggccttttc accctaattc 8700
actggttaga aactaaagaa ctgatacaag agaaatgctc ttttatggct atgtttgtct 8760
attaatttgc ctttcctaga tactacgttt aaaataacta ttaaagaata aaaatagtaa 8820
gtgaactacg acaaacaata taaattttct ctttctgccg ggtgtggtgg ctcatgcctt 8880
taatcctagc actttgggag gctgaggcgg gtggatcacc tgaggtcagg agtttgagac 8940
cagcctggcc aacatggcaa aaccccgtct ctactaaaaa tacaaaaatc agctaggcat 9000
ggtggtaggc acctgtaatc ccagctactc agggcctgag gcagaagaat cgcttgaacc 9060
cacaaggtgg aggttgcagt gagccgagat tgcgccactt cactccagcc tgggcaaaag 9120
agcggaactc tgtctcaaaa aaaaaaaaaa aaaactctct ttccatattc ggtacagaga 9180
aaacctgatt tgtgggaatc aacatgaagt aacattgcat taatgcttaa tttttccttt 9240
ctacttataa ctgtctatta ggagagaaac tagtatttcc tagacaaagc tgcaccttag 9300
gtgaaatagt gccgtgcttt ttttcatgat aatcaatctt taattccctt gtccctcttc 9360
ccatttcaaa ctacctagca aaatgctaaa ctgaacacta agcaaatgag tacttaagaa 9420
acagtatgaa agatggatag caaacaacat atttttccta tttcagaact caaggaactt 9480
cacaatttct ccttagtgct cagtctcttt gaagtaaatt ttacatcaaa ttaaattttg 9540
ctaatgaaat tttactaata tgttttacta atgactcaaa aatatgattt ctagttatac 9600
tatctctaca aaaattattt gcatatccca acatttattt cttcctcctc ttcaataacc 9660
aaacatcaaa tttattcact gtggcaatgc accctgctaa aaggatatct gccacccctc 9720
tacagctaga tgcagccatg gactaaagtc agactaatga gacagaataa gtgcatcaca 9780
ctctcagaga gtgttctata aaggaagaga gtggtagttc cctatgctct ctggcctttc 9840
cactggaggt ttggaaagca gatgtaatga cttgagctcc gtttgctgtg acggaccatg 9900
aaggctgatc cgtgcactct catcacactc cagttcactc tgctgtcaca tttttcccag 9960
gacagtggct ccctcactag actactcaat catctcagtg ctgacaccat gtctcattca 10020
tatgggtact ctcagcaccc aggcattgca gtaattggca cagaataggc gctcaataaa 10080
cgtctattaa ttgaatacat acaagagaat aagtgaatta tacttcctaa aatcaactcc 10140
agattattac tgattaccct ccttgttctc ctgtttctga gaacactgct agcttaaaaa 10200
gtatttaata tgtctgaact tatatgatta tctatctcaa tgctcatttt taagtaattg 10260
tttgacattt aggtcaaatt ttttaacacc tacaatgaag ttcccaggaa acctacaaaa 10320
ttctatttat gttacaaatg tcttaaatat agtcccaata gtgctattgt caggcagtaa 10380
gtaaatatct gtgagattac ctatcatgta ctgagcactc tactgaggat acaaaatgag 10440
tggacatttg ttttctgttt taccagcatc tggcccctcc ttttctggca atagtcccag 10500
attttctttg ggggaaactt acctcactca tgctctgttg aagtggttca gatggcctca 10560
aatccccttc tcctagcttc tagaagcctc ggtcaagtgc ctgagccagt aagactagcc 10620
agaccggttt caggatgggc atgcaactca aaataagcca attaggcaca ataagaggtg 10680
acccctggct ttttcttgtg taactattaa tagcaagagt agcttgatct ttcttaccac 10740
gttaaacctt ggatggagat aagttcagag gctactacag gcattttgat gcaataaggg 10800
gagaacatat ataaaaatgc acccaaccta gaagaaacaa ggctgagata catggagaaa 10860
aaaactggtt tctggtgaca tgatttgagc ccctgcttca aactctaccc ctaactgggc 10920
ctacatttgg acctggtcaa ttaaatgagt ccagagttac aacttttagg aaaccagtct 10980
gagctaagtt tctgtcactt gcaaataaca cttctaagtg gcaatcaggt gaacacaaac 11040
taatatgcca tttgttccta tgaggagaag catttcaaat tccaaactgg caaacagagc 11100
aatatgaatg caaaagtcct gtaaataagt aattaattaa ccatacatct acaaaataga 11160
agtattcttc ttgataaaac tgttctaaat tgaagaaaga aggtcaagag tcttcaattt 11220
ggggagtgaa gacttcctta tggtaatacc caatcactta tttctatata tggatactaa 11280
atacaacaaa tacagagaga gagctactta taaactatag ctcccatgtc ttttaaggag 11340
cacagaatat tttcagttgc ccagtctcat caattcttac aaagctcaag tcctgaaagt 11400
aaaacagtta aaatagaaat ttcattacaa gcaattcaaa aataccaaat tttaaatgat 11460
cgataaacat ataaaagatg ccaaacttca ccaataatga cagaaatcca cattaaccag 11520
ttttacctag cagcttggca aagattggat aaatgaacac tgtctaatgc cagcaaggat 11580
ttaagaaaat gtgctgtcct tcatgtcatt tgtggtataa attgacataa ttcttctgat 11640
tattagacct agttatacta catctggaaa tgttcgctaa ggaaaattgt aaacaagtat 11700
gtaaagataa gagtgtatca ttacatacat gtatgaaaat agagatgtta aaaagataac 11760
atccatattt actgtcacaa agcaatatcc atattattta ctacatagag cagttacaga 11820

atatcttgag taaaaatcca agttttataa agtgtatgtg tatgtgtatg tgtgcacctt 11880
aagacagaga aaacagccaa tatcaacaga ggctatcccc tggtggtgag atttggaggt 11940
aattcttctt tctttaaact cttttgaata attcgatttt tttctcattg agaatatact 12000
attattttat ttatatcttt agttactttt ttgagatgga ctcttgctct gtcacccagg 12060
ctggagtgcc gtggcatgat ctcagctcac tgcaatctcc acctcctggg ttcaagtgat 12120
tcttctgctt cagcttcctg agtagctgag actacaggtg tgtgccacca cgcccggcta 12180
atttttgtat ttttagaaag agacagggtt tcaccacatt ggccaggctg gtctcgaact 12240
cctgacctca tgatccacct gcctcggcct cccaaagtgc tgggattaca ggcatgagcc 12300
accgcacctg gcaagaatgt actattttta taattggcaa aaagcaaaca aaaaaatgac 12360
tcttattttg gaacgaaaga attcaatgct gctcttggtt ttgaccctga gaaacaacaa 12420
aaggaaagta acaaggtcag acaagaaagg tttggctctt ttctcaaggc atgccaattg 12480
tgctgtgacc tgtcacaggc acatacacac cagtaaacaa ttataactca ttataattac 12540
aacaattgta actcagtgca ttattttacc tgctaactca acaataatca aaagaaaaac 12600
taaaaattaa actaccttat gaaagtcttc taaagatcat caagtagcta gcatacgccc 12660
aaaaaatgta gagctgaaag taacctattc acatgcttat agaagtcaaa agatataaac 12720
tacttaaaaa gtcaagaaat aatagaaaca catgtgatgc agtcaattac aggtgtgaac 12780
aacaaagact ttgattttgt tttacataca tttgttttga gtgaatgacc actcatgaat 12840
ggttccttac tataaaatgt ttacggtcct ctttactgat gctttctcat aacttatgag 12900
gtgatcattt ctgacatatt tatggaaaag tcaatgaaaa aatgagaagt gacataaaca 12960
ttagtactct atatctagtt aactgaattt tttaaagact tgtaaaaata taattatttt 13020
aatattttat aacatatagt ataatatata taataattaa ctcacaaaga tccctaaaat 13080
tagcctaaga aaatcctctc taaatttgtc ttcccattct gagcctcctt tgctttatag 13140
ttagccctcc ttgaaagctt ctaattttca tcattattga ctcaataaaa tttcaaatcc 13200
tatcctgcaa aacactagca gtataatttc tgctacagat acctctaaaa ggagagttaa 13260
gttttacaat tttgcactag gccagaaagt ataggcttgt cataagacct atgcacttca 13320
actgcctcac tcatttcccc tttccaagtc tcctcctaac tatatcattc tctacaaatc 13380
tttttccact aagccacaca gtattcagca ttaggcattc ttattttatt ttttatcaaa 13440
gctatcttag ggacagtctt atggaatttt ttattttgtg tgtgtgttca tacgtcctga 13500
tgaagattta gaatatggat atctgtttgt gtatttcttt ctctctttct ctgctagaat 13560
tctgcattta aatcctagct tggctatttc cagttgcgtg atttaggcaa gtttctttgt 13620
gtctttaagc ctcagtatct tcacctatta agaggataat aaagtacccg caccatagaa 13680
ttttctgagg attaaatgag ttaataacta taataggaca tggacatctt tggggattac 13740
tatttgatta gtgtctggca tataataaat gtttgccatt attattattg aaagtgcaaa 13800
gactttttaa aaatgagaat atcaaatgct agaaaggacc aggtgtgatt taaaaatcgt 13860
cagtgcggca cttctcaaaa gaagacattt atgcagccaa aagacacatg aaaaaatgct 13920
catcatcact ggccatcaga gagatgcaaa ttaaaaccac aatgagatac catctcacac 13980
cagttagaat ggcgatcatt aaaaagtcag gaaacaacag gtgctggaga ggatgtggag 14040
aaataggaac acttttacac tgttggtggg actgtaaact agttcaacca ttgtgggagt 14100
tggtgtggcg attcctcagg gatctagaac tagaaatacc atcccattac tgggcatata 14160
cccaaaggat tataaatcat gctgccataa agacacatgc acacgtatgt ttattgcggc 14220
actattcaca atagcaaaga cttggaacca acccaaatgt ccatcaatga tagactggat 14280
taagaaaatg tggcacatat acaccacgga atactatgca gccataaaaa ggatgagttc 14340
atgtcctttg tagggacatg gatgaagctg gaaaccatca atctcagcaa actattgcaa 14400
ggacagaaaa ccaaacacca cacattctca ctcataggtg ggaattgaac aatgagaaca 14460
cttggacaca gggtgggaac atcacacacc ggggcctgtt gtggggtgcg gggaggaagg 14520
agggatagca ttaggagata tacctaatgt aaataacaag ttaacaggtg cagcacacca 14580
acggcacatg tatacctatg taacaaacct gcatgttgtg cacatgtacc ctagaactta 14640
cagtataata aataaataaa taaataaata aataaataaa taaatatggt caatgcagtt 14700
gtgagcatca attaacagtc aatatttcaa tgaatatttg gtggtataca tttaaaaccc 14760
aaaaaattac tttggatttt ttagttttat gttttggcac atttcttaag gtgtgatagg 14820
caaaataata atccccaaag atatccacat cttaatccct gaaacctgtg aaaatgttac 14880
cttacatggg aatcaggatt ttgaagatgt gattaaactt gagatgagga gattatcctg 14940
aattatgcac gtgggcccag tataatcaca ggaaggactg aggaaggtta aaatcagaga 15000
aggggatttg gagatggagt agaggtcaga gagaaggctt tgggatgcta cacttctggc 15060
ttcaaagata gaaaaaggga taatgagcca cgtactatgg gcagccccta gaagccagaa 15120
aagccaagca aatgaattct cccctagaac ctccagaagg aatgcagccc tgttgataac 15180
ttgattttaa gacttctgac ctccagaatt ataagataat aaacctgtgt tgctttaagc 15240
tactaagttt atgtttgtta caacagcaat aggaaactca tacatagata ataattagag 15300
atgtaaagaa aatatttata tataggataa gtgatgctat tttatttata atagtgcaaa 15360
tttatgaatg atctgaatgt cccagaatag atgattaatt cgaacaaata ctgtactttc 15420
ataaagtgga gtatgataca accattaaaa gtttataaat tctacattta atggcaagcg 15480
aaaatattca agatattcta agtaaagaaa gaaagctata atttatttta atgttcatgg 15540
tcacattaga ataaagattt taatctaagc cttcaccagt gtttatgtct tcaccagtgt 15600

tatgtcagtg tttatgcatt agcatcattg gtgagttttt taaattttct tctctatttg 15660
tgtagcattg gtattgccca gtagagcaaa atctctggaa aaaggaacat gactgtctcg 15720
ttcaatgctg tatcctcacc atacaacaca gtaagcaaca aagaaaaatc actccatagt 15780
tatgtactga ataaatgaat attttcagtg tgtcccaatt ttttaacaaa tgtattacta 15840
atattgctaa aaataaaaaa caagaaatgc acataataat aatttttaaa tattcaactt 15900
cactagtaat caaaaattaa gataacaatt tcacctagta aattatatgg gttaacatat 15960
tatattggca tatttatggt cacagagacg ttactgaata taaactagta tgtcctttta 16020
aaaaaataat ttgacaatca tcaacaacct ttaaaatgtt catgtactat agcttattca 16080
ttaattctac aaatatttgt tgagcatcca acatacaatg gtgcttttag atccaaggat 16140
acagtggtga acaaaataga acaaaatgca acaaaaaaat cccaccctca aggagcttac 16200
attctagaag aggaggcagt caattttaaa gataaacaag tacaatacaa aatatgatag 16260
gcagtaataa gtgttaaagt gaaaaacaaa acaggccaga ggatagggag gcagggcaga 16320
ggatacaagt tttggtactg agaacagttc actgtgcatc tttcctgacc tttttctgtg 16380
tctaaacaca aatacattac acacataccc actgtatgtg cccaaacata agacaactag 16440
aataaacgtt aatcccctat tttccccatg aaaatatcca aaattagatt tctggccatt 16500
ttcattatat aaatgtttaa ggatgtagcc aaaatagtca aatgacttca tattgtataa 16560
tttataattt aaacatactt tattttttat attcagttta attactattt ttacattaaa 16620
attaagttct acattagtaa acttaatttt actaatgttt tacattagta aaacaaactc 16680
tgttttttga actttttttt aactggcatg aagaattaca gctcttaggt gttaacatgg 16740
gaagggaaaa gggtatttat cccatttaac aggtctggct agcttgtatt caataaatat 16800
gtgcttgaag aagccatctg aggtaatctt taggatctgt tatgaattta taaagatata 16860
attccaggta atatctttaa aaaatagaga aaggatttat cccctgacta ctgaacggga 16920
caatgcttat gaaaaccttt taccaaccct gtcaagtgtt tattcatgct ctgcaaaata 16980
ttttcagcaa gtatttcctg tgagtttata accaggttca tttatgaatt caagacatat 17040
ttattgagca tgactgacgc tggcgctggc tctggctcca ggcagagatg ataaaacaac 17100
gaatccctgc tcccacagag cttcacacaa ggagaagcct ccaacatatt ttatacagca 17160
ctatcttagc atgttcattt ggaaatattg tatattttct actcaaagtt catgaaatga 17220
ttttattcta ctttctcatg tgcctctttg aaatcagggt cactatcatc aacagagcca 17280
ccttttaaat catctaacag tgttcaagga cacaccaact ttccttcagt cagagttact 17340
tgagaactag ccctttttga gttcgtgtat aatgatgtca ctggaaattt tatgcccacc 17400
acaaataccc agtagctatt tgtgatttcc gcgaagtaat cacctactgt attgctttat 17460
aaagtaattt tcaaaaggct tgtttacaat aacatccaac acttgtaatt gtgaggtcat 17520
cctcctaaaa attacttcta gatctttaaa ttcatttcta tggtttctgg ccaattccat 17580
tgagtgacct caataagcat ctagaaatga tattaagttt cttcaggcta atgtagttta 17640
tccaagcagt actttattga tttgctattt ttgaaaagtt tagtaattga gtaatatgaa 17700
gagatttcct ttcagaagac taaattataa ggccatactc tctttctgaa gtataatttt 17760
tcaggtaaaa tgaatatctc atatagaaga atttatgcca aacatgtcta taagagtgta 17820
tgtatatata tttgtatttt gtttttgttc ttagttaaaa tactgggatc acaagcgttt 17880
tgaatttaca gtcatgaagg aaatgcaaaa gatgatgtct tcatagcgaa aaacccttgg 17940
attttaaata tagtgccttt gccatatatg gatatgcaaa gccataaaaa atttagaaaa 18000
atggtagatt cacagctttt tcttcaaacc atggtaccaa cattctgaca gttcaaaatt 18060
aatgaaaaga aatctcactg gatggagaac aagctagaga ttaagtcaca atcaacaaag 18120
aacatcattt aatctgaata ttcaaataat atagttttaa caattatctc agcaaacaat 18180
aacacaaaaa taaattgaga acacaagaaa aacatccatt ctgaagaagc agagaaatta 18240
ttctggtact ttattaccat ctaaacctta gcgatctatt aaagtttagc atagttttaa 18300
aaattctagt taatttaaaa tcatatgcta acatatgatt tcaatttaac tagaattttt 18360
aaactacaaa tcctttgaat tataggttct ttttgaacca aaattcaact ctaatgaagg 18420
catctgccaa agtctatggg gtcctgtttg tgctaaaaga ctctggcatt tgtttcttga 18480
gtctcattca ttgagtacta gagggtaatt aacttcatta ttgagtttga acaaaaatca 18540
gtaatggatg cacaagaact atttaatatt aatgttcagc tctctaatag agctgtttta 18600
ctttacacaa aggggtttgg ctaggtttgt gttgatttaa agtctcagaa tgaaaataat 18660
tagtggatat aagtaaaaga aaccattggt ccaggcgaag taacggtaat ttttaaaatt 18720
ccaacataca ctttacctag tcgctaaaaa tgagatataa ttttacgctt ttagacaaga 18780
ggagagctgg ttggcactat tcctgggtag cacttttcac caatatttct gataatgttc 18840
catgggaaag caaaaggcaa cagagattct aaaaaggttc cagacaccaa actgtacatg 18900
taaccttgac cttatttatc attatgataa taatcaccgt gtaattatgt gtagatttgc 18960
tctcatacat caacttcttt tcaactttga aaaataaaga gttcatattt cttgacagga 19020
ataaaagagc aaaaaaaaaa aaaaaccact taaacttaat tattttggcc atagctcact 19080
gttagcatat gatttaaatt ttacagtgag taaagaccaa tttattctca aaagccctga 19140
ttattttgac aatatttaaa atgttttaaa taaaaagaac aaaaatgttt tatcatgtat 19200
tacagaaaaa tatttgttat ctattaaaat ctcacacaca accatgccta caaaaactct 19260
tcatgattca gacttaatac ttaagtttaa acttatcaca gaaggatgat atctacatca 19320
agctgcagat atttcacagc aagcatgcct atagttcatt ttttcatttg ttcattcacg 19380

cattcaacaa gtatataaaa ggattattcc agacaaagcc tatcctatca tggagcctac 19440
attctagagg agagagacag acaataaaca aacaagttta aattatgcct aacaataaaa 19500
agtactatgg agaaaactaa aaaccagagc aagacagcat gggagaaggg agtggttcta 19560
gttttatgta ggtcggttac agaatgtctc tttagcagca acctggaaga gatgagggga 19620
tgggcctgac agatatcagg gagaagcact ttctaggaca gagagaagag acacagcaaa 19680
ggcccagaga tgggaacatg ctggttttgt aggaagaaca accaggaggc cagggagcct 19740
ggagtagagt gagcaagggg acagccacag gagatgtggc cccagagaga aagtgtctct 19800
agcccaagca ggcctgggga cccattcagg actctggttt ctgagtggtc ggagaagctc 19860
ctcaggagtt ttaatcacag taataactta tcagatatat tttttaaaga tcactctggc 19920
cactctactg aaaacaaact tgcaatgcca gagcagaagc aagatcagta aagaggctac 19980
tgcaataatg ctttgtccag ggtcccagaa aagtagctca ggccaagctt agctgcagat 20040
gctttatctg gaaggaaaac tccgaagcgg caagagttag ggcagtgagg cagagaagga 20100
gggagtgcat tagcacgtcg gtcaatgctt cccagaaacc cagccaactg ctctggcaat 20160
gagggatttg gtagagccag gctggatggg atgaccacac ctcagagctg tcaggggagg 20220
gctgtatctg ctgtgggaga tgatgagatg ccctgcagta gagacgctac tgggtctgaa 20280
atagcacttc ccatcttctc actggcattt gagcaaggag caccattgcg ggaggaggga 20340
gagagtgtat cttctggctc ctttctcatc ctgtctgtta ttggtcaaaa gtccccccaa 20400
agcgtgactc cccacctccc tgctacctac acacacaagt caggttttgt gatctgactc 20460
ctcaagacag ctactgagca gacagagtgc atgcccacag cctgacactt cacctgagtc 20520
caggtacagg ggtcgacaat cattctgagg gtcaagctcg gccatcaccc cctgacagtg 20580
tcagatgagg caaggtggca ttagtcaggg tgtgacactg aaccagcagc cccaggccag 20640
gaggcaggtg gggccaagca aatccgggca gcgcaaaaat taagtctgac aagacaggag 20700
agagattata gatcaggtgg taaaggtgaa aatggagaga aatgatagag ttataaatat 20760
agtctgaagg taaagcctgt agaactggct gatgtgggtg aaaaagaaga aacaagatga 20820
ctccaaggct caggtgtggg caactagaag aacaaagcca cctgttattg gggtgaggca 20880
ggttacaaga gtggcaagtt ttggaaggga tcagagacca gtagttcagc tttgggcatt 20940
ctaggagaaa acagagatag taagaatgac aatacctgcc taatagaaat attacagaga 21000
agcttatttc aaagttcgtc caaattatga acatagttgg ctaatatgta tagccctggc 21060
gcatcttttc tttcacaacc ccagcatctc cctcagctcc atcactgccc ttttgttgcc 21120
catcagacat ttccggaaac acgcactaag gagcactgtt ttcacaatgt aggtgtgctc 21180
tccttccccg caaaacaaaa gggtgggcga ggggcaggag cagcagggtc cttgcccaaa 21240
tataaataca tatgggaagc actgttttaa gccaaacagc ttctttactt ctcaaaacct 21300
gatacatttc acgtgtcttg agaaggtctc aaggaaagat gttgtgtgga gagtttctaa 21360
acgtatttga acatcaaggt acactgaata aacaaaatcc atatgatcct ctcaaaagat 21420
gtggaaaaag ctttcaataa aatccaacat ttcctcatga taaaaacccc tcaagaaagt 21480
aggcatcaaa ggaacatacc tcaaaataat aagagcatct atgacaagcc cacagccaac 21540
atcatactga gtgggcaaat gatggaagca ttccccttgc gaactgaaac aagacaagga 21600
tgcccattct caccactcta ttcaacatag tattggaagt actagccaga gcaatccctc 21660
aacagaaaga aataaaggag ccaggcacag tggctcacac ctgtaatccc agcactttgg 21720
gaggttgggg tgggctgatc acctgaggtc aggagttcga gaccagcctg gccaacatgg 21780
tgaaaccccg tctctactaa aaatacaaaa ttagccaggc atggtggcgc atgcctgtag 21840
tcccagctac ccaggaggct gaggcaggag aatcgctgga acccaggagg cagaggttgc 21900
agtgagccaa gattaagcca ctgcactcca ccctgggtga cagagtgaga agacgtcaaa 21960
cgatctctct ttgcagatga tattatttta tacctagaat actctaaagt ctccaccaaa 22020
agtctactgg aactaataaa tgacttcatc aaagcttcag gatactaaat taatgtgcaa 22080
aaattagaag catttctata cacaaataat gttcaaactg agagtcaaat caagaatgtg 22140
atcttcttta taacagccac aaaaaacata taaaaatacc taggaataca tctaaccaag 22200
gaggtgaaag atctctgcaa ggagaatgac aaaatactgc taagagacat catacaggtg 22260
acaaacaaat ggaaaaacat cccatgctta tggattcaaa aaatcaatgt tgttaaaatg 22320
gtagtactgc ctgaagcaat ctacagattc agtgctattc ctgtcaaact actagcatca 22380
ttttttcata aaattggaac aaactattct aaaactcata tggaaccata taaaagcctg 22440
aatagccaaa gcaatcctaa gcaaaaagaa caaggccaga ggtatcacat taatggactt 22500
caaactatac tataagacta cggtaaccaa gacagcatgg tactggtaca aaacagacat 22560
atcagacaaa cgaaacagaa tagataaccc agaaataaag gcacacacct acagtcatct 22620
gatcttcaac aaagtagaca aaaaaaatgg ggaaaggact cactatttaa taaacggtgc 22680
taggatagct ggctagccat atgcaggaga atgaaactag acccctacct ttcaccatat 22740
acaaaaatta actcaagatg gattaaagac ttgaacgtaa gacctcaaac aataagaatt 22800
ctagaagaaa acctaggaaa caccactctg gacattggcc ttggaaaaga atttatgact 22860
aagtcttcat aaacgattgc aacaaaaaca aaaattgaca agtgggacct aattaaacta 22920
cagaccttcg gcacagctaa agaaactatc aacagagtaa acagacaact cacagaatgg 22980
gagaaaatat ttgcaaacta tgcatctggg aaaggtctag tatccagaat ctaaaagaaa 23040
cttaaacaac tgaacaagca aaaaacaaac aaccccactt aaaaactagg caaaggacat 23100
gaacagatac ttctcaaaag aagtggccca tcaacaaaat gaaaaaaaat agtcatcatc 23160

actaatcttc aagaaatgca aatcaaaacc acaataggac agcatctcaa accagtcaca 23220
atggctacga ttaaaaagtc aataaaaagc taggcgtgat ggctcatgcc tgtaatccca 23280
gcactttggg agaacgaggc aggtggattg cttgggatca ggagttccag accagcctgg 23340
tcaacatggt gaaaccctgt ctctactaaa aatacaaaaa ttagccaagc gtggtcgtgg 23400
gcacctgtaa tcacagctac tagggaggct caggcaggag aatagcttga acccgggagg 23460
cagaggttgc agtgagccaa gattgcacca ctgcactcca gcctgtgcaa caaagcaaga 23520
atccgactca aaaaaaaaaa aaagtcaaaa aaaaaaaaaa acgcagatgc tggcaaggct 23580
gcggagaaaa gggaaccctt atgcactgtt gatgggaatg taaataaatt cagccatggt 23640
ggaaagcagc ttggaaattt ctcaaagaac ttaaaacaaa actaccattc aacctcgcaa 23700
gctcattact gagtatacat gcaaaagaaa gcaaaatcat tctaccaaaa agacacatat 23760
acttgcatgt tcatcacagc actagtcaca atagcaaaga cgtgcccatc aatggcggac 23820
tgaattaaga aaatgtggta tatatacacc attggaatac tacgcagcca taaaaaagaa 23880
caaaatcata tcctttgtag caacatggat gcatctggag gccattatcc ttagtgaatt 23940
aacacagaaa cagaaaacca aatacctcat gttctcacta ataagtggga gttaaacatt 24000
ggatactcat ggacaaaaag atggcaacaa tagacaccgg ggactactag agagagaagg 24060
ggggcagcaa aggttaaaaa actaactgtg ggtactatgc tcagtgcctg ggtggtggga 24120
tcattgatac ctcacatctg agcatcacac aatataccta agtaacaaac ttgcacatgt 24180
accccttgaa tctagaagct gaaaaaaaaa aaatttaccc ctaaccccaa aaaatacttt 24240
gcagtgaagt aagcacacat tattcctcac aagaaaagat aagtaattca attgtctaat 24300
gggaagttaa agttctcttt tactgaatac tcaaaattca tcttacgtat ttcttttttg 24360
tatttcaagt ttattaactt ccacagctgc cagagaagaa atgataaatt tattttaata 24420
ggacaacttt ttacgttgct tcactttaaa tatgacttgc tatataagag taactagaag 24480
gaaagaggca tgaaaaggaa aattttataa tcccaaagac aggtatgtat aactgaaaaa 24540
aaaattaagc ggttcatagg ccagccccta acaactgatg ctataaaaat atccttaggt 24600
gacattcata tctacataca aatacacttc atcgtaatgt agttgcctga ttcagttaca 24660
aacacaccca gttacaagct ggtctagtag actcccaagt tggcatgtaa gtcgttgagg 24720
aacaaagaca aaagagattc agaaaatgaa taattaatac gtgacaaatg agatgcttag 24780
cattttaaaa gaaaatatta aggctgagta attcttacta aacacaatga agtattatgg 24840
ggtaaaactc aaagccaact ccttggaaga gctttagaag ctatttggca gacaggcctt 24900
tttgctttta aggctgactc caatatttga ctatatagaa aacagtcatc cagctagagc 24960
tttcctttat tcaccacctt aaaccacact ttactcttct cctgatacta cattttaaaa 25020
tgagaaaaat acagaatctt atttgcatat atttgcaggt gaccttgcag gctccactga 25080
tatataacct gtgacactac ttcttccact gctttctgaa agaagatatt ttcacttgga 25140
agattctcat tagccaaaac tttgtagact gtgcagacat aaatatcaaa gtacacataa 25200
gtagtttctg tataggtcaa atatgtattt aaaaatgtaa tagacattgt tatactgtgg 25260
ccatctagaa tctttgccta tgtttcctgt gtttgaaaat ttgccacagt atggatcctt 25320
ccttccccaa atagaagcca gaactcacct tctcagcttc ccttgtctgg ccgtggtatc 25380
tagtgtctac caaccacctg catccacatg ataagacctc agtttagagt tagcagtgtg 25440
aggagagact tcaattcaga ggtagcaatg gaagaactta aacaaacgag gaaacccatc 25500
tttctggcaa gggaggtggt aagaggcatc cacctctcaa gggaaaactg cgacagcact 25560
tctggcttcc agaacccagc actgttggtg taagctgtga tgtcgggata aagcttcact 25620
gtggccatac ccacactata gtgtgggcag tgctcctgtt cacctaacct gtttgttggg 25680
tgtcctggag attctgtgag tttccaaatg tttttaattt tttttttcct gattatgtta 25740
gctagagtgg attctgtggg ttgcaactaa gaactacata taatacaaaa aaagaatctg 25800
ggctaaataa accttcatca tttgataaaa cgatagcaga aggattatca attcaatagt 25860
aacctcaata gtaagctaga aagagtgtgg tactgtgcta gaggctgtac aggaacagaa 25920
aggaacagca aagaatgtta catttaagcc atgtaaatac atgggaaaat agatataaat 25980
aaagttaaat gaaatttaga aaatacagaa gcatctatgt agtgactgta aaaatgcaac 26040
agcatactgg atatgctgat aaggatgaaa aacttttatt gacatatttt ttaaatgttc 26100
actgggtttg aggcaaatct atcatgttgc taaagaaatt aaatatactt tttataactg 26160
aaaattcagt aatgtgggga aaaatatttg caaatcatat atccaataag acattaagat 26220
ccagaatata taaagcctac aactcaataa caaaaagaaa attccaaaat gggcaaaggc 26280
cttgaacaga cacttctctg tattgaagaa aacaaatgac taacaggcac atgaaaagac 26340
cctcaacaac ccggcacagt ggctcacacc tgtaatccca acactttggg aggccaaggt 26400
gggtggatca cctgaggtca ggagttcgag accagcctgg ccaacatggt gaaaccccat 26460
ctctactaaa aatacaaaaa attagccagg catggtggca gggacttgta atcccagctg 26520
ctcgggaggc tgaggcagga agatcgcttg aacccgggag acggaggttg cagtgaactg 26580
agatcgcgcc actgcactcc agcctgggca agaagagcga aactccatct caaaaaaaaa 26640
aagaaagaaa aagaaaagat gctcaacatc tttagttatt aagaaaatgc aaatcaaaac 26700
cacaatagga tatgatttca tatccatcag aatggctatt atttttaaaa aatggaataa 26760
caagtggaga atatggaggt agaggaaacg gagtgctcat gcaatggaaa attgtgccgc 26820
tgctgcggaa aacaggctgg tggttcctca agagttacac agtattatca tatgatccag 26880
caattccact gctaggtcta taccccaaaa cttaaaagta aggactcaaa caatacttat 26940

ataccaatat ccaaagcagc actactcaca atggtcagca ggtaattgat acaactcaag 27000
tgtctatcaa cagatgaatg gatacacaaa attgtacagc tatacaatgg aatattattc 27060
aaccacaaaa agaactgaga tattgataca ggctacaaaa taaatgaact ttgaagacat 27120
tattctaagt gaaataagcc agacacaaaa ggacaaatgt attattctac ttatgtgtgg 27180
tacgcagagt agacaagttc ataaagacaa agcagaatca aggttaccag ggactggggg 27240
gaaagggaat ttggagttat tgtttaacag gtacagagct ctgtttgaga tgattaaaaa 27300
gttttagaaa tgaacaaggg tgatggttgc acaacattgt gaatgtactt aatgccattg 27360
aactatacat ttaaaaatag tttaaatgat acatttcatg ttgtgtttta ccacaataaa 27420
aaaattccat tatgaacctt atctagaaat cagtcaaaaa tgaagttcaa tattattcct 27480
tttgtgctgc ttattatatt atttaatgta tagatccaag gaagttagta gttctaacca 27540
gaacaacggc aacatagcat agttaaaagg tcaggtaggt agacctaggt taaaatacca 27600
gttctaccgc taactagttt attacatact taaaaaagga cctgaattta aaagatccct 27660
aataaacact aaagttagct gctgttatta gtctttatta ttatattaaa aaccacctgg 27720
aaaggagcaa tgcattttat tacaggtaaa ttttaaactt tagtacatca aataagtgaa 27780
catgggaagg caaacaagtg tggaaaaata tttgcatcaa tatgattaag agtctatatc 27840
cctaatatgc agatttccac cgaaaagatg agagagcaga gaggtacagg gaggcaggaa 27900
agttgctttg gatcacaggg tcagccaggg aaggccaata tatgaactaa gacatgaaat 27960
gtgaaccgaa gctagagtga aaaagtctgg ggttagggag cactctggat gaggcaacag 28020
caaaagcaaa ggccagaaca tcgaagaact tcgagggttt gaggagtaga gagaaagaaa 28080
aacagtaagc ctcgacaaga aaggctgagg aaagaattca gggaatcgtt taattagaat 28140
ttcagctttg ggtgttgacg gtgaagcggg atgctgaagg gagagtggca gataaaattg 28200
aagagaagac agaagccaga actcacaagc ctgttaagat gggactgtga ttacaatctc 28260
aaaagcaacc atttaagcaa cgaagtaaca tgatctggtt tactgtgcaa atgatcatgc 28320
tgcctgctag aaggaaaacg gagtagggag gcagaatgga agtaaggaga gaagctggga 28380
agctatgtta ataatttaaa ttacagaact taatagcttg gattcacaac agtagaactg 28440
gcaatagaaa aacgtgtaca aattcacatt gcattggcta ataggagaga aaatatttta 28500
ctctttcatt aatcccaaaa ctgtcactta aaattacaca atttccccta acaaattagc 28560
aaggattaaa aattgataat acatatccct gatgaatatg tagttaaggc actcaaaaat 28620
ctctgaaaag caatttgcta tcctctctct caaaagcctt aaaaattctg tttttcactt 28680
agtaactagc acacagttaa gtcttactgt tgaattaatt ccaacagtta gtaaacatgc 28740
ttaaagagaa aatcggcatt atgctcaaga atgtaactgt agcacactgt acttgcaaaa 28800
aaactgaaga atgatctaaa tattcgacac ttaaggaatg gctgaattac agcttatgat 28860
aaagtactaa gcaattcatt aaggagtttc aagatatggg aaaacgcgga cattatatca 28920
ctttagagaa ttaacaaaga gtaaatagta ctagagtttt gctttgatgc tgctaagttt 28980
taaaatgaga aataaagata gccggcaaac aagtactaaa aacttaatga tcattaacct 29040
agtctagaat tgaaactcaa taaatctgct caaaaaactt atacgctaaa cccattaaat 29100
attgtctgta tcttacagtt actcttgtta ctcttggtcc taaataagga agcaggaaag 29160
tttcagttcg gtggttgtct tagtaattta agaataaaca tgcaccttca aagcctgttt 29220
gtcattctgt atccccagct cctagcacaa aatctggcac agaataggag ctcaataaat 29280
aattttgagt gaatatcacc aaaagtaaaa ctttcagttc caactcagca tgaaagaaat 29340
caaaaacctc aaaatcaaag acgtgcataa aagtaatagt ttgcttgata atatctcgtg 29400
atgtcgtttt aaagtaatag tgataataat gtataatatt tggagtggtt acaatgtatc 29460
aggcactaac aagcacttga cagactttaa tccttataat aatcattctc ttttaaaacc 29520
aggaaacagg ttcatcactg ttcacgaagt agcccaacgt cacactgcca ccaagctgga 29580
actcagttct ctggcatcta aacgcagaac tacagaagac acgtttcttt ccaacgatca 29640
gacgtccaag ttcttcaagc ttcctctacc atcctattta gcattcgcaa acttctacct 29700
tgaattgtag ctattatccc ttccaagatc catctctcac caggggagcc cctgacactc 29760
cctccctccc cagctgggaa gccccccagg atgaaggacc gagtctccat gcttctcggt 29820
ggctgcacac ttagaatggg ggcttggcac caggcgaggg tctcgcccct ttgataccct 29880
ttgaggttga tttactgcct tgcgaatccc gactcccaac tgggcccttt gaggcgccat 29940
gtgcggcccc tcgagccaga ctcggcgcca ggagaaggga gtcagcgtct ggaggggtgg 30000
acacggcggc gcgcaggagc tgtttccagg acaaccgagg gccgccccac agtgtccgcc 30060
ccacagtgtc cgccccttgc gtctcgccgc tccgcccctt cccggcgcgg gtgctccaac 30120
cagcccccgt gagcgaatag agagaggggt gctagatgga ctctcacctc tcagcgcgtg 30180
gtgcatgccg cccacgccgc tgtatagctc cagcacccgc aggggctcca tccccgcgcc 30240
tcagccgccg cagccccgga gctaggcctg ccggtccgtc gctcctcctc cctccatcgc 30300
gccgctccct cagcctctgg ttccgcggcg tttcagttcc cccgtctcct accgctccag 30360
gctcagggct tcagaggccc tcgcggcctg gggattggca agggtctaga ggggaacaaa 30420
ggacggaaaa caataaggct tcccgaatga atggcagcca ctctgtagcc tgatttacgc 30480
tccatttcct tctttggtgt caggggcctt gattggaaaa gaaatccaga gttcagtaaa 30540
ggggagaaaa ctgccttggt ttccaagtga attaacactg gttagaagcc agaaaaaaat 30600
catttttcag acaagctgct tttctgtcac ttagaagcct gaacattcaa acattctttc 30660
cacagtgtgc cttgtttcct gcctgccctg ggtcaataga gagaaattag accagcctct 30720

cccctagaga agattccaga attataagta aggacacaag tccactcgca gcagagaaag 30780
ccctgtgtgc gtgctgataa tacagaggat gtttgttgtg tctattagtg gggttaatta 30840
tgttttagct tggaaattac acctacatag aatacacagt gaatatcatg taggattatt 30900
tatgggtctt taagtgctat ggccttgatt cctcaggatg atccagtaac caactgttag 30960
aggctcatgt tgacgaaaaa agccaaactc cgtaaaatgt ttgaagaggt ttattctgag 31020
cacaaggtga ggaccgtgac ccatgacaca accccacagg gtcctgacaa catgtaccca 31080
aggtggttgg gctacagctt gggattatat ggttttaggg agacataagg acattaactg 31140
gtatatggaa ggtgtactta gtttggtcca taaagatggg acaactcaaa aaggggaagg 31200
attccaggtt acaggtggat tcaaagattt tctgattggc agttggttga aagagttaag 31260
ttgtgaccta aagacctgga atcaatagaa aggaatgtgt ctgggttaag ataaggtgga 31320
gaccaaggtt cttattatgt agacgaagtc tcatacgtaa caggcttcag agagaataat 31380
tggcaaatgt ctcttatcat gtgtaaaaag atgccagact cttagttaaa tatctcttgg 31440
atcaggaaaa gacctggaaa gggaaaggga gtctctatag aatgtagatt ttccccacaa 31500
gagatggctg tgtagggcca tttcaaaaag tgtcaaagaa atttatttta gggtaaaatg 31560
ctttgattta gggcctgcta tctttcatgt gatgctatac tagagtcatg ttggaatttt 31620
ggtatcttat tgctaagaag agtctgttat gtcggtatga agatctcttg ttttaataat 31680
aatactggtc agttgtgcct gaactccaaa gagaggaaag tataatgagg catgtctgac 31740
ttccctttcc cagcatggcc tgaactagtt tttcaagttt ctttggaatc cgcttggtgg 31800
aggtgttcaa tcagtcagtt ggggggagct tagaaatttt gtggggggag cggtgtttac 31860
agtcaattat aaataaaaat aatgtaccca tatggaggat agaaaatgtt cccgttgtga 31920
agtgtttttc tctttgttcc caacactgcc acaggttttt gttaggggga acttgggggt 31980
accacttgta ataacaataa ctgatttgtt agaaatgttg catgcctgct aatgtaatgc 32040
tgttaacaaa taaaagtata accactgcat ccaaattata acattcactg gttccttttg 32100
atgtggtcta tgaaattaac tttttaaatt gtacatgtgc attggccacg tgctttgctt 32160
tttgttagtc actttcagat aagaagaggt gtatggtaga aagataccag ataactcagc 32220
tttagccctg gcgttgccag ttaactgatt gtgtgacttt gagatgaccc tggtttcctc 32280
atctgtaaga taaagcagaa gggaacagaa agagatggat tgttgagggt gttttagttc 32340
caagaaatga attcatggct gaattcaaat tagaaaaaat gagacctgat ttggcaacgt 32400
gttaattatt tattgaattt gaaatatgta gaaagaagac ttgttacctg ctttaagcaa 32460
atgtactctt aaactaactc agcttacaga aaccatgtac acactgacat atgtataatg 32520
catgaagcaa actttttaaa tatttattac atatagtacc agaaataaac atcagtacat 32580
aaaatgtaaa gggaactata tagtcacagt gatttatata tagccaaaaa ccaatatatt 32640
aaacaaatct ggaattttca gcattcgtgc tataaatcaa aataaatgtg acaaaagacc 32700
ttacctgtag gggagagaca gtacagacac atggcagagt taatgaaaaa taccaacctc 32760
ctgagaggaa ggagcctcaa accccttccc cacacaggct gattacaaac tgtttactct 32820
gatacttaag gatcttcacg gtatagccct gaacttactt ttcttcctta acttttttct 32880
cttcttgaac tctgctgtaa atgaaactac ttggtgttcc ccaaacatgc ccttcagttc 32940
acttctctgc ttttgttctt tcccttctct agaaatctcc tagacttctt taagacctat 33000
acttcaagtg gaaccttcct tctagactcc cagcctaaaa tatcctgaca cattgcaact 33060
cctaggtctc cctcatcttc tgaaatacag atcacccttg tacttgtcat tcaatccttc 33120
cagttattct agcccaattg tcacctcata tgtgccacat ctttatgtat tatttcctct 33180
ctgaggctta tctcgtgata attatttcaa gaatgaaaac tctctccata atcataattt 33240
acaactatct ccatggaagc atctgtagat attaggcccc ctgggattat gtatgcaaat 33300
gtcttccaat cactggcaca aattacatga tttggtggaa gagtgggagg gctggtggag 33360
gtggtacaat agggagtagc tgaggagaaa agtgaggtga gcagaatgaa agtaaatatt 33420
gattactttt tccgaaagca aatcccgaag gctcataaga gagaagaaaa actctctagt 33480
cccataattt cccataaagt catcctcaat gatatgtaca atactattta tattttaatt 33540
agagaaacta gacatacttt taaaaaagca agcaaatatc aaaactggaa tgttagtata 33600
tttactacat tttctctaaa agcagaaaat gcgtttcttt aaaaaaaaaa cacattaatt 33660
aacacctgga aaataaaggc acatataaag catatcagaa ataattaagg ggcacatcac 33720
aatactaagg accatagtaa aagagtaata gcacacactc aattattgaa ggcactgggc 33780
ttggaatcta atgacatttt tatcttctac ccttatgtgt tctttcactg atcattaata 33840
tctaattaaa tgaaataaag ttttccaagt gctgaaaatg ctttttcaat catctttgaa 33900
tttccgtgat ccaggccact ctctttctca gcaaaacatg atgtggattc ctatacaggc 33960
tcgcttacag tctgaggaca ccctgcttat gccaatttca gtcatgaata agttgcattc 34020
tggctttata ccaaaaaaga aggtctttat gaattgaatg gaaaaagaaa aagtcatatg 34080
agaagaaact gactataaga aatgtcctca agagagtaaa ttttacaaat ttgaattgga 34140
aagaccaaca gagctcattt accacttctg agctcccact tatttgagaa tgccttctac 34200
tattttcctg atccatgggc aataagcctc agcttgcaaa ctgctaatga aatgtttcat 34260
ggtgaacagc tggaattatt agaaaattct tccttatagt ctcctaaatt ccagtttctt 34320
ttagatttgc cctctgcact gattttaaag aattattcct cttctggctg ggcactgtgg 34380
ctcatgcctg taatcccagc actttgggag gcagaggcgg gtggattacc tgaggtcagg 34440
agttcaagac tagcttggcc aacatggcga aacccatctc tactacaaat acaaaaatta 34500

gtcggttggg ggctgggtga ggtggctcac acctgtaatc ccagcatttg ggaggcagag 34560
gcgggtggat cacctgatgt caggagttcg agaccagcct gaccaacatg gtgaaacccc 34620
gtctctacta aaaatacaaa aattagtggc tcatggtggt gcatgcctgt aatccctgct 34680
cctcgggagg ctgaggcagg agaatcactg aacctgggag gcagaggttg cagtgagcca 34740
aaattgtgcc attgcactcc agcctggtcg acaagagtga aactccatct caaaaaaata 34800
tatatatatt ttttctcttc tataatgatt tgctttgtgg ttaagaatgc aagcttcaga 34860
gccacagtgt agacaggaag cacacgtctg ctgctgcttc tccctagcta ggtgacgtgg 34920
agaaagtcac tacatccttt tttgagccaa agtgcacctc taaaatgtat aaataatagg 34980
acatgcagtt gctatgaaga ttaaaagccc ttagcccggt gcctagcaca atacaaataa 35040
atgtatgtta aataaagagt gcctgtgcaa tgacatcctc taagtgatgc attctcagtc 35100
ctgtcggcta tgaactatca cttggcatga ttcagatccc tcgcattagt cacagtccag 35160
cttggtgata cccattttag gagccaacac tgcatgaaac aaactgaaca tagatcagca 35220
tatggtagag cgaaccagat ggctacagac attcaagggc tctagggcag ctttaggccc 35280
tgtaggaagt gttcagggtt cctcggctta gaccatcgtg gagtaaagac tgaaagtctg 35340
gaagctacct cagccctgac cgagtaggtg ctgagcatgg aaataaagaa gtcagacaat 35400
agacagcaga attcatagta aaatagatac cgcttaactc ttaacttcca gaagaaccag 35460
gattgctgtg aaggccagag ctacagctct gacaatcttc ggggaaaatt ttttcagtga 35520
ccttaaccag gatgcatgaa ctggggagat ctggggtgca gaggagggga acctatctta 35580
tagcagctag tcatgtgttg actctttcat acgctaggca ctgaggagat ttgcaattaa 35640
aaaaatttaa aaagcagccg gagtggtgga tcttgcctgt aatcccagca ctttgggagg 35700
cctggatgac agagcgagac tctgtctcaa aacacaaaca agcaagcaag caaacaaaca 35760
aaaaacttac aaagtgcaaa actttataat ctatttcagg ccataaaagt gccaatagta 35820
aaaaatgcaa ttgaatactt tcatttccta agcatagggg attttttcta atttaattga 35880
ttctcactat ccaataggat aaccagtagc cacatttggt aaattaagtt taaattaatt 35940
taattaacat taaaattgga ttaattatac taggcatata ccaagcgctc cgtattgctc 36000
agcacagatg tagaatattt ccatcatcac agaaagttct atcgggcaga gctgctatgc 36060
agtgatggtc ccttttagtt acagccttta ttaatagccc atcaataaat gaccagtgtt 36120
ggaatgtgtg catcaattat tgcatactga gcaccaattt taaataaatg tcccccaaaa 36180
tatggggcaa aatgaactca aattaaagca aaccttacag ctaaacattc tttctctttc 36240
tctatcatcc agtcacctta ctgataaact gtcatggagg aaaagaaaca ggaacagcag 36300
atgtgttctg tcagtggatg tgataaactt tcaagagaaa aagaacagat ggaaaatgaa 36360
cttcatgatg tctttaagga gtagtacttt tttttttttt tttttttgag acagagtctc 36420
actctgtcac ccaggctggg gtgcaatggt gcaatttcag ctcactgcaa gctccacctt 36480
ccaggttcaa gtgattctcc tgcctcagcc tcccaagtag ctgggattac aagtgtgcac 36540
caccacaccc agcttttata tttttagcag agacggggtt tcgccacgtt ggccaggctg 36600
gtctcgaact cctgacctca agtgatccac tcaccatggc cccccaaaag cctgggttac 36660
aggcatgagc caccgtgccc agccagaagt agtacttttc tgttataaag tatattctag 36720
tgacttctga ctgtgaataa tgttatctta cacttatctc agaaggaata aaaagaaaac 36780
atgtttttaa aaattaggat catgaatcat gaataaagtt aattatacat tttctgccaa 36840
ataaaataga aaccatgccg tttgcctcat actttaccat ttcaaactac tcatagattt 36900
ttaacatgga ttttgctctt ggaggctcat tctgaaattc tcaatttaaa aatattttgc 36960
agcaaattta aaagatgatg tttcaaagta aatatagtgg gatccattct ttaaaactgc 37020
tttcttaact tcacagaaaa ttagaactag aaagtatctt aaggactatt tgatacactg 37080
ttttttaaac tatttttaga gataaagcct tttgttcaaa ccaaatctcc tctggaatgg 37140
ggagcaaaga tagagaggta gagcaacagc tacactgtaa cttagtataa aagatactaa 37200
gtagactcag ttggtcctgc ctgctgcccc tctacaccca ggcaaggcca gggatctaaa 37260
ccaaagcaat cataggagcc acaatatgtc acttcctgag agcaggctga cctgaggact 37320
gaggggaact tcagagatct atgaagcaaa cttgatctga tatggtaatg catgctataa 37380
aagccagaca aaaatgttaa catagctgca caaaatagag actatctcag aaactaatgt 37440
ttaacattga taacagatcc cctgcccaat cactcctgca aagacagctt gatacaggac 37500
aaagaatatg gtccttagaa ctagatggat taccatggat cctgcctcaa atactccctt 37560
tcttcacata ttaaacggga aatatttgat aacaaggtaa ctgtgtgtgt atatatatat 37620
catgtgtaaa taattgtaca catatcaatt atatatgtgt acatatcaat atatacatat 37680
atcatttata tgtgtacata cctatatata caatgtgtgt atatagacaa tgtatatatg 37740
tacatttcaa tataaacaca atgtatataa gtgtgtatat atgtatatat atcagtatat 37800
acatatgtac acacacacac tgatagatat atatcaataa tgtgcctaga acaataaata 37860
ctggtttcct ttcactttct gaacaacttt gtcataatat agaacccatt tcttctaaca 37920
atagggagaa ttattttcct agtatgtttt cataaagggt atactaccaa gagattaatt 37980
tgcatattgc ctaacaagat gtctttctgt attgttttct attatagcct tcaatctttt 38040
caggtggaaa aacacgaccc atgttgagaa tataggagac agaatttagt accttggaga 38100
gctgttagta ctttgctcct tttctcttgt aatttatctg aacatttaga actgctcaag 38160
gtaaccaaag aacacaaatc agacctactc acaccaggaa agggaaaaca cacatacaca 38220
tacacacata cacaggcaca cactccttca tagtcttttt tctgcattgc tacatcatca 38280

gaactgataa actaaaaccc agaaaaaatt caaagacaaa gacttttaat tttaccctac 38340
ttaggtacta gtttaggtag acaacagtat cacttagaat tgttctcata tgatccaata 38400
atgacaaata taactaaaat gtaataacac tttcatagta ctgccatgac tgtatctatt 38460
cactctgtaa gaaaaaaact tcctattatt taaaattatt aaaacgtttc ttgtcagttt 38520
caagtcactt acgtgagcat attacaacat acaagcacat ttcatttttc aaactttctc 38580
ctctagcctc atgctctaga ggctgtcaac ctttacttta cagagttaaa cttattcctg 38640
cagatcaaga ctaaaatgtt ttatttccat ctaaggtaat tactgctaag ttaaaatgta 38700
taaatcctcc aatgggattg gtactatgtt tagtcactca gagaatactg gctgatgaaa 38760
atagaccaca ttccaaaaga cagaaaaagt atgtttgact aataggaaaa taataataac 38820
acaataatga cttgtatgtt tcaaataact ttataaatgt ttaaaatgct tattaaccca 38880
ttttagacct atcggctttt ctttcttaat attcactttt ttacaaataa aaagtagaaa 38940
acttcatgtc atttgctcaa agtcacataa gttaacattc tgcaggttaa cagatttatt 39000
tgcaaatcac acaaacatat ttttaaaaga agggaattta gacgataaca ctagtttttt 39060
gaggaaaaaa taggctggaa ctcatcaaat aaaaatgaaa gggagttagt atatatttga 39120
ggaaggattt catattagga gttagacagg attctggtta ggaaagaatg tcaggaatct 39180
gcatgtcttt tttttttttt ctcctttcat tttaggacca aatcctagaa tagagtgaaa 39240
aaagaatgca acaaacagca ttatttatat atctttgcac agttttaaaa tcaaaaagca 39300
gttttatcgt gttacttgtt ccctcgagag atgaggcatt attatttgga aaataaattt 39360
ttgattatca cctaaagtga aagggactta ataaacaaac attggaaaag cccaaagtac 39420
tgaaagctca atataactgc aagagtctct cttctgacca ttttttctct tttttgctaa 39480
tagtaaaata ggcatgcagt tgtgcgacct cagctcactg caacctccac ctcctggatt 39540
caagtgattc tcctgcctca gcctcctgag taggctggga ctataggcat gcaccaccac 39600
acctggctaa tttttgtatt tttggtagag acgggtttca tcatgttggc cagactggcc 39660
ttaaactcgt gaccttaact gttctgcctt tcttggcctc ccaaaatgct gggattacag 39720
gtgtcagcca ccgtgcctga ccatagctaa cattttgagg tttctccccc atagtctttg 39780
aactaattgt attttaccca aatcagaatc ttattgcata tgttttatac ctagctcttt 39840
ttcacctaat atgttatcat tatcagttct gtacttcatt aaacacatca aagatatata 39900
ccctttttaa atgctgactt tatttttctg aattttaaaa ataatacatg ctaatttcca 39960
ggggtaaaac aaattacaaa agtatacaaa aattaaaggc acttccttct gttaaccttg 40020
attccatcct cccccttaaa aaaaatttta agtttgttga atgtctttta ataaagatgt 40080
ttatttccaa aactatgatg aggcagtcgc agacattttt gacatgagct cagagttgta 40140
cgcatatgtt tatgtggagt tttcatattt aaaaatattt atgcctgaag tgcaaaaaat 40200
aataatttaa agaaaataca taatacattt tgtttaattt gtcttaagac tgatgtattt 40260
tatatacatg ggaatctgga atcatcttac aactgagaaa agttagcagt taacttcctc 40320
tgaacgttac attttaaaca cattttagct cccaaaacaa tatgtcaggc ctcagatacg 40380
cactctttca aaattctgcc tatgaggaaa atactaaata tgaacatttc aaaaataatt 40440
cacctttaaa gctctatttt gtgattagac tgagtacctt gaaagtcatt attacaaccc 40500
ttgcgtgtgt aaagcatctt ctcctcagca gcctcacctc ctacacgatg atggtctgta 40560
aagtcagggc atccacaaag tgatttattc ccccttttac ctttaatatg gcgctataga 40620
ccacattttg gccattgcag tgcagtagct caggtctaca atcccagtgt tttgggaaac 40680
cagaacagca ggatcccttg aggccagaag ttaaagacca gcctttaaac aaaacaaaac 40740
aaaacaaaac aaaacaaaaa aatttcatta gccaggtgtg ggggtgtgtg cctgtagtcc 40800
caggtactca ggaggctgag gcaggaggat cacttgagcc aaggagtttg aggctgcagt 40860
gagctatcat tgtaccactg cactccagcc tgggtgacta gtgagacctg tctctcaaaa 40920
agaaaaagga aaaaaaaaat gagtatattt tgcctaaggt acccaaacac tagaaacata 40980
ttccaaagtg aaatatagca gtataatgca agttttccaa aggaaggggg gcagaaatag 41040
ttacataatt cttacaacca tatacttatg ttgaaggtat aaaagagttg aagattcttg 41100
aagacaaaga gaaggtcttt ggagaaagtt atcagcatgc tatagaggct gtctgcttct 41160
cacctcaagc ctgagacaag gagattcaga gaaccaccga gagagtttaa aatgtaacca 41220
tctaaccttt gagtttgggg aggcaaagta attcccttgg atagatctga gccatacaag 41280
acagaaagct gtcttaagcc ctgttcaagg ggtcactcac aagaaaaaaa ggtgtcatgc 41340
aaaaaggaac aaggggtata ccactgaatg actcctaggg gctgtggcag cagtcacaga 41400
gaagcaggac acaagggcat gtctgcatgc cagggaagct ttgagaaaga gaaagagagg 41460
gagagacaga gagagagaga gagagagaga gaaagagaga gagagagaag aaaaccccaa 41520
agaagcacct gctagaaaaa gggccagctt tgagcatatg ccaaaccttg aggtgtaaac 41580
accagatcac caatagccct tgtcaagtaa gagttttctt atctcctgca cctctttccc 41640
tctgtgagct ctaatcctgc agtggcaaga aaccttagtt agcaaaatga gggaagagga 41700
agggaggcac ctaagtaggg agagtgagaa acccaggact tccctttcct aaggcacagt 41760
caagccatgg gagggaggat attaactctt gggcaagatt gaaattttga aagttacata 41820
catgtaagcg ttgtaaatta ctatgatcac ttttctcctt agaagtgatc aaagaatggt 41880
ttggttttaa taaatgactg gaaaagtcac aggagctgca caaattttca tttgtaaaat 41940
gatcgtttat caactgctct cttgtcttct tattccttcc tgcccttggt ttttggacac 42000
aggttctttt ttctcctctt tatgccatag tttaataata tcatcaccaa ccatcaacac 42060

ttattgagga tttactctag gccagacaat cgattatgta ttttatatac atttctcatt 42120
tcatgcacat acaactgtta ttatcacctc cattttttat atcaagaaaa tgaggctctt 42180
ccaaggtagc ataattacta agcataggag ctgagaaatt taccttgggg actctgactc 42240
cagagtccca ctttttttcc ctgaaagaat gaatgaaatg tattttctca gttcaaagga 42300
cacaaagtat caaatttgcc ttttttttta aacaaactct gtcttccaga ccggagtgca 42360
gtggcatgat catagcttac tgctcactgc agcctagtcc tccagggctt aagtgatcct 42420
cccacctcag cctcctgagt gactggtact acaggcatgc aacaccatgc ccagctaatt 42480
tttaaaaatt tttcatagag ataggttttt gctatgttgc ccaggctggt cttgaattcc 42540
tgagctcagg tgatcctccc acttcagcat cttgaagtgc taaaattata ggcatgagcc 42600
accagacttg ctttctttaa taaaacagag ctctcaaaaa aatggatctc ccccatctca 42660
tgagaaaatt gaaacattcc actgaaggag aattaacaga aaagtaaaca tagctacttt 42720
ttgtaatcag aaaacttgct gatgtatact cttattccaa acccctggat gatgtcatta 42780
tgtcaacagt cctttaggat tcctcaaccc agacataaaa taacagtcag agcactagca 42840
tatccaaaaa tggtattgct ttatttttta aaaatatatt tcttttttga aatatcaatc 42900
gaatcaattt tattctcaca acaatcctga gtaaatattt ttaaaaaata tattttttga 42960
ggaaacagat taaaagcttt aaaaaatgtg attcactgaa aatcaaatag tgaatacatg 43020
atagagtcag cactaaaaag gcagatatat caaagagaga ctgaaataag caactcattt 43080
tttgcatcga cttcgaaaca cctctttacc atcagtgttt gtaataatca ttcttgatct 43140
ttatacttca catttcttgg taatccaaat gcttcataaa ttctcttaca catcctgaat 43200
ctcttgcttc attcagcgag ataattcctg gtgagcagga atgcatatgg ttttcagatg 43260
tagaaactgg gatctccaga gacaaacaag ttggccatgg caatgggata caataatctt 43320
ataggatttt acaaacctga tttgaagttt tgtcctaata tggaaaggta tgccttttct 43380
cactagagag attattctag aattttagag ccataccaac atatttcacg gtaacctagt 43440
gatctggatt gcccctctct gagaaagcaa agattctgca agcaattagt ccttcaaatc 43500
aaattcctgg cttttacaaa ctgcttctct tttgaattcc aaaagttaac taattctttt 43560
ctatctattc ttcatttcct cataagcgaa cactcaccag tttccccatt ttccaaccct 43620
atggtttctt tattctacgt ggccacagac aggaacaatt cactagcaca gtggttctca 43680
gaatcacctg gtaagtcttg taaaaacaca aattgctggg ccccacgtac tcccagagtt 43740
tcacattcag ttggtctaaa gtggggccta acagttggaa tttctaacaa attatccggt 43800
gatgctgatg ctacaggtct gagtagcaca tttggaaaat cacttcacta gtacaccccc 43860
gacgtgttgt cctgatggat ccttccaaaa aattgttcag atgccaaggc ttgtgaatcg 43920
cacacatggt actcaatgcc tgggcctcac atcgaaggct tctatattga gaagaaaaga 43980
aatatttgtc atgactcatt gcctttttca agctgttcca gctacaaaat taaaaccaga 44040
aaaggactca attgaaagca aaataaaaac agctcaagta acactgtcca aattttgaga 44100
cttatggcat gatttctgga attaggacaa aaggtatact agtgattcag gtaaatcctt 44160
ggctgaccac actggaagct ggcatctcca aggctcccga caaaccctgg aacccaagcc 44220
aagttgctcc tgcactaccc cagaagccag ctcaggtcct tggcttgtgg cttgcttttt 44280
ctgtcaacag taagttcttc cataaagctc tccaaacaca ttagcaacct ataactcttt 44340
taattaacta atttttctac aacttctcag ctatttttaa cacagaaaaa aatgtcaatt 44400
gtcaagaaat aaatgctgtt gggccatcaa gcaggtaggc tgtaaatctg aaccgagaac 44460
ctagccaatg aatttcctta ggatttcttc cttttgtaat agcaacactc agtggagatt 44520
ttaattccat ttttcatata tttggggaaa aaatacggca ttttatgtca tagcataaaa 44580
ttcttcagca ttccatttta gttattaaat aatgactagt atttgaaaca gcactgttac 44640
attaagtaaa aaaaaattgg caagaaatgt caacttagac atttaataag ataatagttc 44700
tgcttagaaa atgaggtgtt agttctgcct ttaacgtatc aacatattgt aggaaatact 44760
gaccttttat tctgatcacc acgagtccat taaatttcaa aatgtgggac agccaataca 44820
taatatacta ctaagtaata aatctatgtc atccaaaggg agctttgctg tctgagtcca 44880
aaaaaaaaga aaacagaagc ataatggtaa ggttactgaa cactcaggtc ttgaaataag 44940
tcattctgtt atgagcatga gtcaccaaaa ctacttcctt agtgataaaa gttgcctaag 45000
taacatcaat gcctccctat gctaagaagg atatgggcag tgtgttgggt ttcagaccca 45060
ctagtcatag tcatttcctt tacttaattg ctaactgccc ttcctggctg tagtttgggg 45120
cctgtgtttt aggtagtaac ccagcaacta gttattatgc tatattttat caaggagaac 45180
aaaacagtaa attttgtatt tccatggatt tcagagtaaa agtcaatgtt atttcaccaa 45240
ggccctctga cttaagggag ttattgtgaa actgttcaac taggtctcaa tgtggccaca 45300
tcattatgca agactcaaag gcaaaatatt taacagagaa aagaggagca gattgggagc 45360
cctctgagag gaagggaggg gtggtattca tttctgcgtc cttagcacct agcagggagc 45420
ctgctcacct aaagattcaa aacgcaggcc gcgcaggcgc agtggctgat gcctgtaatc 45480
ccagcacttt gggtaggcca aggaggggtc acttgagctc gggagcttgc gactagcctg 45540
acaacacggc aaaaccctgt ctctacaaaa tatattttaa gaaaattatc tgggcatggt 45600
ggcatgtgcc tgtagtccca gctgctcaag aggctgaggt aagaggattg cctgagccca 45660
ggaggtcgag gctacagtga gctgtgatgg agtcactgta ttctagcctg ggtggtattg 45720
caagctcctg tctcagaaaa aaaaaaaaga ctcaaaatac atccaccaaa tggggtttgc 45780
tgcttctttt caaaataatc caatctatga agtaattgcg ccactgctga gttaggattt 45840

catcaggctt tgctgttatt gatgttgttg ttgctgttga gatggagttt tgctcttgtt 45900
gcccaggctg gagtgcagtg gtgtgacctc agctcactgc aacctccacc ttctggtttc 45960
aagtgactct cctgcctcag cctcccgagt aactgggatt acaggtgcct gccaccacgc 46020
cctgctaatt tttgtatttt tagtagagac ggggtttcac catgttggcc aggctggtct 46080
cgaacacctg acctcatgat cctctcacct cggcctccca aagtgctgcg attacaggca 46140
tgagccattc atcagacttg ttaacacata tatgcctttg tttttccctt atatttacca 46200
tgggactgtg gtatttgctg ctgttcccct tgcagacatc tgaagtatat taagcatgct 46260
ctattttaaa atctgacata caaaagcaat gagattagta ttgcatgaga aaatttaaaa 46320
actggatagt aagcacacat gtattatcag tatcgctgtt gggtttggcc ttgagaatct 46380
tggttgccta taattccctg agcctcctct catttccaaa aactccccca taattcttgg 46440
attgcacaat agtactacca tgaaggacta aaatgttaat aaatctccct aaaaatcaaa 46500
ctaatagatg gtaagtatag ggagaagttg tttttaactg atataagtat tgcattccaa 46560
aaatgtggtt ttatttcttt tctactagca aatggggcaa agcattttta ttcacttaat 46620
catatgcaag attcaaatgc cataattatc cattcattct ggtgggttga gtcagaatta 46680
agaaatgaga tctccacaga aaacccctaa aactgtctat ggacttcttg atgattcatt 46740
aagggcacca tattctctga atcaaaatgg gtcctgttgc tgcatcagaa taggaaaaaa 46800
gtagaccaaa gagacaaaca tcatgataac aatgccaaag agatttgggg acatctccaa 46860
tggtagcaca aaattactat ggcatataaa gtactacagc acggtgaggt ttagagttcg 46920
gggactggag ccagaagtcc tgtgttcagg tctgccactt cttggctaga taaacttgag 46980
taagctactt accttctctg tgcctcagtt tcttcatatg tcaaaccggg ctgtcgtgag 47040
gattaaataa gttaacccat gggcagtaca taatgattgc tttcatatgt cataggcact 47100
caatatatga aagcaatcat tacggttatc attagccttt ctatccaggt cagactccag 47160
ttttagcaat catatcaaag tgtgcctggt gatgattgtt tggggttcgc tcaaggacct 47220
agtattggtc aaggactgtg aaagctagcc tagagttagc aaaggaaaaa ccctggcctg 47280
gtgtggtggc tcacgcccgt aattacaaaa ctttgggagg ctgaggtggg tggatcactg 47340
gaggttagga gttcgagacc agcctggcca acatggtgaa acctcatctc tactaaaaat 47400
acaacattag ccgggcgtag tggtgggcac ttgtaatccc agctatttgg gaggctgagg 47460
caggagaatc gcctgaactt gggaggcaga ggttggagct gagatagtgc cactgcactc 47520
cagcctgggc aacagagtga gactctgtct taaaaaaaga aaaagaaaaa ccctgatcaa 47580
acacataaat atctctgcag cgtattctat cacacctgtc tgaagttcta gagatgccat 47640
tcaagtaaat tacaatattg tatcggaaaa acttctcata atttatgtct caaacaacat 47700
gtacttaatc tatattaagc ttgagtacca cagaattttt ttgtccatat gccacatatc 47760
catatgagtc acattagaga acatatttct agtacttagc taattagaaa tacaaaacct 47820
tattcagtgg tttggaaatc gtcatggtag aattacaaag agggctgcca aatcctttat 47880
tctgctagtt aactctgaat ggtctttctt tacttatatc tcattgtaat gttaatgaat 47940
tttcttatct tttcatcctt gcatattgtc attatgtcat tttatatttc cttccatttg 48000
ctacatggct tcatttaggg ttactttgtg ttattttttt tctagaattt gtctttgatt 48060
ttcatttcca acctctgcaa atcacactga tctttgtcac tatacacaaa aaccaaagct 48120
tgagaggttt attttaaact ctgagtttat caaacatatt cacaattaaa tataatattt 48180
aatatactga agaaaatcat ttcactggct gagtgaatgt gatatgccag agttaaagta 48240
tctcccaatt gtccattttt gaaaatatca gtcaattcat taatcagttt tgaaatacat 48300
aagatggagt tccattatat tttgcagatt tagaggaaag agattatgac tatatagatg 48360
gtcaataatc cccttccaat gtcgcaggtt accataaagg caaataaaaa tttacattat 48420
aaatttatct gttctttaaa agaaaattaa taaactgata tgcacatcaa taggagactg 48480
gctaaggaaa atgtggtaca tatacaccat ggaatacaat gcaaccatga aaaagaacat 48540
cagccaggca cggtggctca cgcctgtaat cccagcactt tgggaggcca aggtgggcag 48600
atcacctgag gtcaggagtt cgagaccagc ctggccaata tgatgaaacc ccgtctctac 48660
taaaacacaa aaattagcca ggcatggtgg catgcacctc taatcccagc tacttgggag 48720
gctgaggcag gagaattgct tgaacctggg aggcagaggt tgcagtcagc caagatcgtg 48780
ccactgtact ccaacctggg agacagtgag actctgtctc aaaaaaaaaa aaaaaaaaag 48840
agatcatgtc ctttgcagga acatggatgc agctggagac catcatcctt agcaaactaa 48900
tgcaggaaca gaaaagcaaa tactgcatgt tgtcacttat aagtgggagg taagatgaga 48960
acacatggac acaaagaggg gaacaataga cactggggcc tacttgaggg tggagggtgg 49020
gaggagggag aagataagaa aaaataacta ttggatacta agcttaatac ctgagtgatg 49080
aaatcagcca tacaacaaac ccacatgaca cgaatttacc tacataacaa acctgcacat 49140
gtacccttga acctaaaata aaagttaaaa aagaaaattg tgctattaaa caaaattatt 49200
attaggtccc ttacctaaat atcttgagaa aaaaaattct accactgggg aactacatga 49260
cctcctcaaa gtaggcagag taaattccca agacgccaat tgcacttggc aaattttcta 49320
gctgtggcat aatctttttc tgtctacaaa aaaaaaaaaa aaaaaaaaaa aaaaaggaaa 49380
aacaacatta taggccactg ggcaaatacc tgctagtttt cttatggtag tctaactcag 49440
ccagtaacta aactgggatg atgtcattca gggctctttc aagtgacatg aggtttttat 49500
aactggattg atgggttttt tgtttgtttt tggttgttac tgttttttga tcaagtgaac 49560
taatactgaa aatctggctt taattctttg tccaacatca gctggaactt atttttgcac 49620

tagatttctc ctccatttgt taattaaact cttgtgttcc ccctacatac ttaccacctt 49680
ctctttcctc tgtcatagac tgtgaattag acattgatga agttaaaaaa tcacttatag 49740
tctacaggga attgttagct atgttttccc cctcaaatgt tcaaagatac ctcccagaaa 49800
gtgtctttaa ggtttttctt tcccttctga tgttttaaac actgttttag agaatcgtga 49860
tgttcatgat tttatactag ctatgggaac aagactaaca cactcaaagc agctaaatga 49920
caataaaaga ccatctactt tattccttta agggaagaga ttagaagtgc ttagaaatgc 49980
tgtagaccct gggctatggt ctgatagtgg tcggtgggta ttagattctt gtaggagcgt 50040
gaaccccact gtgaactgtg cataccaggg atccaggttg tgcgctcttt acgagaacat 50100
aacgcctgat gatccgtcat tgtctcccat catccccaga tgggaccata tagttgcagg 50160
aaaaccagct cagggctccc actgatttta cattatggtg agttgtataa ttatttcatt 50220
atatattaca atgtaataat aatagaaatg tttgaatcat cctgaaacca tccccctgcc 50280
tggtccatgg aaaaattgtc ttccatggaa tcggtccctg gtgccaaaaa ggctggagac 50340
cactgctgca taatgtaaaa ccacactatc caattatagc tcaccttcat gagactataa 50400
tttatataga ctagagaagg gaagatattt ccaggttaag ggacctgaat taaccgtctt 50460
tcaactcaaa ttaactttcc aactaattat ttctgtctgg aatgcatcta ttctttcttt 50520
tttcttcttt tttttttttt tttttttttt gagatggagc ctcattctgt cgccaggctg 50580
gagtacaggg gcacaatctc ggctcactgc aacctccgcc tcctgggttc aagtgattct 50640
cctgctccaa cctccctagt agctgggact acaggcgggc accaccaggc ccagctaatt 50700
tttgtatttt tagtagagac agggtttcac gatgttaacc aggatggtct ccatctcttg 50760
aactcatgat ccacccgcct gggtctccca aagtgctggg attacaggcg tgagccactg 50820
cgcccagccg catctattct tttaattatc ctgttcacaa ggctcagagc ccactcgatt 50880
tttcttatcc cctagccctt aaacatcaca attccttaaa tcatctccgc gtcaggctcc 50940
ctttaccatc cacttgatct acctcaagca gacctcaaat cagcctcatt ctccacccct 51000
aaaatacagc tacaagcatg tctttctcct agttgaaatt cacaatgcat tccctctgcc 51060
caccacatta attatcaact ccttttcctg gcatttactc atccaacgca tggccccacg 51120
ttaactttca gttccctttc tcccctacaa atactccata atccagcaac cctgggatta 51180
ttcctgttcc ctaaatgtcc tcagcactct gccacccacc cagtttttgc ttatactgtt 51240
tggtcagcac tagagcaacc tttctttgcc atgataagct gtagaaattg tacttattct 51300
tcaaatacta tcttttctaa tgacattttc tgttctcccc caagtagata ggatcatttt 51360
ttttctcaac aaaatttaac ttatttttag gtatagtatt atagtttttt tttttaattt 51420
agatttaggg ggtacatgtg caagtttgtt acataggtat gttgtgtgat actgaggttt 51480
gggcttctaa tgatatcgtt gcccaaatag tgaccatagt actcgatagg tggtctttca 51540
atccttgcct cctccctccc ttcccacttt gggaatccct agtgtttact gtttccatct 51600
ttgtgtctgt gtatactcaa tgtttagctc ccacttagaa gtgagaacat gcagtatttc 51660
gttttctgtt tctgcattaa tttgcttagg tttatggttt ctacttgtat ccgtgttgct 51720
acaaaggaca tgatttcatt ctttttaatg cttatgtaat attctacagt gtatatgtac 51780
catgttttct ttatccaatc cactgttaat gggcacctga gttgattcca tgtctttgtt 51840
attgtgaata gcgctgcaat gattatttct ccacctatgc tctttagcat tttatcctat 51900
ctgtgctaca gcactcactt accacattct gctatgtatt ccaactcttt acctgctttc 51960
taatccttat acaaagctat gagatcccta gaggcacaaa aagcacctta cttactgaaa 52020
tatactaaga aacattgcag gcagtgcaaa aaaatatgaa tacatctatc tgttaaatgg 52080
aatatgagga aagacctgag ggtgggggaa atgtactata ttcatggaag aagaaggagc 52140
ttagggttct actgaaattg ctgcttgtgg ggaacagtgg aaaatgagga tagatatata 52200
tacttgacat aacttccaaa ggccactagt taactatgat cctcaacctc ctctgcagga 52260
aaatttctgt ttccctcata gtctttatca ccagttgcaa tcattttgtt tgttccttta 52320
cgagtattgt ctctctgtat catatacata tacgcagact caacactatg ttccaagagg 52380
ctttgggcca tacctgcttt ggtcccttcc ccagtgtcta ccagactgcc cggcacatgg 52440
caaatactca ataagtatgt ttggagtaaa agaataaaag ttgtgtatca cgataccttg 52500
tgaagattaa atgcagtcat gtctaaagtg actagcacaa tgcttaaaca taataagcac 52560
cagtaaatat tagccccaca cctatatttc ttccttctat gaactactca tgtattaatg 52620
tatatattcg acagatgtgc ctggcttttc tctagccact ggaaatacaa tagtcaacaa 52680
aagagagaaa aattcctacc tgcccttagg gagattacat tccagtgagg aaatgagcaa 52740
ttcctgccaa gaagtttaaa gaaggaagtt tttggccaaa tgtggaggct cacacattta 52800
atcccaacat tttgggaggc cagtgctgga ggattgcttg agcccaggag ttcaagacca 52860
gcttgggcaa catagcaaga ccctggctct acaaaaaata aaaagttagc tgggcatgga 52920
tggcactccc agcttctagg gaggctgagg cagtaggaat acttgagctc aagagttaag 52980
aggctgcagt gagctatgac tgcgccaccg cactccagcc taggcaacag agcaagcctt 53040
tgtctctctc tctctctctc tctctctcta tatatatata tataagtttt aaagaaaaag 53100
gggtgatggc ttgaagatag atgcattggt ggcagaaaag aaagcatgga ggggccgggc 53160
gcagcggctc acacctgtaa tcccagcact tcgggaggcc aaggcgggtg gatcacgagg 53220
tcaggagatt gagaccatcc tggctaacac ggtgaaaccc cgtctctact aaaaaaatac 53280
aaaaacttag ccgggcttgg tggcgggcgc ctttggtccc agctactggg aaggctgagg 53340
caggagaatg gcgtgaacct gggaagcgga gcttgcagtg agccgagatc gcgccactgc 53400

actgcagccc cgggggacag agcgagaccc cgtctcaaaa aaaaaaaaaa aaagaaaaga 53460
aagaaagcaa gaaagcatgg aggaacggaa gccaattgca gtattgcagg aaggaggtat 53520
gaaatggctt ggatctgagt gacagcaacg taacagagga gtggatccag gaagagcggg 53580
gcatgatacc cattggatct gagatggaag agttagttag gaagtggcaa tgatgaaagt 53640
ttccaatctt gttgttttgc tgtattgccc tgaatagaca gggaagctgg caggggaagc 53700
gatggtggtt catgggcttt agggcgtgag acgagctaaa cccagtcttg aatgtgattc 53760
ctcctaagag gcaaaatgga atctaaatga aaatttttaa ctcactaatg aaagtaatgt 53820
tttagaatgc aggtgaaaac aaatctccct tcttacatag tatgatcact gttacgtcat 53880
ttgattttgt tttttacatc attttacatc gtaaacatgg tgttatagct gtctgccaaa 53940
ttcctctaaa aatatgcccc ttcgcagcaa gatctgtgtt aatcatccca tctcttcagt 54000
tcctggaaca cagtaggtcc ctaaatgata agtccagcac ttaatatttg tgaattgaac 54060
tggttgatag taagcaaaat tttgttatca aggcaaacag aaaagagaag tgcatttaga 54120
gtcagaaagg cctcgattca aattcttgtc cccttcttcc cacccctgct agtcatacaa 54180
ccacaagaaa attgctagcc tgcatcttag gctcctcatt agtgaaatgg atgatcaagt 54240
ctatcttgca ggagggtgtg agtgcatcaa gttaaattaa atatatacac agtgtctgtc 54300
attcagttgg catcgacatt acctattata ctatttatta ttattacctg tatttccatg 54360
gattttaggt tttgctatgg ttttcatgct ctcagtgtac cctccttctt ctttctaaca 54420
ttccatccaa acagtcctgc tagatgctgt tctcttggca tggctagcca gctttcccaa 54480
agcctccata ggccactaag cttctgtcca cacggctggc tggtccgtca gtttaagtca 54540
cccaattcaa ctgacatcat gacagcttgc aaattccaaa atcactgttg gtaatttaga 54600
tgtggtagtg tttatgcctg gcctatgaat tataccacca cgtctttatt tatatgcaac 54660
ttgcatcatg ctgtgttgaa agcagcttcc cagtccagca cttgaaaata cttctcagat 54720
aggttttcat agcaattaaa aaataaacac aaaacccggg aaagctatgt ctgtgtaccc 54780
aaaagatacc taagaacaca gtagcataga gagcaggctg agttgtctca ctcaaggatg 54840
tgagttcaga ttcattgatc tccctgactg aaaactatca cccattgatt gaggcactgt 54900
atatcttatc tcagaatgaa atgaattctt aagtctcatg aattctacat gaatgaatga 54960
aagtgtttct gcaactttca cttctcttgc agcccaccaa aaacaatggc attttctaat 55020
aggaaaaaca cattgaacgc tgtatatgtg cgtatgtata tgtgtgtgta gtacatacat 55080
atactttatt ttgagacagg ttctcactct gttgcacagg gtggagtgca gtggtgtgca 55140
cagcctactg cagcctcaat ttcccaggct caagcaatcc ccctgcctta gtctcccaag 55200
tagctgggac cacaggtgtg caccatcacg cctggctttt tttggtttca tatatatatg 55260
ttgcctaagc tggtctcaaa ctcctgatct caagcaatcc tcctgctttg gcctcccaaa 55320
gtgctgggac tacagaacac ctacactttt atacattcta tatgcatcaa acaaacatat 55380
gtctgtgaca agagagcagc ctatcacagc ccagagactt cagaatgggg acaggttgga 55440
aggaggagct acctatccct gagatgatga agaggaccag tgcccattcc aggagacatc 55500
accgcagccc tgaggaatcg gctatgggca ccagcagggc acagtgccac acctcgccaa 55560
tgccttgtcc tccttttcca tagtgagtca gtcagcaagc gtcgcattgc cctgggatcg 55620
gcactgcacg tagaagtgag ttccacactc tcttcctccc atagggagat cacttttctc 55680
attctaaggg ttccaggcac actcacaatg gtggcatttg ctgagcagtg gcttgaataa 55740
agggctctca gaaagcaaga tgtaactcag agcataggct tgtcctccag ggcacccagc 55800
cccaggaatg ctcttgggga atgacctgca gcctcccagt gaaagagaga ataaaagaaa 55860
gccccagcag gcgagctggg cagtagagag tcctgtaatt ccaccttggc aagcaccatt 55920
tgcaagaacg aactgggata aggtaaacaa aatattgcct aaaagaggct tgtccaaaga 55980
agtcagaata cgctcttcat ttacctctaa attatgtttc ttaaatacca agtaacctgc 56040
agtaccccct gccgccacca aaaaaaaaga gagaaatact tttttttctc ctatccactg 56100
cagaatcaat tttacagtgg ttctgcagtt aatcctttca gtacaccata aatctaaata 56160
ctcaaaaaaa cctgtgcctt ttcaattgct actaaatcac gagaagactg atttacatag 56220
tctcctttta tctcccttgg cgggtaagta ctcagctctg ctcgttacta atattgaaac 56280
aacagccctt gaattgagtg atttccctag aaaggttaag gtgaccgaat ctgaacactc 56340
cctccatgtc ttggacacga agttttttgc tgcgtagaca gttttatccc cctcacccca 56400
aggtcaattg cacgaattct tttggaaaac aggacctatg gcatttccca gacaaatcac 56460
cgtgaaccct gtactgtgca ttgctgtcct aaaattaaca cataaatcta ttgccgccaa 56520
agattctgtc atttgtgtta cataattgcc tttcatttga actcattaat caaattgggg 56580
tttttaagca acacctaatt aattctttaa ctggctcata ttataccttt aatgacttcc 56640
accagggtaa aaaccactga tcactgagtt ctattttgaa actacggacg tcgagtttcc 56700
tctttcaccc agaattttca gatcttgttt aaaaagttgg gtgtggtttc atggggggag 56760
ggggaagagc gagaggagac cagagggacg ggggcgggga ctctgcaaga aaaaccttcc 56820
cggtgcaatc gtgatctggg aggcccacgt atggcgcctc tccaaaggct gcagaagttt 56880
cttgctaaca aaaagtccgc acattcgagc aaagacaggc tttagcgagt tattaaaaac 56940
ttaggggcgc tcttgtcccc cacagggccc gaccgcacac agcaaggcga tggcccagct 57000
gtaagttggt agcactgaga actagcagcg cgcgcggagc ccgctgagac ttgaatcaat 57060
ctggtctaac ggtttcccct aaaccgctag gagccctcaa tcggcgggac agcagggcgc 57120
ggtgagtcac cgccggtgac taagcgaccc cacccctctc cctcgggctt tcctctgcca 57180

ccgccgtctc gcaactcccg ccgtccgaag ctggactgag cccgttaggt ccctcgacag 57240
aacctcccct ccccccaaca tctctccgcc aaggcaagtc gatggacaga ggcgcgggcc 57300
ggagcagccc ccctttccaa gcgggcggcg cgcgaggctg cggcgaggcc tgagccctgc 57360
gttcctgcgc tgtgcgcgcc cccaccccgc gttccaatct caggcgctct ttgtttcttt 57420
ctccgcgact tcagatctga gggattcctt actctttcct cttcccgctc ctttgcccgc 57480
gggtctcccc gcctgaccgc agccccgaga ccgccgcgca cctcctccca cgcccctttg 57540
gcgtggtgcc accggacccc tctggttcag tcccaggcgg acccccccct caccgcgcga 57600
ccccgccttt ttcagcaccc cagggtgagc ccagctcaga ctatcatccg gaaagccccc 57660
aaaagtccca gcccagcgct gaagtaacgg gaccatgccc agtcccaggc cccggagcag 57720
gaaggctcga gggcgccccc accccacccg cccaccctcc ccgcttctcg ctaggtccct 57780
attggctggc gcgctccgcg gctgggatgg cagtgggagg ggaccctctt tcctaacggg 57840
gttataaaaa cagcgccctc ggcggggtcc agtcctctgc cactctcgct ccgaggtccc 57900
cgcgccagag acgcagccgc gctcccacca cccacaccca ccgcgccctc gttcgcctct 57960
tctccgggag ccagtccgcg ccaccgccgc cgcccaggcc atcgccaccc tccgcagcca 58020
tgtccaccag gtccgtgtcc tcgtcctcct accgcaggat gttcggcggc ccgggcaccg 58080
cgagccggcc gagctccagc cggagctacg tgactacgtc cacccgcacc tacagcctgg 58140
gcagcgcgct gcgccccagc accagccgca gcctctacgc ctcgtccccg ggcggcgtgt 58200
atgccacgcg ctcctctgcc gtgcgcctgc ggagcagcgt gcccggggtg cggctcctgc 58260
aggactcggt ggacttctcg ctggccgacg ccatcaacac cgagttcaag aacacccgca 58320
ccaacgagaa ggtggagctg caggagctga atgaccgctt cgccaactac atcgacaagg 58380
tgcgcttcct ggagcagcag aataagatcc tgctggccga gctcgagcag ctcaagggcc 58440
aaggcaagtc gcgcctgggg gacctctacg aggaggagat gcgggagctg cgccggcagg 58500
tggaccagct aaccaacgac aaagcccgcg tcgaggtgga gcgcgacaac ctggccgagg 58560
acatcatgcg cctccgggag aagtaaggct gcgcccatgc aagtagctgg gcctcgggag 58620
ggggctggag ggagagggga acgccccccc ggcccccgcg agagctgcca cgcccttggg 58680
gatgtggccg gggggaggcc tgccagggag acagcggaga gcggggctgt ggctgtggtg 58740
gcgcagcccc gcccagaacc cagaccttgc agttcgcatt tcctcctctg tccccacaca 58800
ttgcccaagg acgctccgtt tcaagttaca gatttcttaa aactaccact ttgtgtgcag 58860
ttgaaggccc ttgggcacaa tgagagccag tcctccaaac tttcagaaag tttcctgccc 58920
cttctggcag gctgccaatc accgggcggg agaaggaagg aggggaaggc ggtggaggga 58980
gcgagacaaa gggatggtcc ctcgggggcg gggatggcgg ggctgtcctg taggtctgtg 59040
cggccaccgt gattgcccct ctgcgcggtg cccgaagtcc cgctgaaacc tgccgagggc 59100
agcaggtctg aaagctgcag gcgctagttg cgcggaggtg gcgcagctgc tctggaggcg 59160
cagagcgaat acgtggtgtt tgggtgtggc cgccccgccc ctggcggttt cctcgttccc 59220
ctttggttaa tgcgcaactg tttcagattg caggaggaga tgcttcagag agaggaagcc 59280
gaaaacaccc tgcaatcttt cagacaggtt tgtagactct cttcccactc gcagccgcct 59340
gaccccaccc aacacaaccc acgagcaatt ctaaaagttg cttaactcac gtctaaaaag 59400
tgcaaaactt cagggctgcg cgtaaagccc tctagtggcg ggaagaccac aggttggagc 59460
ttctcatgat tagaaaaata ttaataaaac cccttgagcg attttttttt tttttttgag 59520
acggagtctt actctgtcgc ccaggctgga gtgcagtggc gagatcttgg ctcactgcat 59580
cctccgcctc ccgggttcaa gcgatccttg aatgatttct aagcagttcc ttgggacata 59640
aagaaaaatc ttttaacttt ttactttgtt tcccaaatgt tgcacagttt tgcaacacat 59700
tgaccttctg gtttcgaacg gttacaattt tagattgtgg tttgccaaag tcaagttgct 59760
taatttttac aaggccacaa aaagcgcaat tatgccctgc agtttaaaat ggaaaacgtg 59820
ttggaagata agaaaactta gtttccaact ggaatggagc cagcaagttt cttttcttct 59880
ttgcaaattc tattgtgtca ttaaagttcg atggaagtat cactatgcac aactattttg 59940
tgatctaata agggtgaaaa ggagccatct gtccccttgg ctaaggggta ttaatggttt 60000
ctatgggctt cactatggaa tgtagataca gacattctgg caaatgtggt ggctctggac 60060
agaaataata ggagtctttg tattcccagg gaagctttgc aacaggctac attcttactg 60120
aatatgtaat gatgtaagca cggttctaat tggacacaag tatttgctaa catccgttat 60180
ctaatatctg gcccagactt gagaagtagg taatgtaaaa agtttttaaa gctacaagca 60240
tacctcacat tttaaaagtc ctttcttgat tgggttcttg tgttctttag cactcttgcc 60300
ataaaaaata ataacagtaa taaacccaag gctgaaaaac tgaattttaa ctaaagggtt 60360
tttgtgcgtg tttttttttt ttttcaccaa aattagatgg acttacagaa tttttaactt 60420
aaaattggaa tccaaaagcc agaagatccc cattatagtt tatagttgta ttccctggaa 60480
tatttactgg gattaactgc aaagcactct cagatgaata gtgtagtata acattttgaa 60540
actgaaatac atttaccaaa ttaatttaac cacagcaatg tgtgtggttc attttagtcc 60600
ttgagcattt ttgattatca tacctgtcat gttttctgca gtgtagtgag ttaacataaa 60660
acaacatcaa tacaaaagat cctctgtttc gagattaagc aaaattcctc attctcttca 60720
atgtgataga ataccacatt gatctttctt tggaggttag taaaatatct tttatgtatt 60780
tttcagggct taacaagtaa aaatcaatgt tttcatcaag tctgatcttt ttgtcaccca 60840
ctcttcattc atttttccac taaggtgata gaaaagtctc aacagtttaa gaccgtaagg 60900
ctatgaactc caaatataat tgctgacaag ataagcaatc ctcacgcatc cttttgagag 60960

gaaataaaat cttagttgca agattacata ttctgatttg gaatgctgag ctttttaaat 61020
ggaaatatag aaggacggct gaatcagcaa aaatccttta tgtagtttca ttctttgcaa 61080
gcttgaccag tcattctgaa acaggctaac tgaactgata cagtggcaag tgaaaaagac 61140
atgcctttac aggatgagtc aaaggagttt tagaagaaaa atccaccaga gaaagccaag 61200
caaatacagt tcagagttac atttcttttc cattttttcc tgaactgaat ctttggcatg 61260
catatcctga attgggttat tgaatataaa tctagccttg tacaatggat gccagatgac 61320
tacatatttg ctttggagcc taaggataag tttcaaaaga tttgagtgga gaagaaaagc 61380
taaaactctt gaagcacaag tttctgttct ccatgtactc aagtgtacat gaagttgtga 61440
aaatttgtcc acctctatca tcatgttatt ccatgaaatt acaaaacaaa tcttaaaaat 61500
gttgtggcat agattttcta gatttaaaaa gtaattaaat taaatgaatt actttatttt 61560
ttgagacaga gtgtcactct gttgcccagg ctggagtgca gtggcactat gttggctcac 61620
tgcaacctct gcctcctggg ttgaagaaat tctcctgcct caacctccca agtagctggg 61680
actacaggca tgtgccacca cacccagcta atttttgtat ttttggtaga gacggggttt 61740
cgccatgttg gctaggctgg tctcgaactc ctgacctcaa gtgatccacc cgtctcagcc 61800
tcccaaagtg ctgggattac aggcataagc caccatgacc agccttaaaa agtaatttta 61860
aaatatcact ggtaaaatgt ggattcagtc atgattgagt gcagtttacc atgtgtgtgg 61920
acatttattt attttaaaat tgtctgatca ccaccttgag taaaacacaa gcagtcacaa 61980
ttaaaatata ttagtgagca ggagaaagca cagcatatta tagcactgaa tgatttataa 62040
acctattcca gggtcataaa atgtgtcaac ggcttttcta tagtaaggag actaggttca 62100
gatggttaat ctaagacaaa taaatgagat aagccataca cttttacatc ctccatgtcc 62160
tgtcttttct ctgttcaaaa taggatgttg acaatgcgtc tctggcacgt cttgaccttg 62220
aacgcaaagt ggaatctttg caagaagaga ttgccttttt gaagaaactc cacgaagagg 62280
ttagtggagt gactttcggg gaatgaatga gggtaaggca gcccccacgg ttggcagagc 62340
tgaccgtctg tctgttcttt ttgcaggaaa tccaggagct gcaggctcag attcaggaac 62400
agcatgtcca aatcgatgtg gatgtttcca agcctgacct cacggctgcc ctgcgtgacg 62460
tacgtcagca atatgaaagt gtggctgcca agaacctgca ggaggcagaa gaatggtaca 62520
aatccaaggt aggaaacaaa tcagtgcggc ttcaaccaaa gaaaagcatt gtgttctcaa 62580
aaccccatac ctgtgtgtga ttcctaaata tcctctagct ccaatgcaaa gctggctttg 62640
acttcttgct catattgtgt ttgccaccac agcctcccca ccactcacat cacctccttt 62700
atttatttat ttattttctt atttatttat gagacagagt cttgctctac tgcccaggct 62760
ggagtgcagt ggcaacatct tggcacactg caacctccgc ctcccaggtt caagtgattc 62820
tcctgcctca gccccctaag agctggaacc acaggcaagc accaccatgc ccggctaatt 62880
tttgtatttt tagtagagat ggggtttcac catgttgacc aggcttctct caaactcctg 62940
acctcaggtg atccaccctc ctcagcctcc caaaatgttg ggattacagg catgcgccac 63000
cacgcctggc cacatcacct ccttcagaat agcagactct cttcccccta accttgcctc 63060
caagtaaacc ccaatgccat acctttgacc tccactgtgt tgaaatgagc actgtagagt 63120
gaactctgaa aatactaatg tcagtactcc actgctcttt ccctggcttt caaaacagaa 63180
atttaaacct atactggaag acattcagtg agaaatatga tttttttttt ctaagagagt 63240
caaaagactt gaatgtgagc aatctacatt tctgttttct tcccaacagt ttgctgacct 63300
ctctgaggct gccaaccgga acaatgacgc cctgcgccag gcaaagcagg agtccactga 63360
gtaccggaga caggtgcagt ccctcacctg tgaagtggat gcccttaaag gaaccgtgag 63420
taccaaccct gcagtaaaag agggaaaata atgacccatt ctgctgacta ggctcatgat 63480
gatacctgaa caaaatgttg agtgagtaaa aatgtatatc ataatgcaaa gaaaatgagt 63540
tatcaagaca gactcaaaag ggacttcatg gaactcttga aggttttagc ttgcctatat 63600
cattgcttct aatatgaagg acttggtact cgcattctcc acctaaaatt agagtggtcg 63660
ccatttgccg ctaatggaaa ttattgcaga aggtctgtaa atggttctgg gaacagctgg 63720
gtttttctga gaaataacac cagacatctt tctcaccccc tgcagaatga gtccctggaa 63780
cgccagatgc gtgaaatgga agagaacttt gccgttgaag ctgctaacta ccaagacact 63840
attggccgcc tgcaggatga gattcagaat atgaaggagg aaatggctcg tcaccttcgt 63900
gaataccaag acctgctcaa tgttaagatg gcccttgaca ttgagattgc cacctacagg 63960
aagctgctgg aaggcgagga gagcaggtag ggaactcaga cttggatgcg tgaactaatg 64020
gtgaccattt gttaggccct gtgccactgg gctctaagca gtgtcacatt taatctttag 64080
aaagtttctt tgaggtaact gctttccact ttttgtagag gaggaatttg aattgagaga 64140
gagtaagtga cttgctgaaa aagggttaat caacagcaga gctgggattt gaacccataa 64200
ctctgtcaaa gcctccactc ctaactcctg ttcatgctcc tgtggagaaa atgcttgtag 64260
taacatattt taaatgtact aacaagacca gtcatgggaa aatgtttctg agacaaatct 64320
ctagtttatg atttaaaaca gtacgttttc ttacgtgacg aaaacaaaaa gtgtgttaat 64380
ttgttcccag tggttgaagt tatttgccaa caattttact gtttctcttc atctgtttat 64440
aggatttctc tgcctcttcc aaacttttcc tccctgaacc tgaggggtaa gcattttatt 64500
tccctttagg aaaaacgtca gctgcttgta accactgtgt ttatgtcaaa gcattcattt 64560
tttttaggat atctgaaaaa atgccatata agagaaaact ctataaaaca tctataattt 64620
tcgaacccaa gtacactctt gcattctatg ctttaagtta aatgcaaact cctttttcct 64680
tcttcctgct gcaagtacta tctcatcctg atgctcaaga gtgtcagggc ctgggtttcc 64740

aaacagagac taccctaaaa ttatttggcg agtagtactt tacacaattg cctctccccc 64800
acaaatcata attgtttcag taaaatggtt acttggtttt tccaagaaaa aactcgtttt 64860
tactcatttt tggcctgttt gtttatttag aaactaatct ggattcactc cctctggttg 64920
atacccactc aaaaaggaca cttctgatta agacggttga aactagagat ggacaggttg 64980
gtatctttta aggaaaaaat agggtaatct cagacaggag ttgatatatt ttaaaatcag 65040
tgaatctgaa tctcagatac agctggctaa tttgagaggt tcaggtttca ttcatgccta 65100
ctaaaaaaag aataggcttc ttcttccagc agtacacaca gccaactaat tatttggctc 65160
ctggatgtga agttgagata gcagtcttcc tgtgctccag aattagtgat ttgctttggt 65220
gcttaatttg aagtgggagt aagcttcctt aaaccacttc ctaaagcagc tacatgaaac 65280
agcttcacta gactacctca atatgaggaa tgttttgatc ctggacatat ggtgtcttcc 65340
tacctccata ctttatagat tcctaaaccc atctatataa tacaagcatg tgccatacga 65400
tcatttagtt tcttattacc tccctatgcc aggaaagaaa tagttgcaat ttattgtagt 65460
catcatgaaa tcttcccttg cacataaatt taaaatgtac ctgctgcaca ttttaatatg 65520
tcttaattgc ttttaaactt ggctgtattg tgtacaacta ttataccatc ttttataaac 65580
acagtttttt aagaaatttc tttttgtaag ttacaacatt ccactggatc cttatattgc 65640
ctgtagtgga agagggtctt gtgtgtctgc cccttctagt tttcactcat gcagaagcaa 65700
cataaccttc tgatttgcac aataaattac atatatttag caggattttt atttgccgtg 65760
atatatagga taatttagtc tttggcatgt ggcattatat ttattttggt tttttttttt 65820
aaacaggtta tcaacgaaac ttctcagcat cacgatgacc ttgaataaaa attgcacaca 65880
ctcagtgcag caatatatta ccagcaagaa taaaaaagaa atccatatct taaagaaaca 65940
gctttcaagt gcctttctgc agtttttcag gagcgcaaga tagatttgga ataggaataa 66000
gctctagttc ttaacaaccg acactcctac aagatttaga aaaaagttta caacataatc 66060
tagtttacag aaaaatcttg tgctagaata ctttttaaaa ggtattttga ataccattaa 66120
aactgctttt ttttttccag caagtatcca accaacttgg ttctgcttca ataaatcttt 66180
ggaaaaactc ttttgttgtg ttatttattg gataatatct aaacaattct ctacttggtc 66240
ctattagtta atttgtcatt acaatcatgt aagttgataa attcaggtta tttatgcttg 66300
agatgtagtt cttaattttg tcatttttga tagacctcac ttctttttat tattacttaa 66360
aaacatttac aaataggtgg tgtcgaataa aataacttgt accaaaatga agataggtct 66420
ctctaaaatg agctcaggtc tgtgatttta atcataacaa acagacttcc taaaattaaa 66480
aaataaaaac tttttttagt acatatagca tatgagtaac acaaattcca ctttgggagt 66540
taaagcatag gaagttgcca agatataggg gcttatcttc cgctagcaag atgcagagaa 66600
atggaaaaag ttcaccaagt ttttctttta tttaagacag gatctcactt tgtcactcaa 66660
gctggagtaa agtgactcaa tcatagttca ctgcagccaa caactcctag gctcaggcaa 66720
tcctcccacc tcagcctcct cagtagctag gagtactcaa aacaaataaa cgaatgtcta 66780
actttagcct aaaatgtgag ttgtcttccc aaaactagcc acacacctgg ctaatttttt 66840
taattttttg tagagatggg gtctggaagt gttgtccagc ctagtctgga actcttgtgc 66900
tcaagtgatc ctccctcggc ctcctaaaaa gctgggatta caggcatgag ccaccacact 66960
cagcctagtt caccaagatt taatatatga aaccgttctg ctttattgtg tgtgtgtgtg 67020
atagctatat ctatatccat ctatctatag atacagatat ttgtaaagac ctttgtgttt 67080
ggagaatggc attaggcaca aaataaaacc tcctaccttt tcttccaggg agatgagatt 67140
attacatgtg aatcgcatgg tgctaactgt aggtgtaata aggtttcaaa gtctggagca 67200
ataaaaatga agtggggcag tcatggaaag ctccctagaa agcactcagt gtgagcatag 67260
aacttacata aatagacgag agaaggtatt gccaagaggc aatagtgtag taatggtcaa 67320
taatgtgtgt tctggagtga aaacttcgag tacattctcg gacatctcta agtttcaaat 67380
tatggaaata ttattatctt ctctcctccg agaatgatgt ggtagacatt gtcataattt 67440
ttctgtgttt gcatctgcct ctctctggag ttaactagtg agatggttct cacaaagccc 67500
tgcccatttg caggaatgtt cactgtgatt ttcatcattt tggttaccgt gggtactgca 67560
tgaataaaga aaggattggg gctggatgca gtggctcatg cctgtaatat cagcactttg 67620
ggaggctgag gcgggtggat cacttgaagt caggagttcc ggactagcct ggccaacacg 67680
gcgaaatcct gtctccacaa aaaatacaaa aattagccag ccgtggtggt ggatgcctat 67740
aaccccagct actctggagg cagaggcagt agaattgctt cagcttggaa ggtagaggtt 67800
tcagtgagcc aagactgtac cactaaactc cagcctgagt gacagagcga gatcctgtct 67860
tagaaataaa gaaaaaaagg attggctgct gaagtaactc ctagccattc taaaggtaaa 67920
ttctaaatgt aaaagtaaac tttacattct agtttacttg ggacattctt ggtttatgac 67980
tgttgtgtaa gcataattat taataataac agtttggaca atagattgta tgtggtcagg 68040
tcatgctacc cttagaataa tcttacagaa aatgtgtctg tcccctcagc ccccatattc 68100
aaacaactga catggacaat ggcataatta attttattag tgtaaacatc caggtctttg 68160
ggcccttatg aaaccttttc tatttgattt ttcgttttgc tttctgttct ttagccattc 68220
tcaccactgt tccccgcagc ctcctaaccc taggatccct gtctcctctt ccacttgtat 68280
cccacctttg gcttataagg gtcatccagg gaagacccca gccccatatt ggctttcttc 68340
acacaccaga ctcattatta tttttttaaa aattgaaaaa caagccacag ttttctttca 68400
ggggaaaaga accaggacag caagttctgc tcatacctat tgagattagt agactcccac 68460
tcttaaaaat cctttacaga tggaggccag gcatggtgac caaggtgggt ggatcccctg 68520

aggtcaggag tccaagacca gcctggccaa taaggcgaaa ccccgtctct actaaagaca 68580
caaaaactag ccaggggtgg tggtgcacac ctgtaatctc aactactcgg gaggctgagg 68640
cacgagaatt gcttgaaccc aggagatgga ggttgcagtg agccgagatt gtgccactgc 68700
actccagcct gggcaacaga gtgagactct gtctcagaaa aagaaaaaat atcctttaca 68760
gatggaaaca acagacactg ggcacttcaa aaaggaggag gttgggagga gggcaagggt 68820
tgaaaaacca cctgttgagt acttgggtga tgggttcaat agaagcccaa gctctagcat 68880
tacgcaatat atctatgtaa caaacctgca catatacccc ctgaatctca attaaaaaaa 68940
aaaatatctt acagctcttt ctaaagtcac atcccagaaa tggaaaagag gagtgggcct 69000
cccttatctg tgactgtcag aaattagacc ataatgaaaa gcttcacagg tgccccatct 69060
ccgaagaatt caaatttatt tcagtcttct tctattttat ctttaattat actgttagtg 69120
tttttcctta tcttccactt tgaaaaattt ataaaaagaa attatgttct caccacctat 69180
ggtgaaagag agattaatgc ttggttcctt catgtataat attaatccaa gattaaagga 69240
gacggaaaaa gtagggggat ggggagaaag aaatggtggc ttaaagtgtt taagtctgaa 69300
aaaagttact aaggggatgg tgaaatccat ggaactcatg tgactgtcaa gtcctactaa 69360
gttttctcac agtgacagat ggaaaaggct gctggtgaat gaaacaaatg tctactttta 69420
gcctaaaatg tagtgagttg tcttcccaca actagccgct aacaaaggcg ctaaaaaaat 69480
aaaatctctt cccaggctat gagactaagg gagtgagtgt gtgtagtgat gggggaggga 69540
gacagacttt cactccaact atcgtctgaa tgtttgtgtc cccccaactt tgtatgttga 69600
aacctaacct gcaacgtcat ggtattcaga tatgtctctt gtgaagtgat taggtcagga 69660
ggctgaagcc cttatgaatg agattaatgc ccttataaaa gaaactccag gctgggcgtg 69720
gtggctcaca cttgtaatct cagcaggttt ggaggctaag gcaggtggat tgtttgagtc 69780
caggaattcc agaccagcct gagcaacatc gtgaaacgct atctctacaa aaaacaaaaa 69840
acaaacaaac catgtgtggt ggtgcacacc tgtagtccca gttactcagg aggctgaggt 69900
aggagaatca cttaagcccc ggaggcagaa gttgcatgag ccaagatcat gccactgcac 69960
tctagcctgg gtgacagagt gagaccctac ctcaaaaaaa taaaataaaa taaaataaaa 70020
taaaacaaaa aagcccataa tcccaacact ttgggatgct gaggcaggag gattgcttga 70080
gaccagagtt caagaccagc ttgggcaaca ataagaggcc ccatatctac aaaaaataaa 70140
aatataaaaa aaacaggcat ggtggtacgt gcgtatagtc ccagctactt tggaggctga 70200
ggtgggagag gatcacagga acccaggaac tcaaatctgc agtgagctaa gaaggtgcca 70260
ctgtactcca gcctgggtga cagaggagac cccatctcaa aaaagaccct taaaaaccca 70320
cttgtccctt ccatcatgcg aggacacagc aagaaggcac cttctataaa ccaggaagtg 70380
gccctcacca gacacggaat ctgctggtgc tttgatcatg aacttctcag cctccagaac 70440
tgtgagaaat gtttctgttg tttaaaagcc acccagtgtg catgattttg ttacagcacc 70500
cgaaacagcc taagacagct ccatcctaag gattactcat gagctttgtg aaggaagcaa 70560
ggataaagca gacactatgc ccacacgaag tctccctctc caacctgcca gaccagctgc 70620
atgaagaagg gtctgtacta ctcagctagt tttactgtaa cagaatacca cagactgggt 70680
ggtttaaaca acagaatttt attgtttcac agtaacagag gctggaagtc caagatagag 70740
gtgctggcag ggttggttta ttctgagcct ctcccattgg cttgcagatg acacgttctc 70800
tctatgacct cacatggtca ccccttagtc tgtttggtct gtgtcctagt gtcttcttct 70860
aaagacacta gtcccattgg attagggcct acccatatgg ctccatttta ccttaatcac 70920
ctatttaaag gccttatctc caaatactgt tacaattttt tttttttttt ttgagatgga 70980
gtctcactct gtcacccagg ctggggtgta gtggcacaat attggctcac tgcaacctct 71040
gcctcctggg ttcaagcgat tctcctacct caacctccca agtagctagg attacagcta 71100
cacaccacca tgcccagcta attcttgtat ttgaatagag acagggtttt gctatgtttg 71160
ccaggctggt ctcgaactcc tgacctcaag tgatctgccc acctgggcct cccaaagtgc 71220
tgggattaca agtgtgagcc accatgccca gccctccagt acattttgaa gtactggagg 71280
ttagggcttc aacatatgaa ttttggggaa acacacttca gctcatacta ggatcaccac 71340
taaacattgg gaaacactct ccttgtctgt actcacaata ggtctgtgat acagattttc 71400
aaaagctgta aatttgcaaa atcattgata gggaatgcat ggcttacaga tttgttgatc 71460
atttgtgact gcccagcttc tccatttttt tttttttttt tttttggtcc cttgtctaga 71520
gtcagggcag tttgaggtca gttccatgga gtctttctac aaagatcttt catgtcaagc 71580
aaatacttct tttagggcac tgtaggaggt gagcaggcag ccgtcaaaga ccttggaaga 71640
gtcaataagt agggtacccg caagtgaaaa tttttctcat tccacaatac cagaaggccg 71700
atttcttagg aagtttcagt ttcttgtatg tatcaggcac tatgctaggt gctggctgta 71760
caatgataaa gtttcctccg ttactgagtt cacagtctat cagcctagtg ggaaagaaca 71820
gagaacataa ggaaaatgca tgaggtgtac tatatatgtt taactgtaca tagtacatac 71880
aaaagctata cagcacaagt aaagcctaca ggacatgctt acaggcaagc tctgtctact 71940
tcttaaatca actgtaatcc attaattcca gtccccatat aacagctatc ttctccagga 72000
atattgacca gtacggccta cccgttttac ttggtaaatg tggcaaagca gaccaccagg 72060
tcaattcagt tcaattcaat tctccttgac gcaaggtaac ctgaaggagg gtcaagttct 72120
gtccttggag ttcagacagt agattccagt cctgatgctg ttcataactc aatataggct 72180
tctgtctctc cagaatatga gatatttttt ctattatctt ctcctttatg ttgttaatta 72240
tataaattgt acttatatta ttttatttat ttatttttca tagtggctta aatgtccttc 72300

tctgctaatg tcaacatgtg aataacatgt gaattacatt tatccatctt ttgtttttgt 72360
tatgatcatt tggtctctct ttttttattt tttagcatgt ctcataattt ctccttaatg 72420
ctggactttt taaatggata cttaggtagg acctggatga tgttctcttc ctctaaagaa 72480
caagaaaagt tttctttttt acaggcaaag ggagaaccag gcattacctc gatcctggcc 72540
agagttagtt ttagcctttg ttagggctgg tctattttaa cattatctta gtactggtac 72600
ttacttccca actgcgttct ttagccctag tgtgggctat ttctcggcct caactacaac 72660
ctgcgtgcgt tttatcaagc ccttccacct tggcagagct aaactctaac ttctgtttct 72720
ccaacactac tcaggtgctg aaatctctgc ccaactttta gcttcctggg tgctgttttc 72780
tgctgtgttt cttggagcct cttcctgaac atgcagctta ggagacggcc aataattgga 72840
atgaattcct gtcccctaag cccaataaga attccatttt atgtttgagc tctctctccc 72900
tcatttggaa attaccctca tgggaaaagc caggcaaaca tgaattttac cttggatatt 72960
tttcttcttt caaggagtat aactctccaa ctcaattcag gactgcattg tttgctttca 73020
taggcattca aacaggtagg ggtgtgtgtg tggtggggca gggtgtgtgt gtgtgtgtgt 73080
gtgtgttcca aatactatag ttgtttttat ggaggggaga ggtacagaca tccccagctt 73140
acaatggttt gatgattttt ccactttaca atggtgtgaa agagatatgc attgagtatg 73200
ctccttgact taagatttgt ttactgggag gtaaccttac cataagttga atagcatttg 73260
cattccagtt atctattgcc actctgaaac atcgaactgc aaaactcagc agcttaaaac 73320
aacaatttat gtttctgtga gttggctcag ctggagagtt tactagggaa tgttcatgca 73380
attacaatga gatggcagct gagctgtggt tatctgaagc aaactgtact ggacttgtaa 73440
gatgtcttct tcattcttct gtctctttct tcagtgctcc ttgacttatt ttactttcta 73500
ctaattatct cagaatccag ggcctaggca tctcacagca agatctcagg ttatttatgc 73560
ttctggcatt gtgactcact gctaagaggc agaaagcagt aatgtccagg tttgataagg 73620
gttgcatctg taaatggcac aggataatta ccattatatt ttgtcagtca aataagtcac 73680
aaggaccacc caaattccag gatagagaag cagattccac ttcttcatgg gagagtggca 73740
aggtcacact acagaagagc atgtgagatg agataagatt gtgtgcaatt ttggcagata 73800
caatctacca tagatacaac ctattcttct attgtttact ttatgatttt tccactttat 73860
gatggtgcaa aagtaccatg catttggtgt gctccttgac tgaagaagag tttattgcag 73920
ggtagctcaa ctaggttaat gaaacaccat aaatctttta aacaagaagt ctcaatcaaa 73980
actgactgaa gaagatatga aaaatctaaa taggctggga gcggtggctc acacctgtaa 74040
tcccagcact ttgggaggcc gaggagggtg gatcacctga ggtcagaagt tcaataccag 74100
cctggccaac atggtgaaac cccgtctcta ctacaaaaat tcgctgggcc taatggcatg 74160
cgcctgtaat gccaactaca tgggaagctg ggaaaggaga attgcttgca cccgggaggc 74220
agaagttgca gtgagcagag gtgacgccat tgcactccag cctgggcaac aagagcaaaa 74280
ctgtctcaaa aaaaaaaaag aaagaaagaa agaaagaaaa atctcaatag tctcgttacc 74340
atcaagtcaa gtcaagttct gccaagcatt taatatacaa agtcaagtca aatgcttcca 74400
gatcagaaaa tgaggaaaca ttatcaaact tactatattt gtgaatctat tcgtgctact 74460
gtcatgaaat atctgagact gggtaattta taaagaacag aaatttattt ttcatagttc 74520
tggagctggg aagtcgaaga tcaaggcgct agcaggttcc gagtctggtc aggtcagctc 74580
ttcatttcca agatggtgcc ttgttgctgt aatgttcccc tctggaggaa tattgtgtct 74640
tcagatggca aaagagatgg aaggggtgaa aagggttgta ttccctctgt caagaccttt 74700
tataagaaac ctaatcccat tgatgagagt gtaactgtca tgacttaatc accccctcaa 74760
gcccacgcct ctcaatactg ctgcattggg attaggtttc aacgtgaatt ttgaagggaa 74820
caaaaacagt agcataacct taattccaaa actatacaga gatgcaacag gagatgaaaa 74880
ttgcagccta actttattca gggatattga ggcaaacatc ctaatgaagt atcagcaaaa 74940
caattgtgtt ggctaaggtt ctttgagaag caaaaagtgg gcttagatgt acaagagact 75000
tgtagagaag gataaatggg aagaagtagg agtaggcagg taagacttca ggttttaatt 75060
taactcttac accagcgaaa ggagagaggg aaggatggag gaataggagt acctcggatt 75120
gcagggcaga ctcaagaaaa ttttggccaa gtcaatgagg gggtccttaa gtcagtcaca 75180
gtttagatga attttatgtc ccataagaat gggtcagtat cataatcttt accacatgca 75240
gtcattggtt gggaggaggc tgggggaagc atgcttttga cccagatgta gtgatgatcc 75300
agaagggtgg caagtggagc tgtcagttaa tgatgctccc tgaaacaaga gatgttcatg 75360
atctattttc cttgccacta cacaaatcta gcaatatatt ctaagatgtt taaatgtatt 75420
atcagtgtat aaagataata tgcatgttgg atatagctca gaaaagtaat attattttaa 75480
tattcaaaat tgatgtacac aatttaatac attaagaaat tagaggaaaa aaatggtgag 75540
atgcaatggc tcacacctgt aatcctagtg ctttgggagg ctgaagtggg tggatcacct 75600
gatcccagga gtttgaggcc agcctaagca atatagtgaa acctcatctg tgcaacaaat 75660
taaaaaaaat taagaagaaa ataatttaaa aaacaattag atgggcatgg tggtgcatgc 75720
ctgtagtctc agctacccag gaggatgaag tgggaggatt gcttgagccc cagggattga 75780
ggctgcagtg agttatgatt gaaccagtag actccagcct gggcaacaga gtgagaccct 75840
agctcaaaaa aaaaaaagag aggaagaaaa aaaattaaag cagaaaatca tgtgatggag 75900
aggggaaaaa ggaaaaaacc tggcaggcag gcagttaggg tgggtcctca gttgaatttt 75960
ttcaaacaaa agaacagcct gaaaactcaa gctgcaggca cagataaggg aacttgaaca 76020
agggggttgc ttgagacatg cctacagcca cacagataag aaacactaca caggtgactt 76080

gtccagacat gcctgcaatg gaaaattctg tcccctaaca tgtgcacacc aaggggaaca 76140
aaacaatacg gagtaactca acctaatggc ctgcatgtgc attaggagga cagggtggag 76200
ctaccagaaa tccacacctt atgcaaatga gatgtctagc cctcatcggt tttttataaa 76260
agcttttgca ttcaattgta aaaatggcaa cccttttcca ggccctttct ccacagcaga 76320
gcgctttctt cttttgttta ttaaactttt gctccaacct caccctttgt gtccacactc 76380
cttaattctc ttggtcatga gacaaagaac tccaggcgaa acctcataac gagagactgc 76440
tacattgtgg tgtattggct aggctgtaac atgatctgcc taatcatgtt gaaacatcta 76500
atcattatga aaacttaaga aaaaaatagg aatataaaga cttttcttaa cttggtaaaa 76560
gatctataag aaaccaaact ttgggaggct gaggtggggg ttcacttgaa gccaggagtt 76620
caagaccagc atggtcaaca tggtgaaacc tcatctctgc taacaataca aaagaaattt 76680
agctgggcat gattgtacat gcctgtaatc tcagctactc aggaggctga ggctggagaa 76740
acacttgaac caaggagaca gaggttgcag tgagcagaga ttgtgccact atactctagc 76800
ctgggtgagg gagtgatacc ctgtctcaga aaaaaaaaaa aagaaagaaa ccaagagtaa 76860
atataaaaaa caatactgaa ataatataac tagtccctat aagatcaaaa gaatacccac 76920
tatccatccc cactcctatc caatattgta ccacaggaaa aaaaaaaaaa aaggtcaggc 76980
acagtggctc atccctgtaa tcccagcact ttgggaagcc aaagtgggag gattgcttaa 77040
gcccagcagt ttgaggttaa cctgggcaac atagtgaaac cttatatcta caaaaaatta 77100
aaaatttaac tagacatggt agtccatgcc tgtagtccca gctactcagt gcaggtaggg 77160
ctgaggtggg aggatggctt gagcctggga agttgaggct gcagtgcact atgatcatat 77220
cactgcattt ctggctgggt gaaagaatga gaccctgtct caaaaaaata aagacttaaa 77280
aggcatagtg gcctggcaca gtggctcaca cctgtaatca cagcactttg ggaggctgag 77340
gtgggcggat cacgaatcag gagatggaga ccatcctggc taacacggta aaaccccatt 77400
tctactaaaa aatacaaaaa attagctagg catggtggtg ggcgcctgta gtcacagcta 77460
ctcaggaggc tgaggcagga gaatggcgtg aacttgggag gcggagcttg cagtgagctg 77520
agattgtgcc cctgcactct agcctgggca atagagcaat actccatctc aaaaaaaaaa 77580
aaaaaaaaaa ggcatagtaa tagaagagat taaaatgtca tgaattacaa atgatactat 77640
tgcttatttg gaaaatccaa aactatttac aggtaaatta tgtataaaga aaagcaaaag 77700
gccaagagga gataagacac cccagacaag ataagataag ataagacaag ataagataag 77760
ataaggtaag gtaagatgag ataaggtgag aatgggataa gataaggaga atatcaagat 77820
agaaaaacac atcctactag atacggctgt agtaaaatgt aatagtgtag attggtacag 77880
aaacaggcac caaggaaaca taataaagca ctcaagaatg aacccatgtg tatgaaattt 77940
tatttttgat agagcaagca ttgctgtaac atgcattaat tagaaaatta cctcgaggag 78000
gacccttaaa agggagatgc cagcattata taaccaaaag tttttctgac agttttctgt 78060
aacatgtacc aacattctct ttctgtcccc tgtcttctga cctgaagaaa tgtgttcctt 78120
ctttaaggaa aaaggatgat gaatggatgg ttttaatgtc cctttatgac ttttcaagga 78180
tttctttgac aatacacaga tttcatacct aattgtccac caactacaaa caaaattaat 78240
tttagataaa ttcatagtct gaagttaatt ctggaaagcc catattatgt ttgacaatct 78300
taaaaacgtt tctctttcta tttgttgcca atttcacccc acaaatctta tctttgaatt 78360
ccaattaaat atggataata taaattacca ttgtagaatc cagtgtatgt agggttacgg 78420
gtgtagtcag ataactgaat gttctcctta ccacctccag ataaattctc ttcccctctg 78480
tgattcactg atcataaatc ggtttactgt gaggaaaaaa agaaatggat aggttgacgt 78540
tgtccccagg tggtaggtag gatgaactgc ctatgaaaca atgcaatcca ctccaggcag 78600
tacacatttc aaaatgcttc tccctatggg gtttgacagg tacagacaat tgttgttcca 78660
aatgccatgt gctttagcaa atcattctgc ctattgtgag tagtcacctt tcaagccacg 78720
agttaccatt tgagttcttt tgcctcaaac tgatggagaa aatgaaaaca gaatgtcacc 78780
catgcagttc cagtctgttg tattcaagaa tttgatatat tcagacctat gcctggcttt 78840
cagtaccatg agctaatgac atttcttggc ttgactataa agttctcttg acaatactgt 78900
gtggatgaga ttttaccgtt tgtgctaaag tcaacaacaa aagcaggacc tggtaatgcc 78960
aacaactatc taaagaaggt cgatcagaaa aacgacaagg tggcctattc caaagcaact 79020
ctgcattctt ataattttgt taatggacct tctttttaat aggtttctgc aaataaagtt 79080
tgaggttata aaatgtgagc ctactagaat gacacaaagt tgaatttcaa atgttgtttt 79140
tttggatgtt ctaaaaatac attgtaaatg gatttctgct aaggatggac tactgtcttc 79200
aaagctagag aagataaatt gtggaatcaa gccagctgct cagagaacat agagaggttt 79260
ggggtctctg gttatgcata ctaagtgcaa agctatcacg tggcccagag gcgggcaggc 79320
ctgcttgact ttaataatca gtgcctgcag taggtagcct gtccttggga aatgggtgtc 79380
tatagctttg ggcccatgtg tagcctctgg ttggctacca tgcccactcc atttttatgc 79440
tcattttccc agttccagag agatcaatgc tgaggctggc taatattaat tggctgtgtc 79500
attttgtcca cttgactgtt cagtgtgact tctgtggtgg gatgctctct ggtggccatt 79560
aacatgtgat acaaaaactt tgtgtacaaa actttttgga tcacatgtta atgttttcat 79620
tgctgtgcat ctccattttg tccatccacc tgcctctacc ccagccctcc ttgtgtctaa 79680
tggtctggtc cttttcttta caggcctttg acccgctagc catgctgttc gtctcagcct 79740
agggatatct atataatctt accttgggtt ccttctcctt ctatgccaga aacaccactt 79800
gcatcctgct cattaggaaa ctttttttcc cctccccatt atcttttttt tctttttgag 79860

atggagtctt gctctgtcgc ccaggctgga gcgcagtggt gcaatcttgg ctcactgaaa 79920
cctctgcctc ctgggttcaa gcgattctcc tgtctcagcc tcctgagtag ctgtgattac 79980
aggcacgtgc caccgtgtct gctaattttt gtatttttag tagagacagc gtttcaccat 80040
gttggccagg gtggtctcga actcctgacc tcagatgatt tgcccacctc ggcctaccaa 80100
agtgctggga ttacaggcct gagccactgt agccagcccc cactatcttc taaggccacc 80160
tctgaatatg tcagcaatgg aaccaccagg tattttcatc atatacccac aaactgagcc 80220
aacccatata taaaccaaac tcaagttgtt cctcgttcat ctggttgtgt ggcatctctc 80280
cccatagggc cacgggtgtg agctggggaa gggggattaa tgcaaccatg gatggtgact 80340
tggggacctc tgggctgcct gatcctgcag cttacctgtg ctctctggcc ctgttcaggc 80400
ttaacttggg atgtatcatt ttcatcttta gtaagatgca gtaacggtaa ctattcctca 80460
atgatgtgat tctctgcaga tgacatggcc ttccttcaga atccaggggt ctgcatagta 80520
tttttcctgt ttggactttc tataaatgac acactacatc ttttctcacc gaggacccct 80580
ccgacatcaa aacgtcagct ggatgttatg gccaatgaat caaggctgct cattccatgg 80640
gctggacctg ctgcagagtc cttccttggg tgttatggcc aatgaatcaa ggctgctcat 80700
tccatgggct ggacctgcgc agagtccttc cttgggtgtt atggccaatg aatcaaggct 80760
gctcattcca tgggctggac ctgctgcaga gtccttcctt ggatgttatg gccaatgaat 80820
caaggctgct cattccatgg gctggacctg ctgcagagtc cttccttgga tgttatggcc 80880
aatgaatcaa ggctgctcat tccatgggct ggacctgctg cagagtcctt ccttggatgt 80940
tatggccaat gaatcaaggc tgctcattcc atgggctgga cctgctgcag agcccttcct 81000
tggatgttat ggccaatgaa tcaaggctgc tcattccatg ggctggacct gctgcagagt 81060
ccttccttgg acgttatggc caatgaatca aggctgctca ttccatgggc tggacctgtg 81120
cagagccctt ccttgctctg gactcacaaa gcaggcagcc ctctgtgtta gctggtgtat 81180
ggacaagagc agcattccat aaaccaagaa ggttccaaag aggtttaaca ggctgtgtgc 81240
tgttctttgg ggaaagggat gcaagatgca gcaattgttt ttcactttgg agggaatagc 81300
caggcatttc cctaacctct gacccctaaa tactttataa agcagcaggt ccctgacttt 81360
tcacagggtt tatcctccac cttctgctat gcatgtttct taccaaggcc ttcagtgtcc 81420
tggccatctc ttcctggtcc tgctcaatca gtatgatggc attgatatgg ggaatggata 81480
tgatattcta tgggaggtct agatctcctt ggattccacc acagctgaga gtggggaatt 81540
aacagaagcc tggagcaaaa ctgtaaatga atgttcctgt ccattccacg tgaatgcaaa 81600
ctgtttctga taagaataga agctgggcgc agttgctcat gcctgtaatc ccagcacttt 81660
gggaggccga ggtgggcgaa tcacctgagg tcagtggttc aagaccagtc tggctaacat 81720
ggcaaaactc gtctctacta aaaatacaaa aattagccag gcatggtggc acacgcctgt 81780
agtcccagct acttgggagg ctgaggcagg agaatcactt gaacctggga ggtagaggtt 81840
gcactgagcc atggttacac cactgcactc cagcttgggt gactaaatga gactccatct 81900
caaaaaaaaa ccaaaaaaca cacacacaca aacccacatt tgccaaatca atggcagcac 81960
gctcttactg gagacattat taatctgttc tagcaatgcc actttcagca ctgcagccaa 82020
aataggacta ctacttggtt gagtgtgcat cattagtcta cagtcatttt cttagatcca 82080
gccagtttct gcaaggctca ggccagtggt gaattgaaca gagataggac agagctcacc 82140
attcctgcat tcctttggtt tttaatgatg gtatttatgt ttgccacccc ctggtatgtg 82200
atattgtttg tgatttacta tcttgaccat ggagggggca agattcagac ttctacttga 82260
ccttttctac tgtgatagct cttacctcgt aagctaatgt cccaaggtgg ggatactcct 82320
gctgtcaagt acattcattt caactctaca cttgccgact agaaaaatga tgagtggact 82380
tgtctatgca cccagtgggc ccactctgaa ttacacttta cccagaaccc tgtttgtttc 82440
tttgtcccca aatcccccat tctaacagag gggccatagt gttgcttcag atctctgggt 82500
actataatca gttgagagcc tgtgttcaaa agtcccccaa aaggtctaga gattccactt 82560
accccagtga acagttgctc aggtaaacag ctacagatct gtctggagaa aagtcttggg 82620
aatcattaca ggaattattg ctgcagcatt gcagagttct ttctcttagg gctccaacta 82680
cctcttgagc caatagcttc tagatctgaa aattgagtaa agtctgagaa ctgagcaagg 82740
tatgaaaact ttttattggg tgatcaccat tagttttctg ctcctccatt cttgcctttt 82800
ttcttgttgt agaaattaag aaggcccctt gctggttgac tgtctatttt tcccctaaag 82860
gagttaggtt ctattaacca tcttcacaac tttctgtagg ccaagactct ttggctgctc 82920
ttccaagctt gctggtcact atggtcatcg tgactccctg gcttctaatg gttaagtgac 82980
accccatcct ccaccatcag tccaggcctg cagaccttat cttactagct gttttctctg 83040
atcactcctg gttccaactg tgtctgttca ggtcttctaa gaagtagaag ccaagacagg 83100
attatatgta caaaagattt attggatgaa atgtctgtga tatggtttgg ctgtgtcccc 83160
acccaaatct catcttgaat tcccatgtgt tgtgggaggg acccagtggg agatgattga 83220
atcatggggg caggtctttc ctgtactgtt cttgtgacag tgaataagtc tcatgagatc 83280
taatggtctt aaaaatgaga gtttacctgc acaagctctc tttttgcctg ctgccatcca 83340
tgtaagacat gacttgcccc tccttgcctt ccaccatgat tgtgaagcca gccacgtgga 83400
actgtaagta cactcgacca ctatcttttg taaattgccc agtcttgggt atgcctttat 83460
cagcagcatg caaacagact aatacagtct gtgacagata aaagaagctg gagaagaagt 83520
aggcaggaca agccttcaga tcacagtgta agttttatat ctgtaaaagg agaggaaaaa 83580
ggaagaattg atctggaaga gtctgagctt acagagcttc tctaagaaaa gtcttggcta 83640

ggactatggg gtgtcctgaa caaaagttac ccattagagg aatctttcaa caggcaaatg 83700
gtcctatgcc agtgtacatg cccggctacg tcattgggtg ggaacagcct agtggtataa 83760
ttggttcaga aagaatgcag gggtatgaca gcttgagact gtcaatcagc tgtattccat 83820
ttctcaaagg gagctctgaa cctccatagt catctgtttg gtcatcaggg ccacacacta 83880
attattatta aagaagaata tttccgtaaa tgcagcagaa tctgcaaaga ttcctctgag 83940
cctccaatga ggagaaaata ttacattttg agacagaaca gtgtatgtgt tcattctcat 84000
ttttcagact gtgccttggc tctctctgca tttgagaaaa gaaactgatt acatatccag 84060
ccatcctttg atggcctcat cagctacacg aaggcccaat taaagtttta aaattattgt 84120
tttcctttaa tgttattaat atggtttggc tccgtgtccc cagctaaatc tcatgtcaaa 84180
ttgcaatcct catgtgttgg aggaggggca tggtaggtgg ccattgaatc atgggggcgg 84240
acttccccct tgctgttctt gtggtaacaa tgagttctca gaagatctgc ttgtttaaaa 84300
gtgtgtagca tttccctctt catgctctgt ctctcctgct gctgcgtgaa gaaatatttg 84360
cttccccttc ccctttccac tctgattgta agtttcctga ggcctcccca ggcaccttcc 84420
tgtacttcct gcagaactgt tagtcaatta aacctctttt ctttataaat ttatactagg 84480
taatttatgt aattacccag tctcaggtag tgcttcatag caatgtgaga agggactaac 84540
acagtcattt actcaaagta actttccttg atcttcttct ttactttgtt tccttatcct 84600
accttacttt tcttctacac actcattact atctatgtta ttccgtattt atgtgtttgt 84660
ttactttata attttctgtg tcctccatta gaatgtaagt ttcatgccag tagaagcctc 84720
ttctgtcttg ttcactggag tatcttcggt gttcagttca tactgcctgg cacagagtat 84780
gtgcataact tgtgattatt ttgaaagagt agataaaatc tgtggtaggc cagcctcccc 84840
catacatacc taaaaccagt cctcagagtg gaggatgggg atagagaact aataacatta 84900
tagacacttc acaatcatcc tacaaataaa gcattatcac ccccattttg cagataagga 84960
aactgtggtt tatagaagtt aagtaatatt gacaaggtca tttggctagt aaattacaaa 85020
gctgggctct gaatgcagtt acacactata tgtcctcaac cactatagag tattaatagc 85080
aaaaaaaaaa aaaaactctc tagcttgtaa atagatgagg ttctgggaaa cactggacta 85140
gttcaatttc caaacttctt gaaagtaatg actctcacca tcaagctaca taatcgctaa 85200
attataaaac cggcagtgtg aaaaatccta aagcacttct gaattgtcca cctaacaact 85260
gattacatcc tcttctgcaa agggaaataa aactgtttgc taatcaggcc tcagaaaggc 85320
ttaagtgttc cacaccgaat atcagaaatc gggaaaatca ttaagtcatt acctcgtgcc 85380
aaattgaatg ctacagtgta cttttctggt attttaacct gtctagcatc taatgactca 85440
gaaagcattt ctcttattta atccctctca gcgttttttt tttccttact gagaagctac 85500
tattaacctc tcttgtaaag aaagtgagtc tcttcagata tctgtcaaat aattttatgt 85560
ggttaaccta aagcacagct ttttctaaaa aaaaaataga aggatcgttc tggaaaggag 85620
acaagaaaaa gcagtccagt gggaagaaca taattgtctt agcatacccg ttgctggcag 85680
atgcatgaaa aagccacaca aatgagacac cagcatctaa aatcaaatgc ctgtcagctt 85740
aaattgtagt ctttggtaaa cctgatcaat tattcagcct tcttacacat ttaataaagc 85800
tccagaacag agcccagaaa atagttgcat ataaaatatt ctttgagaaa taaattacgc 85860
tttagtagaa agctacagtc tttacctaag ctaactcgga tgatactcca agtgagctat 85920
ccttcctgct tacttctgcc tttttttctt caaagggcaa tcctttttga attggcattt 85980
agcatggtag tattaggttg gggggaaaaa tcgtgttttt ttgccattaa aagtaatagc 86040
aataactttc gccccaacgt aatagttgct tcattcctgg agagtgggat ctgcttggtt 86100
taccagccag agggcttgac aaaatctgac tttagtgtct tcgaatgtcc ttttctaaca 86160
tctgcacaga aatatcttca tgggagaaaa tcatcttttt cattaaaaaa tgtcagtgtg 86220
ggagtatggg gagaggaaat agggagagaa agagaaggaa agaggcgcac acatacagag 86280
acagagagag acagggagag agagagggca agagagtact gccttggggt cactaacacc 86340
cttgaagata tctcagaaga ttgttccaac caagtaaaaa tgtagacaaa aagtcgccgc 86400
tccttcttca gacacaacgg atcttgtgaa gagcctggta gggcaaaaac ttgagaggcc 86460
attgtggtgt ccaaagaaat actgacatct actggtgggt tcggaagtca caggactttt 86520
ccaagggcca catttagttg cacggaattg aaagtattaa aatccagtgg atacaattag 86580
gcattcctga ggatcccttc taaataaatt tttaaaatta acttatgacc atcaagagca 86640
tttctgaatt gtctgttctt taatccacca gatagataat cattgactta gtctctgtac 86700
ctgaaatatt tcacacagtc cagggaagag taaaatgaac aaaattcctt tctctttatg 86760
accccaggac ctacctcatc aaaaacggtg ctttgaaaaa cagcaagaag aatgggaagc 86820
aatattgctc agtcagaaga taaacctgtg ttcacatcca ggaactttca tttattggct 86880
gtgtaatttc taccaagtta ctttgtctcc caaatctcat tctttctcag ctggaaatga 86940
gaattgagga tacctgccct gaatgactct gtcagtaatt agagaccacg aactcaaagt 87000
gcctgtggtt cacattacac actgagtaat tgccgggcgc ggtggctcac acctgtaatc 87060
ctagcacttt gggaggccga ggcgggtgga tcacctgagg tcaggtagtc gagacaagcc 87120
tgaccaacat ggtgaaaccc catctctact aaaaatacaa aaattagctg ggtgtggtgg 87180
catgcacctg tagccccagc tactcaggag gctggggcag gagaattgct tgaacctggg 87240
aagtggaggt tgcagtgagc tgagatggca ctactgcact ccagtctggg cgacaaagca 87300
acgctccatc tcaaaaaaaa aacaaaaaca aacaaataaa agaaacaaaa aaactcagta 87360
atgggattgt tgttacattt aaaggataga aacatcctgg gaccacttgc ttcttgattg 87420

accaagctgt gatttaggag agtacttctc aaactttaat ttgcactcga ctcactaggg 87480
aatcttgtta aaatgaaggt tctgtttcag ctgccaaggg cggggcctga gatactgcat 87540
ctccagcaag ctcccaagag atattgatat gctggcctgg gacagcactg agtagcaaga 87600
atctgtgggg tccaatcccc actagaggtc aagagcattg aggtggcccc tgtcaattgg 87660
cttcctatgt gaggggaaat agctctcaaa atttcagttg tttcaaagta taaacaaagt 87720
gtacataaga aaattgccag gaaagatggg tttgcctaat gtagaaacac taaaaattgg 87780
ccgggtacag tggctcacac ctgtaatccc agcactttgg gaagccaagg tgggtggatc 87840
acctgaggtc aggagttcaa gaccatcctg gccaacatgg cgaaacccca tctctattaa 87900
aaatacaaaa aaacattagc taggcatggt ggcacgtgcc tgtagtccca gctactcggg 87960
aagctgaggc aggagaatcg cttcaacccg ggaggtggaa gttgcactta gccgagattg 88020
caccacggca ctccagcctg ggcaacagag caagagactc tctctccaaa taaataaata 88080
aataaatact aaaaatttac ttcacaccca ttcccaatat acagattcta cttatcatga 88140
tagagaagag gtgtttgggt tctgaatcag aaatgccccc ataggttaga catggtaggt 88200
cacacctgca atcctagcac tttggggggc tgaagtggga agattactcg agccctggag 88260
tttgagacca ggctagtcaa tgtagcgaga tcctatctct acaaaaattt taaaattagc 88320
caggtgtggt ggtgcatgcc tgtagtccca gctactcagg aggctgaggt gggaggatcg 88380
cctgagccca gaagttcatg gttgctccag ctagggtgac agaatgaaat cccagtagtt 88440
aatttattct gtttcagctt taggctttct gtagaatctg gcaatggcaa gcaattaagc 88500
taaggggatc tggagtcaca ggtgaatttg catcctgact cggtaacttg ccatctgcca 88560
ggtcatagac aacttcctta acttttctag gcctcagatt tctcagagta tattgtgaat 88620
attgtgaaca attcaatgag ataatacaga taacacatta gcacatatac tgctcaataa 88680
atgattgtat tataattatt tttagacaaa ataatgcagc tgttatttct ccccagcaca 88740
cttttttttg agaatagagt ctctctgtgt tcccaagact gatcttgaac tcctgggctc 88800
aagcgatcag tctccaaatt agctggtact acaggcaaga gtcatccctt aatttttaag 88860
tatagcacta tggctttggg gaagagtgtg gtagagaaag cagtggaaca gcacagaatg 88920
ctgttttgca ggaagtaaga tgcaggttta aatcctagtt ccaccaccag aatggtggag 88980
gactttgggc tgggttatat agtgtctctg ggcttcaggg ttcatctgca caatagagag 89040
atgacaatgt aggcggtgtg aggattagaa aactctgtca agtcctgcta tgcaatcagc 89100
cctcaaaatt agttgctatt aacaacagtg ggtttttggg tttatgtttt tgtttttgtt 89160
ttgagacgga gttttgctct tcttgcccag gttgtaagtg cagtagcaca atctcagctc 89220
actgtaacct ctgcctcctg agttcaagtt ctcctgcctc agcctcccga gtagctggga 89280
ttacaggcac ccactactgt gcctggctag tttttagtat ttttagtaga aacagggttt 89340
caccatgttg gccaggctgg tcttgaactc ctaacctcag gttatctaca agatccgaag 89400
tgctgagatt acaggcataa gccaccgtgg gatattacat aatacagtgg gatattaata 89460
ttgatatagt gttttctgac ttaggttcgg tagctcctta gactcccctg actcctctac 89520
ctagattcct gctttttttt tttttcaccc aggctgcagt gcagtggagt gatcttagct 89580
cactgccacc tccacctcta ggttcaagcg attctcctgg ttcagcctcc tgaggagctg 89640
agggaggctg agggattgca ggcgtgtgcc accacgcccg gctaattttt gtatttttta 89700
gtagggacag ggtttcacca aattggccag gttcatctca aactcccgat ctcaactgat 89760
ctgcctgcct tagtctccca aagtgctggg attatgggct tgagccatca tgcccagccc 89820
aaagtcatgt tttaattatt gtaattagtt aaatgatatt ttaaagtatt tttacacacg 89880
gataattttt tacggtatac aaatgttttc cttacatatt agcatcatca ttttgtaaaa 89940
caatggtgta aggtaggaag ggtgagtgtg tttttattta tttatgtatt ttacaagcaa 90000
agcaacagga actcagagaa attaagcaac ttttttaaga ccatggagtt cgcagtgaaa 90060
gatattctcc ctcccacaga acttatggta gagcctcatg gagcagaaag tagtatttaa 90120
ttgggctggt gaaaatgccc tgtgttttta ccactattgg gtgattgaga cttgagcatg 90180
attgacggaa aaagtcactg tatatcggct cagaagaaat gagttcaagg cccagcactg 90240
ttacttttca ccggtgatgt tgagcaaaac catggttatc tcagattctt gctttacctg 90300
tagaaggagg actttgaact agatcatagc caatagtctt ctttgctttt aaactggtgg 90360
tagggatggt ggggttaaaa gaaaaactgt agccaagtta aatttaaaat aatttaattg 90420
agcaaagaac aactcatgac tcagacagca tcctgagcca gagtgagctc agatactcca 90480
ggacagccat gtggtggaag aggatttatg gacagaaaaa ggaaaatgac atacagaaaa 90540
tggaagtgag ggacagaaac agccagattg gttgttgctt ggcatttgcc ttatctgaac 90600
atggtttgaa cagttggcca atgtgattgg caaaacttgg tgactggcgc aagagtaggc 90660
tacagtctgt ttacaattcc atttaggtta tggttcacta tgtacagaga aacctttagg 90720
ctgaacttaa aatatgtagg gaggtaactt taggctaaac ttgatttaac agtgttcatt 90780
gccagaggaa gagagatgta gaaagcaaaa agttcctctt caaattatcc cttcttgtta 90840
aagaataaat cataagtgtt agaaatcata gtttctttta aagactaact ttcttcaaac 90900
ctccttgctt cacactaata actcgttttt aagccctata ttatgtagct gttagatata 90960
aaggaatgag tacattctat gtccttgtac tttaaccaag atatctgtgc tggacatgct 91020
cacaggcatg tcccagctcg cagcctatac cccttcctta tttaggaatg ttactacttt 91080
tctaagtctt ttcataaaca acttccactt ttcgtttgtt gtacattgct tttatctatt 91140
tagaaaattt taagttgtta gtcaatcagg ttcagtttag attgtgtggt ctggctccag 91200

ccaatggaga caggacacag tagcaagggc aagcacttta agggataaaa attgcttccc 91260
tccttctcgc cattattcca tctgcgagga gcaccctttc tgcagaaagt aaatttgcct 91320
tgctgagaaa actttttggc tgaatggtga tttttccttg cagtaccgag gaacaagcat 91380
tctgtttctg aataaacatt ttacttataa caagagataa gattaaatat aagaatatat 91440
tatattctct attttagata agcttgtcct tgaatgttga gcatataatg tcatccaaag 91500
catcgtccaa cagcaacact attctcctgt tagtacccta ttctcccttt taggaaatct 91560
cccatagtgt ggaagtcttt ctactgaaat ctaagtaaca gacaattcct ctttctacca 91620
caaagcagtc agggatggag gactctttcc ttggacttca aatcttcagt gagtgttagg 91680
aaggtagaga gatgtctgag gttcatgttc acggccacat ggttgatgat ttatggacta 91740
gacacagcgt gctcttttgt gtgattccag ctctctagct tccccagact tcttggctgt 91800
tttttccaag cctggtcgtc catctgccat ctcattccaa taaattggaa atgcagttca 91860
agatagctag agttggagtc tgtggtattg ggggaacccg cccccaatat ttcaatgtag 91920
gttctttcta ttttccctaa gtgtcggcgg gtctgagaaa taaagagaaa gagcacaaag 91980
agaggaattt tacagctggg ccgctgtggg tgacatcacg tatcagtagg actgtaatgc 92040
ccacctgagc ctgaaagcca gcaagtttta ttagggattt taaaagggga gggggtgcaa 92100
gaacagggag taggtcacaa gatcacatgc ttcaaagggc aaaaaggaga acaaagatca 92160
catgcttctg aggaaacagg gtaaggacaa aatcaggaac tcctgagaag ggtctatgtt 92220
tagctgtgca cgtattgtct tgataaacat ctcaaacaac agaaaacagg gttcgagagc 92280
agagatctag tctgatctta aatttaccag ggtggggttt cccaatccca gtaagcctga 92340
gggtactgca ggagaccagg gcatatatca gtccttatct caaccgcata ggacagacac 92400
tcccagagtg accgtttata gacctccccc caggaatgca attcttttcc tacggtctta 92460
atgttaatat tccttgctag gaaaagaatt tagcgatatc tcttctactt gcacgtccat 92520
ttgtaggctc tctgcaagaa gaaaaatatg gttttttttg cccgacccca caggcagtca 92580
gaccatatgg ttgtcttccc ttgttcccta aaatcgctgt tattctgttt gttttcaagg 92640
tgcactgatt tcacattgtt caaacacatg ttttacgatc aacttttaca gttaacacaa 92700
tgatcacagt ggacctgtgg tgacgtacat cctcaactta cgaagataac aggattaaga 92760
cattaaagta agacagtcgt aagaaatttt aagaatatta ttagggaagt gataaatgtc 92820
cctgaaatct tcacaattta tgctcctctg ccatggctcc agccggtcct tccattcggg 92880
atccctgact tcccataaca tctggcttgc cactaagacc ctgatgaacc aagcctgcat 92940
tttttttttt tttttttttt tttttttttt tttttttttt tgctaaagct actgccaacc 93000
ttcttgtgat gatctgaggg aatacaattt aaaccatgat tcataattgt gcgaggtaca 93060
tgagtcacca agaaaggcag ggtaagtaat tacaaagtag ttatctctct ttccataatt 93120
ttaaattgaa tatcacttta ttctccattt acagacagag agattacagg cagtaatctt 93180
cctttctaaa gctgacagta actcccacag taataaatgg aagcctgttt aataagaaat 93240
atttacgtgt gtgactgtga gcacagctga aaacgtggtg ttcttcgtaa atttagaact 93300
gcattttcta aatgtcgagt gtttattacc tggaaaaaag aaaggaaaaa caactctaca 93360
ttttagacgt gttccacagc ttccttgatg agacatttta aggaccatta gttgtctaaa 93420
tagagattgt attttagagt ggagactatg aaagagtgtt ctctagttta aggttatatc 93480
ctttaccttg tgatagagtc aagctgtaaa ctcaaagcaa gcatctggca ctctggttat 93540
tctaagtcat ctttctttgc tctccctttt aagcatcttt cttcacttta aaaacaaaca 93600
caaataaaag cacctatcac agggagggcc tcaaacgagg gcttaatagg tgttgctttg 93660
cttcctcttc tcttttcttt agtaggcaga ctaaattcta tgactctgat ctgagggtta 93720
acagtagtcc atctatggat tctatggact tgatgtatct tatactgtgg ttcctggaaa 93780
ttcattttat caggagtgag tgatcttttc agaaaagctg ctcaagcttg agcctgggag 93840
gtggaggttg cagtgagctg agatcatgcc actgcgttcc agcctggaca acagagtaag 93900
accccatctc aaaagaaaaa aaaatctgct ccagattttc agaaaggaaa ataagatttt 93960
acacacacac acacacacgg tttttgtgtc tggtaaacta gcaaactgtc tcatctgatt 94020
tctttgccat catttctgct ttaaattaga actctagaga atgtaaatat ctaattatct 94080
actggtcagc ttcagagatt tcaggcttca aagaagttca gtgtggtttt tgtgtgatgg 94140
tttatgatat ggattattca aggaattc 94168

<210> 28
<211> 106
<212> DNA
<213> Homo sapiens

<400> 28
gagcaggagg atggcgtcct acaagccgcc gggcccgtgg cgctcggccg gctcgaggtc 60
ggcctcgatg cactgatgca ggtgggcgtg gatgtcggac ccgtcg 106

<210> 29
<211> 106
<212> DNA

<213> Homo sapiens

<400> 29
gttgtttagg ttgtaggtgc gggtggacgt agttacgtag tttcggttgg agttcggtcg 60
gttcgcggtg ttcgggtcgt cgaatatttt gcggtaggag gacgag 106